# EUROPEAN PATENT APPLICATION

(11) **EP 4 269 395 A1**
(43) Date of publication of application: **01.11.2023**
(21) Application number: 21911371.9
(22) Date of filing: 14.12.2021
(51) Int. Cl.: C07D 239/94, A61K 31/5377, A61K 31/517, A61P 35/00, C07D 403/04, C07D 405/12, C07D 409/12

(54) **NOVEL QUINAZOLINE DERIVATIVE COMPOUND AS SOS1 INHIBITOR, AND USE THEREOF**

(30) Priority: 22.12.2020 KR 20200180879; 15.09.2021 KR 20210123208
(71) Applicant: Hanmi Pharmaceutical Co., Ltd., Hwaseong-si, Gyeonggi-do 18536 (KR)
(72) Inventor: CHOI, Jae Yul, Hwaseong-si Gyeonggi-do 18469 (KR); KIM, Won Jeoung, Hwaseong-si Gyeonggi-do 18469 (KR); KIM, Ji Sook, Hwaseong-si Gyeonggi-do 18469 (KR); KIM, Min Jeong, Hwaseong-si Gyeonggi-do 18469 (KR); PARK, Won Gi, Hwaseong-si Gyeonggi-do 18469 (KR); AHN, Young Gil, Hwaseong-si Gyeonggi-do 18469 (KR); BAE, In Hwan, Hwaseong-si Gyeonggi-do 18477 (KR)
(74) Representative: Müller, Christian Stefan Gerd
(86) International application number: PCT/KR2021/019011
(87) International publication number: WO 2022/139304

(57) **Abstract**

The present invention relates to a novel quinazoline derivative compound as a SOS1 inhibitor and to uses thereof. More particularly, the present invention relates to a novel quinazoline derivative compound having inhibitory activity on SOS1 binding to RAS family proteins and/or RAC1, to pharmacologically acceptable salts thereof, and to pharmaceutical compositions comprising such compounds.

## Description

### Technical Field

The present invention relates to a novel quinazoline derivative compound and a use thereof as a SOS1 inhibitor and, more particularly, to a novel quinazoline derivative compound having an activity of inhibiting SOS1 binding to RAS family proteins and/or RAC1, a pharmaceutically acceptable salt thereof, or a pharmaceutical composition including such a compound.

### Background Art

It is known that rat sarcoma virus (RAS) is a family protein that are found in 20% to 30% of human cancers and are identified as Kirstin RAS oncogene homologue (KRAS), neuroblastoma RAS oncogene homolog (NRAS), and Harvey rat sarcoma virus oncogene (BRAS). RAS regulates cell proliferation through the RAF/MEK/ERK pathway leading to mitogen-activated protein kinase (MAPK) activation and the PI3K/Akt/mTOR pathway involving phosphatidylinositide 3-kinase (PI3K). Cancer-associated mutations in RAS family proteins inhibit intrinsic GAP-induced GTPase activity, thereby increasing the population of GTP-binding/active RAS family proteins.

On the other hand, RAS proteins act as molecular switches, and GTP and GDP exist in an active state (GTP binding) and an inactive state (GDP binding) state in cells. RAS bound to activated GTP recruits other proteins by binding of a cognate RAS-binding domain (RBD) to activate effector proteins, and then generate downstream signals of various functions. The active state of RAS is regulated by guanine nucleotide exchange factors (GEFs) and GTPase activating proteins (GAPs). Binding of a GTPase activating protein (GAP) such as NF1 increases the GTPase activity of RAS family proteins.

Binding of guanine nucleotide exchange factors (GEFs), such as Son of Sevenless 1 (SOS1), promotes the release of GDP from RAS family proteins, thereby allowing the RAS family proteins to bind to and activate GTP. Son of Sevenless (SOS) proteins exist in two isoforms, SOS 1 and SOS2, and only SOS1 is phosphorylated by ERK. Growth factor-induced phosphorylation of SOS1 is mostly mediated by ERK which phosphorylates at least four serine residues in the C-terminal region of SOS1. This suggests that SOS1 plays an important role in negative feedback regulation of the KRAS pathway.

The SOS1 protein consists of 1333 amino acids (150 kDa). SOS1 is a multi-domain protein including a Dbl homology domain (DH) followed by two tandem N-terminal histone domains (HD), a plextrin homology domain (PH), a helical linker (HL), a RAS exchange motif (REM), a CDC25 homology domain, and a C-terminal proline rich domain (PR). SOS1 has two binding sites for RAS family proteins: a catalytic site that binds to GDP-binding RAS family proteins and promotes guanine nucleotide exchange, and an allosteric site that binds to GTP-binding RAS-family proteins causing a further increase in the catalytic GEF function of SOS1. Selective pharmacological inhibition for catalytic site binding of SOS 1 to RAS family proteins is expected that SOS 1-mediated activation of RAS family proteins is prevented by a GTP-bound form.

These SOS1 inhibitor compounds are expected to consequently inhibit signaling (i.e., ERK phosphorylation) in cells downstream of RAS-family proteins. Accordingly, novel SOS1 inhibitor compounds that bind to the SOS1 catalytic site (confirmed by crystallography) and which at the same time prevent binding to and activation of RAS family proteins are being developed. Specifically, a substance that has a significant inhibitory effect on the interaction between SOS1 and RAS family proteins, especially KRAS (low IC₅₀ value) among the RAS family proteins, thereby inducing a significant decrease in ERK phosphorylation in a KRAS mutant cancer cell line is, under development.

The present inventors have found that a novel quinazoline derivative compound as a SOS1 inhibitor has an activity to inhibit SOS1 binding to RAS family proteins and/or RAC1, thereby completing the present disclosure.

### DISCLOSURE

### Technical Solution

One objective of the present invention is to provide a novel quinazoline derivative compound that is excellent in inhibiting SOS1 binding to RAS family proteins and/or to RAC1.

Another objective of the present invention is to provide a pharmaceutical composition including the compound in a therapeutically effective amount.

### Technical Solution

According to one embodiment of the present invention, there is provided a compound selected from compounds of Chemical Formula 1 below and pharmaceutically acceptable salts, optical isomers, diastereomer, hydrate, and solvates of the compounds of Chemical Formula 1:

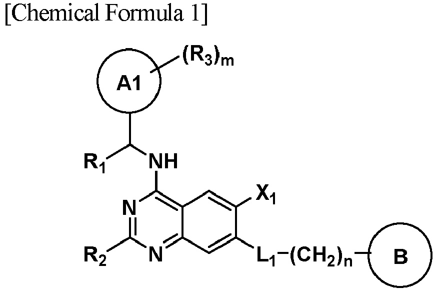

In Chemical Formula 1,
R₁ is hydrogen or C₁₋₄ alkyl;
R₂ is hydrogen, C₁₋₄ alkyl, halo C₁₋₄ alkyl, C₂₋₆ alkenyl or C₂₋₆ alkynyl;
R₃ is R₃ₐ or -L₂- 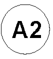 ;
R₃ₐ are each independently any one selected from halogen, hydroxy, cyano, amino, amine, nitro, oxo (=O), C₁₋₆ alkyl, C₂₋₆ alkenyl, C₂₋₆ alkynyl, halo C₁₋₆ alkyl, amino C₁₋₆ alkyl, C₁₋₆ alkoxy, hydroxy-C₁₋₄ alkyl, -CF₂H, -(CH₂)ᵣ-NH(CO)-Rₐ, -(CH₂)ᵣ-NRₐR_{b}, and Rₐ and R_{b} are each independently any one selected from the group consisting of hydrogen, C₁₋₆ alkyl, halo C₁₋₆ alkyl, hydroxy-C₁₋₆ alkyl, C₂₋₆ alkenyl, C₂₋₆ alkynyl, C₁₋₆ alkoxy, -CF₂H, and C₃₋₈ carbocyclyl;
r is an integer in the range of 0 to 1;
m is an integer in the range of 0 to 5;
L₂ is a direct bond, -O-(CH₂)ₚ, or -CH=CH-(CH₂)_{q};
p is an integer in the range of 0 to 3;
q is an integer in the range of 0 to 2;
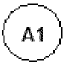 and 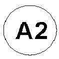 are each independently C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl, wherein C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl of 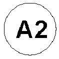 is unsubstituted or substituted with one or more R₃ₐ;
X₁ is -O(R₄) or -N(R₅)(R₆);
R₄ is hydrogen, C₁₋₆ alkyl, hydroxy C₁₋₆ alkyl, C₂₋₆ alkenyl, C₂₋₆ alkynyl, C₁₋₆ alkoxy, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridge carbocyclyl, or C₆₋₁₄ bridged heterocyclyl, which is unsubstituted or substituted with halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, - S(O)-C₁₋₄ alkyl, -S(O)₂-C₁₋₄ alkyl, -C(O)-NR_{c}R_{d}, -C(O)OR_{c}, -OR_{c}, or -NR_{c}R_{d}, wherein R_{c} and R_{d} are each independently hydrogen or C₁₋₆ alkyl;
R₅ and R₆ are each hydrogen, C₁₋₆ alkyl, hydroxy C₁₋₆ alkyl, C₂₋₆ alkenyl, C₂₋₆ alkynyl, C₁₋₆ alkoxy, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ crosslinked car bocyclyl, or C₆₋₁₄ bridged heterocyclyl, wherein C₁₋₆ alkyl, hydroxy C₁₋₆ alkyl, C₂₋₆ alkenyl, C₂₋₆ alkynyl, C₁₋₆ alkoxy, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridged carbocyclyl, or C₆₋₁₄ bridged heterocyclyl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, - S(O)₂-C₁₋₄ alkyl, -C(O)-NRₑR_{f}, -C(O)OR_{c}, -ORₑ, and -NRₑR_{f}, wherein Rₑ and R_{f} are each independently hydrogen or C₁₋₆ alkyl, and
alternatively, the -N(R₅)(R₆) is C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl in each of which R₅ and R₆ are linked to each other and form a ring in conjunction with a nitrogen atom, wherein C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, -C(O)-NR_{g}Rₕ, -C(O)OR_{g}, -OR_{g}, and -NR_{g}Rₕ, wherein R_{g} and Rₕ are each independently hydrogen, C₁₋₆ alkyl, hydroxy-C₁₋₆ alkyl, C₂₋₆ alkenyl, C₂₋₆ alkynyl, C₁₋₆ alkoxy, halo C₁₋₆ alkoxy, or C₃₋₈ carbocyclyl;
L₁ is a direct bond, -C(O)-, -O-, or -NH-;
n is an integer in the range of 0 to 2; and
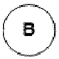 is C₃₋₁₀ carbocyclyl, C₂₋₉ heterocyclyl, C₆₋₁₀ aryl, C₄₋₁₀ heteroaryl, C₆₋₁₄ heterospirocarbocyclyl, C₆₋₁₄ fused heterocyclyl, or C₆₋₁₄ bridged heterocyclyl, wherein C₃₋₁₀ carbocyclyl, C₂₋₉ heterocyclyl, C₆₋₁₀ aryl, C₄₋₁₀ heteroaryl, C₆₋₁₄ heterospirocarbocyclyl, C₆₋₁₄ fused heterocyclyl, or C₆₋₁₄ bridged heterocyclyl is unsubstituted or substituted with one or more functional groups selected from the group consisting of halogen, hydroxy, nitro, oxo (=O), halo C₁₋₆ alkyl, C₁₋₆ alkyl, C₂₋₆ alkenyl, C₂₋₆ alkynyl, C₁₋₆ alkoxy, -S(O)-C₁₋₄ alkyl, -S(O)₂-C₁₋₄ alkyl, -C(O)-NRᵢRⱼ, - C(O)ORᵢ, -ORᵢ, and -NRᵢRⱼ, wherein Rᵢ and Rⱼ are each independently hydrogen or C₁₋₆ alkyl.

According to another embodiment of the present invention, there is provided a pharmaceutical composition and pharmaceutical preparation for the prevention or treatment of various diseases related to inhibition of SOS1 binding to RAS family proteins and/or RAC1, the composition and preparation including the compound described above in a therapeutically effective amount.

According to a further embodiment of the present invention, there is provided a method of inhibiting SOS1 binding to a RAS family protein and/or RAC1 in a subject or cell, the method including administering a pharmaceutically effective amount of the compound to the subject.

According to a yet further embodiment of the present invention, there is provided a method of inhibiting tyrosine kinase in a subject or cell, the method including administering a pharmaceutically effective amount of the compound to the subject.

According to a yet further embodiment of the present invention, there is provided a method of for preventing or treating cancer in a subject, the method including administering to the subject a pharmaceutically effective amount of the compound.

According to a yet further embodiment of the present invention, there are provided uses of the compounds or pharmaceutically acceptable salts described above for the prevention or treatment of cancer or tumors.

### Advantageous Effects

The quinazoline derivative compound of Chemical Formula 1 in the present invention has excellent ability to inhibit SOS1 binding to RAS family proteins and/or RAC1, has anticancer activity against cancer associated with cell proliferation attributable to abnormal SOS1 activity, and can be usefully used as a therapeutic agent.

### Mode for Invention

All technical terms used in the present invention, unless otherwise defined, have the meanings commonly understood by the ordinarily skilled in the related art of the present invention. In addition, although preferred methods and samples are described herein, similar or equivalent ones also fall within the scope of the present invention. In addition, the numerical values described herein are considered to include the meaning of "about" even if not specified. The contents of all publications incorporated herein by reference are hereby incorporated by reference in their entirety.

In Chemical Formula 1, residues listed as R₁ to R₆ are used as commonly understood by those skilled in the art.

In the present invention, the term "halogen" refers to fluorine, chlorine, bromine, or iodine, unless otherwise specified. Specifically, the term "halogen" may refer to fluorine or chlorine but may not be limited thereto.

As used herein, the term "alkyl" refers to a saturated, straight-chain or branched monovalent hydrocarbon radical, unless otherwise specified.

In the present invention, the term "alkenyl", unless otherwise specified, refers to a monovalent hydrocarbon radical containing at least one carbon-carbon double bond, each double bond having a three-dimensional E- or Z-arrangement.

The term "alkynyl" as used herein, unless otherwise specified, refers to a monovalent group derived from an unsaturated, straight-chain or branched hydrocarbon moiety having at least one carbon-carbon triple bond.

These alkyl, alkenyl, and alkynyl groups may be straight (i.e., straight-chained) or branched. Depending on the definition, the number of carbon atoms in the alkyl group may be 1, 2, 3, 4, 5 or 6 or may be 1, 2, 3, or 4. Examples of alkyl include: methyl; ethyl; propyl including n-propyl and isopropyl; butyl including n-butyl, sec-butyl, isobutyl, and tert-butyl; pentyl including n-pentyl, 1-methylbutyl, isopentyl, neopentyl, and tert-pentyl; and hexyl including n-hexyl, 3,3-dimethylbutyl, and isohexyl. Each of the double and triple bonds in the alkenyl group and the alkynyl group may be present at any position. Examples of alkenyl and alkynyl are ethenyl, prop-1-enyl, prop-2-enyl (= allyl), but-2-enyl, 2-methylprop-2-enyl, 3-methylbut- 2-enyl, hex-3-enyl, hex-4-enyl, prop-2-ynyl (= propargyl), but-2-ynyl, but-3-ynyl, hex-4-ynyl, or hex-5-ynyl. However, each of the alkyl, alkenyl, and alkynyl groups may substitute at any position, as long as each compound is sufficiently stable and suitable for a desired purpose such as use as a pharmaceutical substance.

In the present invention, the term "carbocyclyl" refers to cyclic alkyl which may be substituted or unsubstituted and may refer to mono or bicycloaliphatic unless otherwise specified. Preferably, examples of the carbocyclyl include, but are not limited to, aryl, carbocyclyl, spirocarbocyclyl, fused carbocyclyl, and bridged carbocyclyl. More preferably, examples of the - 1carbocyclyl include cyclopropyl, cyclobutyl, cyclopentyl, cyclohexyl, cyclohexenyl, cycloheptyl, cyclohepsenyl, cyclooctyl, cyclooctenyl, 2,5-cyclohexadienyl, bicyclo[2.2.2]octyl, adamant-1-yl, decahydronaphthyl, oxocyclohexyl, dioxocyclohexyl, thiocyclohexyl, 2-oxobicyclo[22.1]hept-1-enyl, or all possible isomers thereof without limitation.

In the present invention, the term "heterocyclyl" refers to a monocyclic, bicyclic, or multicyclic alkyl that contains one or more (specifically 1, 2, 3, or 4) heteroatoms selected from O, N, and S and which may be substituted or unsubstituted. Preferably, examples of the heterocyclyl include, but are not limited to, heteroaryl, heterocyclyl, heterospirocarbocyclyl, fused heterocyclyl, and bridged heterocyclyl. More preferably, examples of the heterocyclyl include, but are not limited to, piperazinyl, piperidinyl, piperazinyl-1-oxide, morpholinyl, thiamorpholinyl, pyrrolidinyl, imidazolinyl, tetrahydrofuranyl, diazabicyclo octanyl, diazaspirooctanyl, and similar groups thereto. For example, in the case of C₂₋₁₀ heterocyclyl, C₂₋₁₀ representing a carbon number means a ring size of a 3-membered or greater-numbered ring including one or more heteroatoms.

In the present invention, the term "aryl" refers to an aromatic group that may be substituted or unsubstituted, unless otherwise specified. For example, the aryl includes phenyl, biphenyl, naphthyl, toluyl, naphthalenyl, anthracenyl, and all isomers thereof without limitation.

In the present invention, the term "heteroaryl" refers to a monocyclic, bicyclic, or multicyclic aromatic group that contains one or more (specifically 1, 2, 3, or 4) heteroatoms selected from O, N, and S and which may be substituted or unsubstituted. Preferably, examples of the monocyclic heteroaryl include thiazolyl, oxazolyl, thiophenyl, furanyl, pyrrolyl, imidazolyl, isoxazolyl, pyrazolyl, triazolyl, thiadiazolyl, tetrazolyl, oxadiazolyl, pyridinyl, pyridazinyl, pyrimidinyl, pyrazinyl, and the like but are not limited thereto. Preferably, examples of the bicyclic heteroaryl include indolyl, benzothiophenyl, benzofuranyl, benzimidazolyl, benzoxazolyl, benzisoxazolyl, benzthiazolyl, benzthiadiazolyl, benztriazolyl, quinolineyl, isoquinolinyl, purinyl, puropyridinyl, and the like, but are not limited thereto.

In the present invention, the numerical range indicated using the term "to" refers to a range including the numerical values described before and after the term "to" as the lower limit and the upper limit, respectively.

As used herein, the term "SOS1 binding to RAS family protein and/or RAC1" refers to binding between SOS 1 and RAS family protein at the catalytic site of SOS 1, and "activity inhibiting binding" refers to that a GTP-binding form prevents SOS1-mediated activation of RAS-family proteins.

As used herein, the term "SOS1 inhibitor compound" refers to a compound that inhibits signal transduction to lower cells by RAS-family proteins, for example, inhibits ERK phosphorylation. Specifically, "SOS1 inhibitor compound" means a compound that binds to the SOS1 catalytic site, thereby preventing SOS1 from binding to RAS family proteins or preventing activation of RAS family proteins.

In the present invention, the term "enantiomer" refers to various possible stereoisomers and geometric isomers of the compound according to the present invention, unless otherwise stated. Since the compounds of Chemical Formula 1 according to an aspect of the present invention may have an asymmetric carbon center (absent carbon), the compounds may exist as enantiomers (R or S isomers), racemates, diastereomers, or any mixtures thereof. All these isomers and mixtures fall within the scope of the present invention. The optically active (R)- and (S)- isomers can be resolved using conventional techniques or prepared using chiral synthons or chiral reagents. When the compounds contain a double bond, the substituent may be in an E or Z form. When the compound contains a disubstituted carbocyclyl, the compound may be in a cis- or trans form. In addition, when the compound of Chemical Formula 1 includes a bridged ring, the compound may exist as an exo or endo isomer. In addition, all tautomers may also be included.

In the present invention, the term "asymmetric carbon atom" refers to a carbon atom in a molecule that contains four different atoms, atomic groups, or functional groups bonded to the carbon atom, unless otherwise stated. In the case of a compound containing such an asymmetric carbon atom, the compound has a photorotation property or optical isomers. Specifically, the compound having the structure of Chemical Formula 1 having the asymmetric carbon atom may be a compound having the structure of Chemical Formula 1a or Chemical Formula 1b below. On the other hand, a 1:1 mixture of a pair of enantiomers is referred to as a "racemic" mixture.

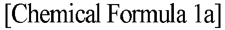

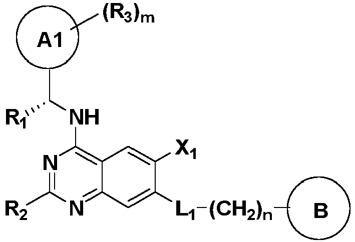

In Chemical Formula 1a, 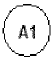 , R₁ to R₃, X₁, L₁, 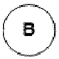 , m, and n are defined in the same manner as in Chemical Formula 1.

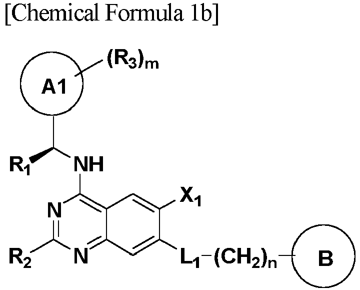

In Chemical Formula 1b, 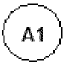 , R₁ to R₃, X₁, L₁, 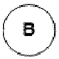 , m, and n are defined in the same manner as in Chemical Formula 1.

In one aspect, the compound of Chemical Formula 1, an optical isomer thereof, and a diastereomer thereof may exist in the form of a solvate. The term "solvate" may include molecular complexes, each containing the compound and one or more pharmaceutically acceptable solvent molecules, such as ethanol or water. Complexes in which the solvent molecule is water are referred to as "hydrates".

In one aspect, the compound of Chemical Formula 1, an optical isomer thereof, and a diastereomer thereof, and a solvate thereof may exist in the form of a pharmaceutically acceptable salt.

In the present invention, the term "pharmaceutically acceptable salt" should have low toxicity to the human body and should not adversely affect the biological activity and physicochemical properties of the parent compound. Pharmaceutically acceptable salts include acid addition salts formed by a pharmaceutically acceptable free acid and a base compound of Chemical Formula 1, alkali metal salts (such as sodium salts) and alkaline earth metal salts (such as calcium salts), organic base addition salts formed by an organic base and a carboxylic acid structure of Chemical Formula 1, and amino acid addition salts, but are not limited thereto.

Preferred salt forms of the compounds according to the invention include salts with inorganic or organic acids. In this case, as the inorganic acid, hydrochloric acid, sulfuric acid, nitric acid, phosphoric acid, perchloric acid, bromic acid, and the like may be used. In addition, as the organic acid, acetic acid, methanesulfonic acid, ethanesulfonic acid, p-toluenesulfonic acid, fumaric acid, maleic acid, malonic acid, phthalic acid, succinic acid, lactic acid, citric acid, citric acid, gluconic acid, tartaric acid, salicylic acid, malic acid, oxalic acid, benzoic acid, embonic acid, aspartic acid, glutamic acid, and the like may be used. Organic bases that can be used to prepare organic base addition salts are tris(hydroxymethyl)methylamine, dicyclohexylamine, and the like. Amino acids that can be used to prepare amino acid addition salts are natural amino acids such as alanine and glycine. It will be apparent to those of ordinary skill in the art that other acids or bases may be used aside from the inorganic acids, organic acids, organic bases, and amino acids exemplified above.

The salts may be prepared by a conventional method. For example, the salts can be prepared by: dissolving the compound of Chemical Formula 1 in a solvent miscible with water, such as methanol, ethanol, acetone, and 1,4-dioxane; adding a free acid or a free base to the resulting solution; and crystallizing the resulting product.

The details in the description of the pharmaceutical composition according to one aspect of the present invention are applicable, as they are, to a prevention or treatment method according to another aspect of the present invention.

In the present invention, the term "treatment" is used as a concept including all meanings of therapy, improvement, amelioration, or management of a disease.

As used herein, the term "preventing" or "prevention" refers to preventing a disease, condition, or disorder in a subject who may be predisposed to the disease, condition, or disorder but does not yet experience or exhibit the pathology or signs of the disease.

As used herein, the term "subject" or "patient" refers to any mammal, for example, an animal, including mice, rats, other rodents, rabbits, dogs, cats, pigs, cattle, sheep, horses, primates, and humans.

Hereinafter, the present invention will be described in more detail.

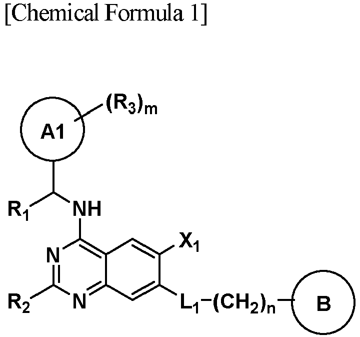

In Chemical Formula 1,
R₁ is hydrogen or C₁₋₄ alkyl;
R₂ is hydrogen, C₁₋₄ alkyl, halo C₁₋₄ alkyl, C₂₋₆ alkenyl or C₂₋₆ alkynyl;
R₃ is R₃ₐ or -L₂- 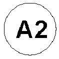 ;
R₃ₐ are each independently halogen, hydroxy, cyano, amino, amine, nitro, oxo (=O), C₁₋₆ alkyl, C₂₋₆ alkenyl, C₂₋₆ alkynyl, halo C₁₋₆ alkyl, amino C₁₋₆ alkyl, C₁₋₆ alkoxy, hydroxy-C₁₋₄ alkyl, - CF₂H, -(CH₂)ᵣ-NH(CO)-Rₐ, or -(CH₂)r-NRₐR_{b}, in which Rₐ and R_{b} are each independently any one selected from the group consisting of hydrogen, C₁₋₆ alkyl, halo C₁₋₆ alkyl, hydroxy-C₁₋₆ alkyl, C₂₋₆ alkenyl, C₂₋₆ alkynyl, C₁₋₆ alkoxy, -CF₂H, and C₃₋₈ carbocyclyl;
r is an integer in the range of 0 to 1;
m is an integer in the range of 0 to 5;
L₂ is a direct bond, -O-(CH₂)ₚ, or -CH=CH-(CH₂)_{q};
p is an integer in the range of 0 to 3;
q is an integer in the range of 0 to 2;
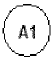 and 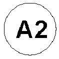 are each independently C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl, in which C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl of 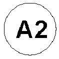 is unsubstituted or substituted with one or more R₃ₐ;
X₁ is -O(R₄) or -N(R₅)(R₆);
R₄ is hydrogen, C₁₋₆ alkyl, hydroxy C₁₋₆ alkyl, C₂₋₆ alkenyl, C₂₋₆ alkynyl, C₁₋₆ alkoxy, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridge carbocyclyl, or C₆₋₁₄ bridged heterocyclyl each of which is unsubstituted or substituted with halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, -C(O)-NR_{c}R_{d}, -C(O)OR_{c}, -OR_{c}, or -NR_{c}R_{d}, in which R_{c} and R_{d} are each independently hydrogen or C₁₋₆ alkyl;
R₅ and R₆ are each hydrogen, C₁₋₆ alkyl, hydroxy C₁₋₆ alkyl, C₂₋₆ alkenyl, C₂₋₆ alkynyl, C₁₋₆ alkoxy, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridged carbocyclyl, or C₆₋₁₄ bridged heterocyclyl, wherein C₁₋₆ alkyl, hydroxy C₁₋₆ alkyl, C₂₋₆ alkenyl, C₂₋₆ alkynyl, C₁₋₆ alkoxy, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridged carbocyclyl, or C₆₋₁₄ bridged heterocyclyl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, - C(O)-NRₑR_{f}, -C(O)OR_{c}, -ORₑ, and -NRₑR_{f}, wherein Rₑ and R_{f} are each independently hydrogen or C₁₋₆ alkyl, or
alternatively, the -N(R₅)(R₆) is C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl in each of which R₅ and R₆ are linked to each other and form a ring in conjunction with a nitrogen atom contained in the -N(R₅)(R₆), in which C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆alkynyl, C₁₋₆ alkoxy, halo C₁₋₆ alkoxy, -S(O)-C₁₋₄ alkyl, -S(O)₂-C₁₋₄ alkyl, -C(O)-NR_{g}Rₕ, -C(O)OR_{g}, -OR_{g}, and -NR_{g}Rₕ, wherein R_{g} and Rₕ are each independently hydrogen, C₁₋₆ alkyl, hydroxy-C₁₋₆ alkyl, C₂₋₆ alkenyl, C₂₋₆ alkynyl, C₁₋₆ alkoxy, halo C₁₋₆ alkoxy, or C₃₋₈ carbocyclyl;
L₁ is a direct bond, -C(O)-, -O-, or -NH-;
n is an integer in the range of 0 to 2; and
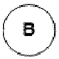 is C₃₋₁₀ carbocyclyl, C₂₋₉ heterocyclyl, C₆₋₁₀ aryl, C₄₋₁₀ heteroaryl, C₆₋₁₄ heterospirocarbocyclyl, C₆₋₁₄ fused heterocyclyl, or C₆₋₁₄ bridged heterocyclyl, in which C₃₋₁₀ carbocyclyl, C₂₋₉ heterocyclyl, C₆₋₁₀ aryl, C₄₋₁₀ heteroaryl, C₆₋₁₄ heterospirocarbocyclyl, C₆₋₁₄ fused heterocyclyl, or C₆₋₁₄ bridged heterocyclyl is unsubstituted or substituted with one or more functional groups selected from the group consisting of halogen, hydroxy, nitro, oxo (=O), halo C₁₋₆ alkyl, C₁₋₆ alkyl, C₂₋₆ alkenyl, C₂₋₆ alkynyl, C₁₋₆ alkoxy, -S(O)-C₁₋₄ alkyl, -S(O)₂-C₁₋₄ alkyl, -C(O)-NRᵢRⱼ, - C(O)ORᵢ, -ORᵢ, and -NRᵢRⱼ, in which Rᵢ and Rⱼ are each independently hydrogen or C₁₋₆ alkyl.

Preferably, in a compound selected from the compound of Chemical Formula 1 of the present invention, pharmaceutically acceptable salts thereof, optical isomers thereof, diastereomers thereof, hydrates thereof, and solvates thereof, 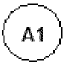 and 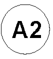 are each independently C₆₋₁₀ aryl or C₄₋₁₀ heteroaryl.

Preferably, in a compound selected from the compound of Chemical Formula 1 of the present invention, pharmaceutically acceptable salts thereof, optical isomers thereof, diastereomers thereof, hydrates thereof, and solvates thereof, F₃ₐ may be each independently halogen, hydroxy, cyano, amino, amine, nitro, C₁₋₆ alkyl, amino C₁₋₆ alkyl, C₁₋₆ alkoxy, -CF₂H, C₆₋₁₀ aryl, C₃₋₆ cyclyl, - (CH₂)ᵣC₂₋₆ heterocyclyl, -(CH₂)ᵣ-NH(CO)-Rₐ, or -(CH₂)ᵣ-NRₐR_{b}, in which Rₐ and R_{b} may be each independently hydrogen, C₁₋₆ alkyl, -CF₃, or -CF₂H.

Preferably, in a compound selected from the compound of Chemical Formula 1 of the present invention, pharmaceutically acceptable salts thereof, optical isomers thereof, hydrate thereof, and solvates thereof, and R₄ is hydrogen, C₁₋₆ alkyl, C₂₋₆ alkenyl, C₂₋₆ alkynyl, C₁₋₆ alkoxy, C₃₋₁₀ carbocyclyl, or C₂₋₉ heterocyclyl, R₅ and R₆ are each independently hydrogen, C₁₋₆ alkyl, C₂₋₆ alkenyl, C₂₋₆ alkynyl, C₁₋₆ alkoxy, C₃₋₁₀ carbocyclyl, or C₂₋₉ heterocyclyl, or the -N(R₅)(R₆) is C₂₋₉ heterocyclyl.

According to another embodiment of the present invention, the compound represented by Chemical Formula 1 can be represented as Chemical Formula 2 below:

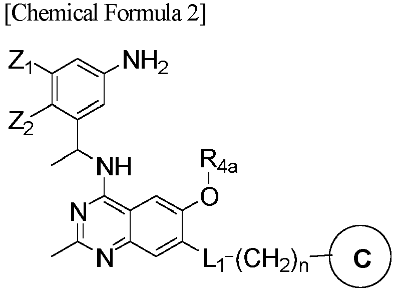

In Chemical Formula 2,
L₁ is a direct bond, -C(O)-, -O-, or -NH-;
n is an integer in the range of 0 to 2; and
Z₁ and Z₂ are each independently hydrogen, -F, -CF₂H, -CF₃, -CH₃, or -NH₂, but for the case in which Z₁ and Z₂ are both hydrogen;
R₄ₐ is hydrogen, C₁₋₆ alkyl, C₃₋₁₀ carbocyclyl, or C₂₋₉ heterocyclyl;
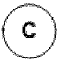 is morpholinyl, thiomorpholinyl, dioxothiomorpholinyl, piperazinyl, thiazolidinyl, tetrahydropyranyl, hexahydro-1*H*-furo[3,4-c]pyrrolyl, oxetanyl or azetidinyl, wherein the morpholinyl, thiomorpholinyl, dioxothiomorpholinyl, piperazinyl, thiazolidinyl, tetrahydropyranyl, hexahydro-1*H*-furo[3,4-c]pyrrolyl, oxetanyl or azetidinyl is unsubstituted or substituted with one or more functional groups selected from the group consisting of halogen or -CH₃.

According to a further embodiment of the present invention, the compound represented by Chemical Formula 1 can be represented as Chemical Formula 3 below:

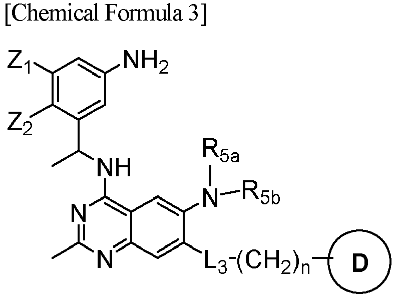

In Chemical Formula 3,
L₃ is a direct bond or -C(O)-;
n is an integer in the range of 0 to 2; and
Z₁ and Z₂ are each independently hydrogen, -F, -CF₂H, -CF₃, -CH₃, or -NH₂, but for the case in which Z₁ and Z₂ are both hydrogen;
R₅ₐ and R_{5b} are each independently hydrogen, C₁₋₆ alkyl, C₁₋₆ alkoxy, C₃₋₁₀ carbocyclyl, or C₂₋₉heterocyclyl, or
alternatively the -N(R₅ₐ)(R_{5b}) is a C₂₋₉ heterocyclyl in which R₅ₐ and R_{5b} are linked to each other and form a ring in conjunction with a nitrogen atom contained in the -N(R₅ₐ)(R_{5b});
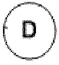 is morpholinyl, dioxothiomorpholinyl, thiomorpholinyl, piperazinyl, thiazolidinyl, tetrahydropyranyl, hexahydro- IH-furo[3,4-c]pyrrolyl, oxetanyl or azetidinyl, in which morpholinyl, dioxothiomorpholinyl, thiomorpholinyl, piperazinyl, thiazolidinyl, tetrahydropyranyl, hexahydro-1*H*-furo[3,4-c]pyrrolyl, oxetanyl or azetidinyl is unsubstituted or substituted with one or more functional groups selected from the group consisting of halogen or -CH₃.

According to a yet further embodiment of the present invention, the compound represented by Chemical Formula 2 can be represented as Chemical Formula 4 below:

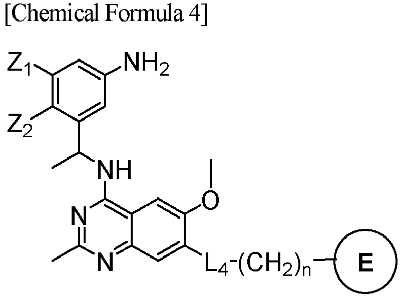

In Chemical Formula 4,
L₄ is a direct bond, -C(O)- or -O-;
n is an integer in the range of 0 to 2; and
Z₁ and Z₂ are each independently hydrogen, -F, -CF₂H, -CF₃, -CH₃, or -NH₂, but for the case in which Z₁ and Z₂ are both hydrogen;
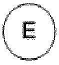 is morpholinyl, thiomorpholinyl, piperazinyl, thiazolidinyl, or tetrahydropyranyl, wherein the morpholinyl, thiomorpholinyl, piperazinyl, thiazolidinyl, or tetrahydropyranyl is unsubstituted or substituted with one or more functional groups selected from the group consisting of halogen or -CH₃.

According to a still yet further embodiment of the present invention, the compound represented by Chemical Formula 3 can be represented as Chemical Formula 5 below:

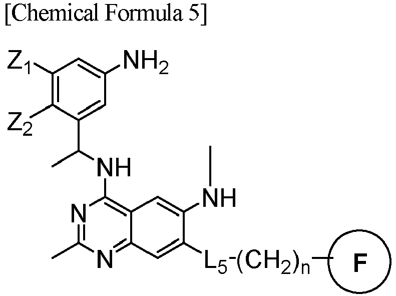

In Chemical Formula 5,
L₅ is a direct bond or -C(O)-;
n is an integer in the range of 0 to 2; and
Z₁ and Z₂ are each independently hydrogen, -F, -CF₂H, -CF₃, -CH₃, or -NH₂, but for the case in which Z₁ and Z₂ are both hydrogen;
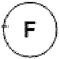 is morpholinyl, dioxothiomorpholinyl, thiomorpholinyl, piperazinyl, thiazolidinyl, or hexahydro-1*H*-furo[3,4-c]pyrrolyl, wherein the morpholinyl, dioxothiomorpholinyl, thiomorpholinyl, piperazinyl, thiazolidinyl, or hexahydro-1 //-furo[3,4-c]pyrrolyl is unsubstituted or substituted with one or more functional groups selected from the group consisting of halogen or - CH3.

In addition, preferred examples of the compound of Chemical Formula 1 according to the present invention are as follows, but not limited thereto:
(*R*)-(4-((1-(3-amino-5-(trifluoromethyl)phenyl)ethyl)amino)-6-methoxy-2-methylquinazoline-7-yl)(morpholino)methanone;
(6-methoxy-2-methyl-4-((1-(4-(2-((methylamino)methyl)phenyl)thiophen-2-yl)ethyl)amino)quinazoline-7-yl)(morpholino)methanone;
(4-(((*R*)-1-(3-amino-5-(trifluoromethyl)phenyl)ethyl)amino)-6-methoxy-2-methylquinazoline-7-yl)((3*R*, 5*S*)-3,5-dimethylpiperazin-1-yl)methanone;
(*R*)-(4-((1-(3-amino-5-(trifluoromethyl)phenyl)ethyl)amino)-6-methoxy-2-methylquinazoline-7-yl)(thiomorpholino)methanone;
(4-(((*R*)-1-(3-amino-5-(trifluoromethyl)phenyl)ethyl)amino)-6-methoxy-2-methylquinazoline-7-yl)(tetrahydro-1*H*-furo[3,4-c]pyrrol-5(3*H*)-yl)methanone;
(*R*)-(4-((1-(3-(difluoromethyl)-2-fluorophenyl)ethyl)amino)-6-methoxy-2-methylquinazoline-7-yl)(morpholino)methanone;
(*R*)-(4-((1-(3-amino-5-(trifluoromethyl)phenyl)ethyl)amino)-6-methoxy-2-methylquinazoline-7-yl)(azetidine-1-yl)methanone;
(6-methoxy-2-methyl-4-((1-(4-(1,2,3,4-tetrahydroisoquinolin-8-yl)thiophen-2-yl)ethyl)amino)quinazoline-7-yl)(morpholino)methanone;
(*R*)-(4-((1-(3-amino-5-(trifluoromethyl)phenyl)ethyl)amino)-6-methoxy-2-methylquinazoline-7-yl)(piperazine-1-yl)methanone;
(*R*)-2,2,2-trifluoro-*N*-(3-(1-((6-methoxy-2-methyl-7-(morpholine-4-carbonyl)quinazoline-4-yl)amino)ethyl)-5-(trifluoromethyl)phenylacetamide;
(*R*)-(4-((1-(3-amino-5-(trifluoromethyl)phenyl)ethyl)amino)-6-methoxy-2-methylquinazoline-7-yl)(3-ffuorazetidine-1-yl)methanone;
(4-((1-(4-(2-((dimethylamino)methyl)phenyl)thiophen-2-yl)ethyl)amino)-6-methoxy-2-methoxyquinazoline-7-yl)(morpholino)methanone;
(4-((1-(4-(2-((aminomethyl)phenyl)thiophen-2-yl)ethyl)amino)-6-methoxy-2-methylquinazoline-7-yl)(morpholino)methanone;
(4-((1-(4-(2-((hydroxymethyl)phenyl)thiophen-2-yl)ethyl)amino)-6-methoxy-2-methylquinazoline-7-yl)(morpholino)methanone;
(*R*)-(6-methoxy-2-methyl-4-((1-(3-(trifluoromethyl)phenyl)ethyl)amino)quinazolin-7-yl)(morpholino)methanone;
(*R*)-(4-((1-(5-amino-2-methyl-3-(trifluoromethyl)phenyl)ethyl)amino)-6-methoxy-2-methylquinazolin-7-yl)(morpholino)methanone;
(*R*)-(4-((1-(3-amino-5-(fluorophenyl)ethyl)amino)-6-methoxy-2-methylquinazolin-7-yl)(morpholino)methanone;
(*R*)-(4-((1-(3-amino-5-(trifluoromethyl)phenyl)ethyl)amino)-6-methoxy-2-methylquinazolin-7-yl)(1,1-dioxothiomorpholino)methanone;
(*R*)-(4-((1-(3-amino-2-methoxyphenyl)ethyl)amino)-6-methoxy-2-methylquinazolin-7-yl)(morpholino)methanone;
(*R*)-(4-((1-(3-amino-5-(trifluoromethyl)phenyl)ethyl)amino)-6-methoxy-2-methylquinazolin-7-yl)(thiazolidine-3-yl)methanone;
(*R*)-(4-((1-(3-amino-5-methylphenyl)ethyl)amino)-6-methoxy-2-methylquinazolin-7-yl)(morpholino)methanone;
(*R*)-3-amino-5-(1-((6-methoxy-2-methyl-7-(morpholine-4-carbonyl)quinazolin-4-yl)amino)ethyl)benzonitrile;
(*R*)-(4-((1-(2,3-dihydro-1*H*-inden-4-yl)ethyl)amino)-6-methoxy-2-methylquinazolin-7-yl)(morpholino)methanone;
(*R*)-(4-((1-(3-amino-5-cyclopropylphenyl)ethyl)amino)-6-methoxy-2-methylquinazolin-7-yl)(morpholino)methanone;
(*R*)-(4-((1-(5-amino-2-fluoro-3-(trifluoromethyl)phenyl)ethyl)amino)-6-methoxy-2-methylquinazolin-7-yl)(morpholino)methanone;
(*R*)-(4-((1-(5-amino-3-(difluoromethyl)-2-fluorophenyl)ethyl)amino)-6-methoxy 2-methylquinazolin-7-yl)(morpholino)methanone;
(4-(((*R*)-1-(3-amino-5-(trifluoromethyl)phenyl)ethyl)amino)-2-methyl-6-(((*S*)-tetrahydrofuran-3-yl)oxy)quinazolin-7-yl)(morpholino)methanone;
(*R*)-(4-((1-(3-amino-5-(furan-3-yl)phenyl)ethyl)amino)-6-methoxy-2-methylquinazolin-7-yl)(morpholino)methanone;
(*R*)-(4-((1-(3-amino-5-(difluoromethyl)phenyl)ethyl)amino)-6-methoxy-2-methylquinazolin-7-yl)(morpholino)methanone;
(*R*)-(4-((1-(3-amino-5-(thiazole-5-yl)phenyl)ethyl)amino)-6-methoxy-2-methylquinazolin-7-yl)(morpholino)methanone;
(*R*)-(4-((1-(3-(ethylamino)-5-(trifluoromethyl)phenyl)ethyl)amino)-6-methoxy-2-methylquinazolin-7-yl)(morpholino)methanone;
methyl (*R*)-(4-((1-(3-amino-5-(triffuoromethyl)phenyl)ethyl)amino)-6-(2-methoxyethoxy)-2-methylquinazolin-7-yl)(morpholino)methanone;
(R)-(4-((1-(3-amino-5-(trifluoromethyl)phenyl)ethyl)amino)-2-(fluoromethyl)-6-methoxyquinazolin-7-yl)(morpholino)methanone;
(*R*)-*N*-(1-(3-amino- 5-(trifluoromethyl)phenyl)ethyl)-6-methoxy-2- methyl-7-(morpholinomethyl)quinazoline-4-amine;
(*R*)-*N*-(1-(5-amino-3-(difluoromethyl)-2-fluorophenyl)ethyl)-6-methoxy-2-methyl-7-(morpholinomethyl)quinazoline-4-amine;
(*R*)-*N*-(1-(3-amino-5-(trifluoromethylphenyl)ethyl)-6-methoxy-2-methyl-7-((tetrahydro-2*H*-pyran-4-yl)oxy)quinazolin-4-amine;
(*R*)-*N*-(1-(5-amino-3-(difluoromethyl)-2-fluorophenyl)ethyl)-6-methoxy-2-methyl-7-((tetrahydro-2*H*-pyran-4-yl)oxy)quinazolin-4-amine;
(*R*)-*N*-(1-(3-amino-5-(trifluoromethyl)phenyl)ethyl)-6-methoxy-2-methyl-7-((tetrahydro-2*H*-pyran-4-yl)methoxy)quinazolin-4-amine;
(*R*)-*N*-(1-(3-amino-5-(trifluoromethyl)phenyl)ethyl)-6-methoxy-2-methyl-7-(oxetan-3-ylmethoxy)quinazoline-4-amine;
(*R*)-(4-((1-(3-amino-5-(trifluoromethyl)phenyl)ethyl)amino)-2-methyl-6-(methylamino)quinazolin-7-yl)(morpholino)methanone;
(*R*)-(4-((1-(3-amino-5-(trifluoromethyl)phenyl)ethyl)amino)-6-(dimethylamino)-2-methylquinazolin-7-yl)(morpholino)methanone;
(*R*)-(4-((1-(3-amino-5-(trifluoromethyl)phenyl)ethyl)amino)-2-methyl-6-(pyrrolidin-1-yl)quinazoline-7-yl)(morpholino)methanone;
(*R*)-(4-((1-(5-amino-3-(difluoromethyl)-2-fluorophenyl)ethyl)amino)-2-methyl-6-(methylamino)quinazoline-7-yl)(morpholino)methanone;
(*R*)-(4-((1-(3-amino-5-(trifluoromethyl)phenyl)ethyl)amino)-6-((2-methoxyethyl)amino)-2-methylquinazoline-7-yl)(morpholino)methanone;
(*R*)-(4-((1-(3-amino-5-(trifluoromethyl)phenyl)ethyl)amino)-6-(cyclopentylamino)-2-methylquinazolin-7-yl)(morpholino)methanone;
(*R*)-(4-((1-(3-amino-5-(trifluoromethyl)phenyl)ethyl)amino)-6-(ethylamino)-2-methylquinazolin-7-yl)((R)-(4-((1-(3-amino-5-(trifluoromethyl)phenyl)ethyl)amino)-6-(ethylamino)-2-methylquinazolin-7-yl)(morpholino)methanone;
(*R*)-(4-((1-(3-amino-5-(triffuoromethyl)phenyl)ethyl)amino)-6-(isopropylamino)-2-methylquinazolin-7-yl)(morpholino)methanone;
(*R*)-(4-((1-(3-amino-5-(trifluoromethyl)phenyl)ethyl)amino)-2-methyl-6-((tetrahydro-2*H-*pyran-4-yl) amino)quinazolin-7-yl)(morpholino)methanone;
(*R*)-*N*⁴-(1-(3-amino-5-(trifluoromethyl)phenyl)ethyl)-N⁶,2-dimethyl-7-(morpholinomethyl)quinazoline-4,6-diamine;
(*R*)-(4-((1-(5-amino-2-fluoro-3-(trifluoromethyl)phenyl)ethyl)amino)-2-methyl-6-(methylamino)quinazoline-7-yl)(morpholino)methanone;
(*R*)-(4-((1-(3-amino-5-(difluoromethyl)phenyl)ethyl)amino)-2-methyl-6-(methylamino)quinazolin-7-yl)(morpholino)methanone;
(*R*)-(4-((1-(5-amino-3-(difluoromethyl)-2-fluorophenyl)ethyl)amino)-6-(ethylamino)-2-methylquinazoline-7-yl)(morpholino)methanone;
(*R*)-(4-((1-(3-amino-5-(trifluoromethyl)phenyl)ethyl)amino)-2-methyl-6-(methylamino)quinazolin-7-yl)(thiazolidine-3-yl)methanone;
(*R*)-(4-((1-(3-amino-5-(furan-3-yl)phenyl)ethyl)amino)-2-methyl-6-(methylamino)quinazolin-7-yl)(morpholino)methanone;
(*R*)-4-((1-(5-amino-3-(difluoromethyl)-2-fluorophenyl)ethyl)amino)-6-(isopropylamino)-2-methylquinazoline-7-(yl)(morpholino)methanone;
(*R*)-(4-((1-(5-amino-3-(difluoromethyl)-2-fluorophenyl)ethyl)amino)-6-((2-methoxyethyl)amino)-2-methylquinazolin-7-yl)(morpholino)methanone;
(*R*)-(4-((1-(3-amino-5-(trifluoromethyl)phenyl)ethyl)amino)-2-methyl-6-(methylamino)quinazoline-7-yl)(4-methylpiperazin-1-yl)methanone;
(4-(((*R*)-1-(3-amino-5-(trifluoromethyl)phenyl)ethyl)amino)-2-methyl-6-(methylamino)quinazolin-7-yl)(tetrahydro-1*H*-furo[3,4-c]pyrrol-5(3*H*)-yl)methanone;
(*R*)-(4-((1-(3-amino-5-(trifluoromethyl)phenyl)ethyl)amino)-2-methyl-6-(methylamino)quinazolin-7-yl)(1,1-dioxothiomorpholino)methanone;
(*R*)-(4-((1-(3-amino-5-(trifluoromethyl)phenyl)ethyl)amino)-2-methyl-6-(methylamino)quinazolin-7-yl)(thiomorpholino)methanone;
(*R*)-(4-((1-(3-amino-5-(trifluoromethyl)phenyl)ethyl)amino)-2-methyl-6-(methylamino)quinazolin-7-yl)(piperazine-1-yl)methanone;
(*R*)-(4-((1-(3-amino-5-(trifluoromethyl)phenyl)ethyl)amino)-2-methyl-6-(methylamino)quinazolin-7-yl)(azetidine-1-yl)methanone;
(4-(((*R*)-1-(5-amino-3-(difluoromethyl)-2-fluorophenyl)ethyl)amino)-2-methyl-6-(methylamino)quinazoline-7-yl)(tetrahydro-1*H*-furo[3,4-c]pyrrol-5(3*H*)-yl)methanone;
(*R*)-(4-((1-(5-amino-3-(difluoromethyl)-2-fluorophenyl)ethyl)amino)-2-methyl-6-(methylamino)quinazoline-7-yl)(1,1-dioxothiomorpholino)methanone;
(*R*)-(4-((1-(3-amino-5-methylphenyl)ethyl)amino)-2-methyl-6-(methylamino)quinazolin-7-yl)(morpholino)methanone;
(4-(((*R*)-1-(5-amino-3-(diffuoromethyl)-2-fluorophenyl)ethyl)amino)-6-((2-methoxyethyl)amino)-2-methylquinazolin-7-yl)(tetrahydro-1*H*-furo[3,4-c]pyrrol-5(3*H*)-yl)methanone;
(*R*)-(4-((1-(5-amino-3-(difluoromethyl)-2-fluorophenyl)ethyl)amino)-6-((2-methoxyethyl)amino)-2-methylquinazolin-7-yl)(1,1-dioxothiomorpholino)methanone;
(*R*)-(4-((1-(3-amino-5-(trifluoromethyl)phenyl)ethyl)amino)-6-((2-methoxyethyl)amino)-2-methylquinazoline-7-yl)(1,1-dioxothiomorpholino)methanone; and
(*R*)-*N*⁴-(1-(3-amino-5-(trifluoromethyl)phenyl)ethyl)-6-methoxy-2-methyl-N⁷-(tetrahydro-2H pyran-4-yl)quinazoline-4,7-diamine.

In the present invention, a method for preparing the compound of Chemical Formula 1 is not particularly limited. For example, the compound of Chemical Formula 1 may be synthesized by the preparation method of Reaction Formula 1 or Reaction Formula 2 below:

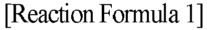

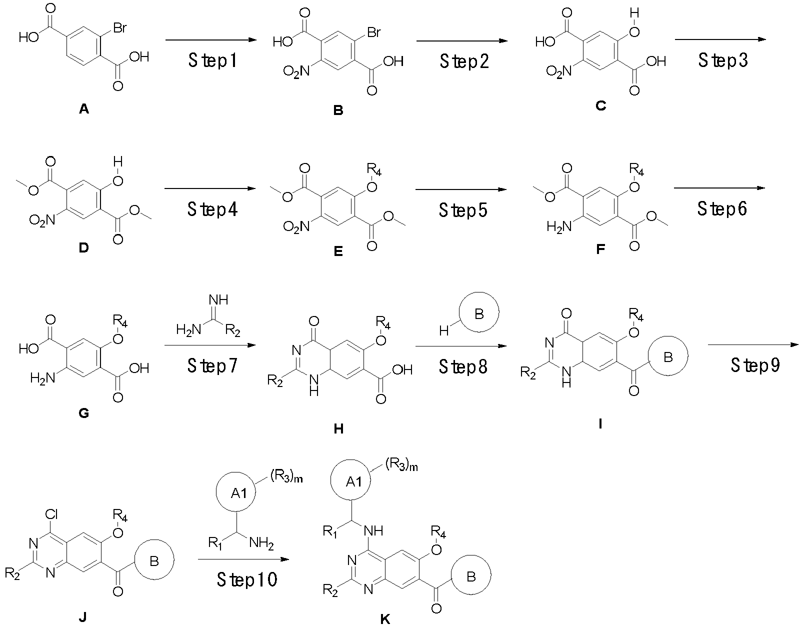

In Reaction Formula 1, 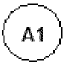 , R₁, R₂, R₃, R₄, m, and 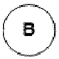 are the same as defined in Chemical Formula 1 but are not limited thereto.

### [Step-1]

2-bromoterephthalic acid (1 equivalent) was slowly added dropwise to sulfuric acid at -5°C to 5°C and refluxed for 4 to 6 minutes. After mixing sulfuric acid and nitric acid, the mixture was slowly added dropwise to the reaction product at a temperature in the range of 0°C to 5°C. After the dropwise addition was completed, the solution was refluxed at 95°C to 110°C for 1 to 3 hours. After the reaction was completed, the resultant was cooled to room temperature and refluxed at room temperature for 11 to 13 hours. Ice water was slowly added dropwise to the reaction product. The reaction product was extracted with ethyl acetate, dried over anhydrous sodium sulfate, and then concentrated under reduced pressure. The resulting solution was concentrated under reduced pressure to produce a target compound.

### [Step-2]

B (1 equivalent) prepared in [Step-1], sodium acetate (2.2 equivalents), sodium hydroxide (3 equivalents), and copper (0.01 equivalent) were dissolved in distilled water, and the resulting solution was stirred and refluxed at 110°C to 130°C under microwave for 1.5 to 3 hours. After the reaction was completed, the solution was cooled to room temperature, filtered through a celite-filled filter, and washed with water. The aqueous layer obtained through the filtration was acidified with 6N hydrochloric acid until becoming pH 1-2. The acidified aqueous solution was extracted three times with dichloromethane, and the organic layer was dried over anhydrous sodium sulfate and filtered under reduced pressure. The filtered organic layer was concentrated under reduced pressure to obtain a target compound.

### [Step-3]

C (1 equivalent) prepared in [Step-2] and sulfuric acid (1 equivalent) were dissolved in methanol and stirred and refluxed at 65°C to 75°C for 60 to 70 hours. After the reaction was completed, the reaction solution was cooled to room temperature and concentrated under reduced pressure. The concentrated solution was extracted three times with ethyl acetate, and the organic layer was dried over anhydrous sodium sulfate and filtered under reduced pressure. The filtered organic layer was concentrated under reduced pressure to obtain a target compound.

### [Step-4]

D (1 equivalent) obtained in [Step-3], methyl iodide (8 equivalents), and potassium carbonate (8 equivalents) were dissolved in acetone, and the mixture was stirred and refluxed at 50°C to 70°C for 16 to 24 hours. After the reaction was completed, the reaction solution was cooled to room temperature and concentrated under reduced pressure. The obtained residue was dissolved in distilled water and ethyl acetate and extracted three times with ethyl acetate to produce an organic layer. The obtained organic layer was dried over anhydrous sodium sulfate. The dried organic layer was filtered under reduced pressure, and the organic layer was concentrated under reduced pressure. The obtained residue was purified by MPLC to obtain a target compound.

### [Step-5]

E (1 equivalent) prepared in [Step-4] was dissolved in a mixed solution of ethyl acetate and ethanol, and Pd/C was added thereto. The reaction solution was stirred at 45°C to 55°C in a hydrogen gas ambient for 16 to 24 hours. After the reaction was completed, the reaction solution was filtered through a celite-filled filter and washed with water. The filtered organic layer was concentrated under reduced pressure, and the obtained residue was purified by MPLC to obtain a target compound.

### [Step-6]

F (1 equivalent) obtained in [Step-5] was dissolved in tetrahydrofuran, and 4% potassium hydroxide was slowly added dropwise thereto. This was stirred at 65°C to 75°C for 2-4 hours. After the reaction was completed, the reaction solution was concentrated under reduced pressure so that an organic layer was removed and an aqueous layer was obtained. The aqueous layer was acidified with 6N hydrochloric acid to become pH 1-2, and as a result, a solid product was obtained. The obtained solid product was filtered under reduced pressure, and the solid remaining through the filtration was washed with distilled water. The solid resulting through the filtration was dried in an oven dryer at 50°C to 60°C to obtain a target compound.

### [Step-7]

G (1 equivalent) obtained in [Step-6], acetamidine hydrochloride (2 equivalents), and sodium acetate (2 equivalents) were dissolved in 2-methoxyethanol, and the solution was stirred and refluxed at 140°C to 160°C for 12 to 20 hours. After the reaction was completed, the reaction solution was cooled to room temperature, distilled water was added dropwise thereto, and the resulting solution was stirred at 0°C to 5°C for 0.5 to 1 hour to obtain a solid product. The obtained solid product was filtered under reduced pressure, and the solid resulting through the filtration was washed with distilled water. The solid resulting through the filtration was dried in an oven dryer at 50°C to 60°C to obtain a target compound.

### [Step-8]

H (1 equivalent) obtained in [Step-7], amine (1.5 equivalents), HATU (3 equivalents), and DIPEA (5 equivalents) were dissolved in DMF, and the resulting solution was stirred under reflux at room temperature for 2 to 3 hours. After the reaction was completed, the reaction solution was cooled to room temperature to obtain a solid product. The obtained solid product was filtered under reduced pressure, and the solid resulting through the filtration was washed with distilled water to obtain a target compound.

### [Step-9]

I (1 equivalent) obtained in [Step-8] was dissolved in phosphoryl chloride and refluxed at 105°C to 114°C for 1 to 2.5 hours. After the reaction was completed, the reaction solution was cooled to room temperature and neutralized by dropwise addition of a sodium bicarbonate aqueous solution. The resultant was extracted three times with ethyl acetate, dried over anhydrous sodium sulfate, and concentrated under reduced pressure. The obtained residue was purified by MPLC to obtain a target compound.

### [Step-10]

J (1 equivalent) obtained in [Step-9], aniline (1.1 equivalents), and DIPEA (4 equivalents) were dissolved in DMF, and the solution was stirred under reflux at 95°C to 110°C for 12 to 15 hours. After the reaction was completed, the reaction solution was cooled to room temperature, followed by dropwise addition of water. The resultant was extracted three times with ethyl acetate, dried over anhydrous sodium sulfate, and concentrated under reduced pressure. The residue was purified by column chromatography to obtain a target compound.

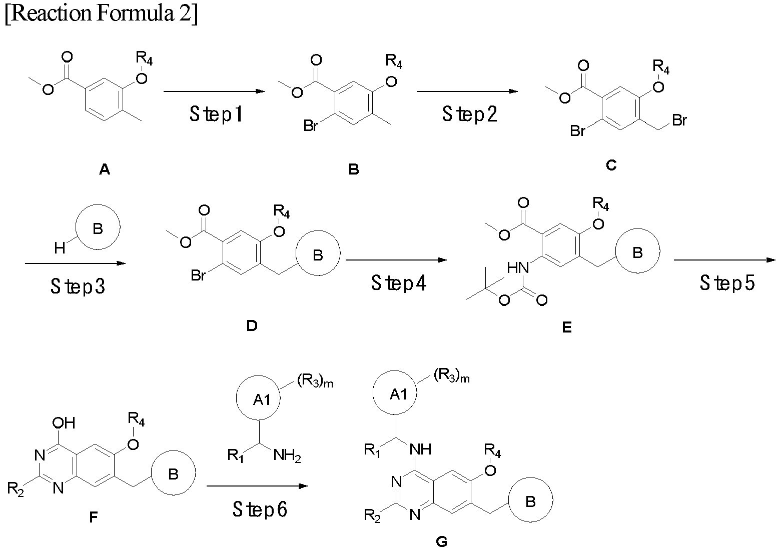

In Reaction Formula 2, 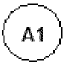 , R₁, R₂, R₃, R₄, m, and 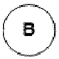 are the same as defined in Chemical Formula 1 but are not limited thereto.

### [Step-1]

Methyl 3-methoxy-4-methylbenzoate (1 equivalent) was mixed with acetic acid and water, and then bromine (1.1 equivalents) was added dropwise thereto. After the dropwise addition was completed, the solution was refluxed at 50°C to 60°C for 1 to 2 hours. After the reaction was completed, the resultant was cooled to room temperature, and a sodium bicarbonate aqueous solution was added dropwise thereto. The aqueous solution was extracted with a hexane/ether solution, and an organic layer was dried over anhydrous sodium sulfate and filtered under reduced pressure. The filtered solution was concentrated under reduced pressure to obtain a target compound.

### [Step-2]

B (1 equivalent) obtained in [Step-1], N-bromosuccinimide (0.9 equivalent), and azobisisobutyronitrile (0.2 equivalent) were dissolved in chloroform, and refluxed and stirred at 65°C to 70°C for 1.5 to 3 hours. After the reaction was completed, the resultant was cooled to room temperature, and a sodium bicarbonate aqueous solution was added dropwise thereto. After extraction with ethyl acetate, the obtained organic layer was dried over anhydrous sodium sulfate. The dried organic layer was filtered under reduced pressure, and the organic layer was concentrated under reduced pressure. The obtained residue was purified by MPLC to obtain a target compound.

### [Step-3]

C (1 equivalent) obtained in [Step-2], amine (1.1 equivalent), and potassium carbonate (2 equivalents) were dissolved in acetonitrile and stirred at 20°C to 30°C for 17 to 20 hours. After the reaction was completed, the resultant was cooled to room temperature, and a sodium bicarbonate aqueous solution was added dropwise thereto. After extraction with ethyl acetate, the obtained organic layer was dried over anhydrous sodium sulfate. The dried organic layer was filtered under reduced pressure, and the organic layer was concentrated under reduced pressure. The obtained residue was purified by MPLC to obtain a target compound.

### [Step-4]

D (1 equivalent)obtained in [Step-3], tert-butyl carbamate (1.1 equivalent), xantphos (0.2 equivalent), Pd₂(dba)₃dba (0.1 equivalent), and cesium carbonate (3 equivalents) were dissolved in 1,4-dioxane and stirred at 100°C to 120°C for 1 to 3 hours. After the reaction was completed, the resultant was cooled to room temperature, filtered through a celite-filled filter, and washed with ethyl acetate. The filtered organic layer was concentrated under reduced pressure, and the obtained residue was purified by MPLC to obtain a target compound.

### [Step-5]

E (1 equivalent) obtained in [Step-4] was dissolved in acetonitrile, and 4N hydrogen chloride dioxane solution was added dropwise thereto. Stirring and reflux were performed 70°C to 90°C for 1 to 3 hours. After the reaction was completed, the resultant was cooled to room temperature, and a sodium bicarbonate aqueous solution was added dropwise thereto for neutralization. The reaction product was extracted with anhydrous sodium sulfate, dried, and then concentrated under reduced pressure. The residue was solidified with ethyl acetate and filtered under reduced pressure. The solid resulting through the filtration was dried to obtain a target compound.

### [Step-6]

F (1 equivalent)obtained in [Step-5] above, aniline (1.5 equivalents), PyBOP (1.5 equivalents), and DBU (2.5 equivalents) were dissolved in acetonitrile and stirred at 75°C to 85°C for 4-6 hours. After the reaction was completed, the reaction solution was cooled to room temperature, followed by dropwise addition of water. The resultant was extracted three times with ethyl acetate, dried over anhydrous sodium sulfate, and concentrated under reduced pressure. The residue was purified by column chromatography to obtain a target compound.

In the present invention, a method for preparing the compound of Chemical Formula 1 is not particularly limited. For example, the compound of Chemical Formula 1 may be synthesized by the preparation method represented by Reaction Formula 3 below:

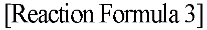

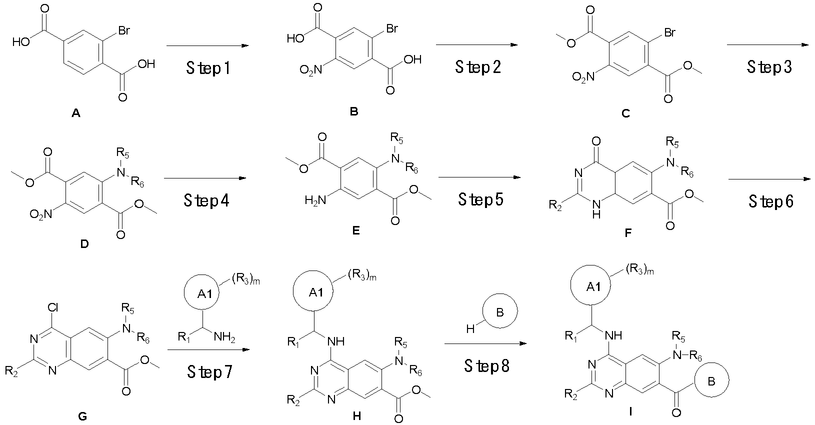

In Reaction Formula 3, 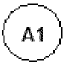 , R₁, R₂, R₃, R5, m, and 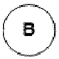 are the same as defined in Chemical Formula 1 but are not limited thereto.

### [Step-1]

2-bromoterephthalic acid (1 equivalent) was slowly added dropwise to sulfuric acid at -5°C to 5°C and refluxed for 4 to 6 minutes. After mixing sulfuric acid and nitric acid, the mixture was slowly added dropwise to the reaction product at a temperature in the range of 0°C to 5°C. After the dropwise addition was completed, the solution was refluxed at 95°C to 110°C for 1 to 3 hours. After the reaction was completed, the resultant was cooled to room temperature and refluxed at room temperature for 11 to 13 hours. Ice water was slowly added dropwise to the reaction product. The reaction product was extracted three times with ethyl acetate, dried over anhydrous sodium sulfate, and then concentrated under reduced pressure. The resulting solution was concentrated under reduced pressure to produce a target compound.

### [Step-2]

B (1 equivalent) prepared in [Step-1] above and sulfuric acid (1 equivalent) were dissolved in methanol and stirred and refluxed at 65°C to 75°C for 60 to 70 hours. After the reaction was completed, the reaction solution was cooled to room temperature and concentrated under reduced pressure. The concentrated solution was extracted three times with ethyl acetate, and the organic layer was dried over anhydrous sodium sulfate and filtered under reduced pressure. The filtered organic layer was concentrated under reduced pressure to obtain a target compound.

### [Step-3]

C (1 equivalent) prepared in [Step-2], amine (5 equivalents), and DIPEA (10 equivalents) were dissolved in DMF and stirred and refluxed at 95°C to 105°C for 1 to 2 hours. After the reaction was completed, the reaction solution was cooled to room temperature and added dropwise to distilled water. The resultant was filtered under reduced pressure to obtain a target compound.

### [Step-4]

D (1 equivalent) obtained in [Step-3] and zinc dust (3.5 equivalents) were dissolved in a mixed solution of dioxane and distilled water, and the mixture was stirred under reflux at 25°C to 30°C for 0.5 to 1 hour. After the stirring, the reaction solution was cooled to 0°C to 5°C, and ammonium chloride (5 equivalents) was added dropwise. After the dropwise addition, the mixture was stirred under reflux at 25°C to 30°C for 1-3 hours. After the reaction was completed, the reaction solution was filtered through a celite-filled filter and washed with ethyl acetate. The obtained product was dissolved in distilled water and ethyl acetate and then extracted three times with ethyl acetate to produce an organic layer. The obtained organic layer was dried over anhydrous sodium sulfate. The dried organic layer was filtered under reduced pressure, and the organic layer was concentrated under reduced pressure. The residue was purified by column chromatography to obtain a target compound.

### [Step-5]

E (1 equivalent) prepared in [Step-4] and acetonitrile (8 equivalents) were dissolved in a 4N hydrochloric acid solution dissolved in dioxane, and the reaction solution was stirred at 85°C to 95°C for 2.5 to 3.5 hours using a sealed tube. After the reaction was completed, the reaction solution was filtered through a filter and washed with hexane. The resulting solid was neutralized with a sodium bicarbonate and filtered under reduced pressure to obtain a target compound.

### [Step-6]

F (1 equivalent) obtained in [Step-5] above was dissolved in phosphoryl chloride and stirred at 110°C to 130°C for 2-4 hours. When the reaction was completed, an organic layer was removed by concentration under reduced pressure, and the obtained residue was dissolved in dichloromethane and neutralized with a sodium bicarbonate aqueous solution at low temperature. The organic layer was washed with distilled water and dried over anhydrous sodium sulfate. The dried organic layer was filtered under reduced pressure and then concentrated under reduced pressure, and the obtained residue was purified by column chromatography to obtain a target compound.

### [Step-7]

G (1 equivalent) obtained in [Step-6] above, aniline (1.3 equivalents), and DIPEA (3 equivalents) were dissolved in DMF, followed by stirring under reflux at 85°C 100°C for 12 to 15 hours. After the reaction was completed, the reaction solution was cooled to room temperature, followed by dropwise addition of water. The resultant was extracted three times with ethyl acetate, dried over anhydrous sodium sulfate, and concentrated under reduced pressure. The residue was purified by column chromatography to obtain a target compound.

### [Step-8]

H (1 equivalent) obtained in [Step-7] was dissolved in a mixed solution of tetrahydrofuran, methanol, and water, and sodium hydroxide (5 equivalents) was added thereto. The solution was stirred under reflux at 25°C to 30°C for 1 to 3 hours. After the reaction was completed, a 2N HCl aqueous solution was added dropwise to adjust the pH to 5 to 6, and then the resultant was washed with ethyl acetate. An obtained organic layer was dried over anhydrous sodium sulfate, and concentrated under reduced pressure to obtain a target compound. The target compound that is not purified, amine (1.1 equivalents), HATU (1.3 equivalents), and DIPEA (3 equivalents) were dissolved in DMF, followed by stirring under reflux at 25°C to 30°C for 1 to 3 hours. After the reaction was completed, the reaction product was extracted three times with ethyl acetate, dried over anhydrous sodium sulfate, and then concentrated under reduced pressure. The obtained residue was purified by column chromatography to obtain a target compound.

Although the preparation method of Chemical Formula 1 has been described with specific examples, specific reaction conditions, for example, the amounts of a solvent, base, and reactant used are not limited to those described herein, and should not be construed as limiting the scope of rights.

The pharmaceutical composition of the present invention is useful for preventing or treating various diseases related to inhibition of SOS1 binding because the compound of Chemical Formula 1 contained in the pharmaceutical composition inhibits SOS1 from binding to RAS family proteins and/or RAC 1.

In one embodiment, the compound of the present invention exhibits little inhibitory action on cytochrome P450 enzyme (CYP) subtypes, thereby reducing side effects such as drug-drug interactions that may occur due to decreased activity of P450 enzyme (CYP) subtypes. Therefore, the compound of the present invention is useful for polypharmacy (multiple drug use). In a specific embodiment, the compound of the present invention has the structure of Chemical Formula 2 or Chemical Formula 3 and exhibits little inhibitory effect on cytochrome P450 enzyme subtypes. In a further specific embodiment, the compound of the present invention has the structure of Chemical Formula 4 or Chemical Formula 5 and is excellent in retaining activity against cytochrome P450 enzyme subtypes. For example, the compound of Chemical Formula 4 or 5 of the present invention exhibits the inhibition effect on the activity of the cytochrome P450 enzyme (CYP) subtypes to the extent that an IC50 value is at least 20 µM.

According to another embodiment of the present invention, there is provided a preventive or therapeutic pharmaceutical composition including the compound of Chemical Formula 1 and a pharmaceutically acceptable salt thereof as an active ingredient.

According to a further embodiment of the present invention, there is provided a pharmaceutical preparation including the above-described pharmaceutical composition.

The pharmaceutical preparation of the present invention may be in various oral dosage forms such as tablets, pills, powders, capsules, syrups, or emulsions, or in parenteral dosage forms such as injections for intramuscular, intravenous, or subcutaneous administration. Preferably, the pharmaceutical preparation may be in oral dosage forms.

In addition, the pharmaceutical preparation may be formulated according to a conventional method by adding one or more non-toxic pharmaceutically acceptable additives, for example, selected from the group consisting of carriers, adjuvants, and excipients, to the active ingredient.

Excipients that can be used in the pharmaceutical preparation of the present invention include sweeteners, binders, solvents, solubilizers, wetting agents, emulsifiers, isotonic agents, adsorbents, disintegrants, antioxidants, preservatives, lubricants, fillers, fragrances, etc., but are not limited thereto. For example, as excipients, lactose, dextrose, sucrose, mannitol, sorbitol, cellulose, glycine, silica, magnesium aluminum silicate, starch, gelatin, gum tragacanth, arginic acid, sodium alginate, methylcellulose, sodium carboxymethyl cellulose, water, ethanol, polyethylene glycol, polyvinylpyrrolidone, sodium chloride, calcium chloride, orange essence, strawberry essence, vanilla flavor, etc. may be used.

When the pharmaceutical preparation of the present invention is an oral dosage form, examples of the carrier used include cellulose, calcium silicate, com starch, lactose, sucrose, dextrose, calcium phosphate, stearic acid, magnesium stearate, calcium stearate, gelatin, talc, and the like, but are not limited thereto.

When the pharmaceutical preparation of the present invention is in the form of an injection, examples of the carrier includes water, saline, aqueous glucose solutions, pseudo-sugar solutions, alcohol, glycol, ether, oil, fatty acid, fatty acid ester, glyceride, etc., but are not limited thereto.

In order to use the compounds according to the invention as medicaments, the latter are prepared in the form of pharmaceutical preparations which, in addition to the active ingredient for oral or parenteral administration, include pharmaceutically suitable organic or inorganic inert carrier substances, for example, water, gelatin, gum Arabic, lactose, starch, vegetable oil, polyalkylene glycol, and the like. The pharmaceutical preparations may be in solid forms such as tablets, dragees, suppositories, or capsules, or in liquid forms such as solutions, suspensions, or emulsions. In addition, they optionally contain adjuvants, for example, preservatives, stabilizers, wetting agents, or emulsifying agents. They also may contain salts for altering osmotic pressure or buffers.

For parenteral administration, injection solutions or suspensions are particularly preferred.

As carrier systems, it is also possible to use surfactant adjuvants (for example, bile acid salts, animal or plant phospholipids, or mixtures thereof), and liposomes or components thereof.

For oral administration, tablets, dragees, or capsules which contain talc and/or hydrocarbon vehicles or binders are particularly suitable. For example, tablets, dragees, or capsules containing lactose, corn starch, or potato starch are preferable. It can also be administered in liquid form, for example as a juice with an added sweetening agent.

In addition, the human dose of the compound of Chemical Formula 1 according to the present invention is preferably in the range of 0.1 mg/day to 2,000 mg/day based on an adult patient weighing 70 kg in general. The compound according to the present invention may be administered in divided doses, once a day or several times a day. However, the dosage may vary depending on the patient's health condition, age, weight and sex, dosage form, and disease level, and thus the scope of the present invention is not limited to the dosage presented above.

According to a yet further embodiment of the present invention, with respect to a compound selected from the compounds of Chemical Formula 1 of the present invention, pharmaceutically acceptable salts, optical isomers, diastereomers, hydrates, and solvates thereof, or a pharmaceutically acceptable salt of the selected compound, there is provided a preventive or therapeutic use thereof for cancers or tumors.

According to a yet further embodiment of the present invention, there is provided a method for preventing or treating cancer, the method including administering, to a subject, a compound selected from the compounds of Chemical Formula 1 of the present invention, pharmaceutically acceptable salts, optical isomers, diastereomers, hydrates, and solvates thereof, or a pharmaceutically acceptable salt of the selected compound. Preferably, the subject refers to an individual or a patient, but is not limited thereto.

According to a yet further embodiment of the present invention, there is provided a method for treating cancer in a subject who requires administration of a compound inhibitor and a standard-of-care formulation, the method including administering, to the subject, a therapeutically effective amount of the standard-of-care formulation containing: a compound selected from the compounds of Chemical Formula 1 of the present invention, pharmaceutically acceptable salts, optical isomers, diastereomers, hydrates, and solvates thereof; or a pharmaceutically acceptable salt of the selected compound.

According to a yet further embodiment of the present invention, there is provided a method for treating cancer in a subject who requires administration of a composition, the method including administering, to the subject, a therapeutically effective amount of the composition containing: a compound selected from the compounds of Chemical Formula 1 of the present invention, pharmaceutically acceptable salts, optical isomers, diastereomers, hydrates, and solvates thereof; or a pharmaceutically acceptable salt of the selected compound.

According to a yet further embodiment of the present invention, there is provided a method for inhibiting binding of SOS1 to RAS family proteins and/or RAC1 in a subject or cell, the method including administering, to the subject, a compound selected from the compounds of Chemical Formula 1 of the present invention, pharmaceutically acceptable salts, optical isomers, diastereomers, hydrates, and solvates thereof, or a pharmaceutically acceptable salt of the selected compound.

According to a yet further embodiment of the present invention, there is provided a method for inhibiting tyrosine kinase in a subject or cell, the method including administering, to the subject, a compound selected from the compounds of Chemical Formula 1 of the present invention, pharmaceutically acceptable salts, optical isomers, diastereomers, hydrates, and solvates thereof, or a pharmaceutically acceptable salt of the selected compound.

According to a yet further embodiment of the present invention, there is provided a method for preventing or treating cancer in a subject, the method including administering, to the subject, a compound selected from the compounds of Chemical Formula 1 of the present invention, pharmaceutically acceptable salts, optical isomers, diastereomers, hydrates, and solvates thereof, or a pharmaceutically acceptable salt of the selected compound.

According to a yet further embodiment of the present invention, there is provided a method for preventing or treating cancer that can be prevented or treated by inhibiting binding of SOS 1 to RAS family proteins and/or RAC1 in a subject or cell, the method including administering, to the subject, a compound selected from the compounds of Chemical Formula 1 of the present invention, pharmaceutically acceptable salts, optical isomers, diastereomers, hydrates, and solvates thereof, or a pharmaceutically acceptable salt of the selected compound.

Hereinafter, the present invention will be described in more detail by way of Examples and Experimental Examples. However, Examples and Experimental Examples are presented only for helping the understanding of the present invention, and the scope of the present invention is not limited thereto in any sense.

### Example 1 (R)-(4-((1-(3-amino-5-(trifluoromethyl)phenyl)ethyl)amino)-6-methoxy-2-methylquinazoline-7-yl)(morpholino)methanone

### [Step-1] Preparation of 2-bromo-5-nitroterephthalic acid

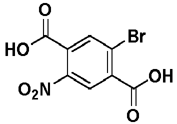

2-bromoterephthalic acid (13.8 g, 56.32 mmol) was slowly added dropwise to 78 mL of sulfuric acid at 0°C, followed by stirring for 5 minutes to prepare a reaction solution. After mixing 7.5 mL of sulfuric acid and 17.5 mL of nitric acid, the mixture was slowly added dropwise to the reaction solution at 0°C. After the dropwise addition was completed, stirring and refluxing were performed at 100°C for 2 hours. After the reaction was completed, the resultant was cooled to room temperature and stirred for 12 hours. Upon completion of the reaction, the reaction solution was slowly added dropwise to ice water. The aqueous solution was extracted three times with ethyl acetate, and an organic layer was dried over anhydrous sodium sulfate and filtered under reduced pressure. The filtered solution was concentrated under reduced pressure, and the title compound was obtained in an amount of 16 g.

¹H-NMR (300 MHz, DMSO-*d₆*): *δ* 8.34 (s, 1H), 8.17 (s, 1H).

### [Step-2] Preparation of 2-hydroxy-5-nitroterephthalic acid

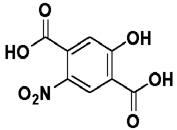

The 2-bromo-5-nitroterephthalic acid (10.5 g, 36.21 mmol) prepared in [Step-1], sodium acetate (6.6 g, 79.65 mmol), sodium hydroxide (4.35 g, 108.63 mmol), and copper (46.5 mg, 0.72 mmol) were dissolved in 60 mL of distilled water and stirred under reflux at 120°C for 2 hours under microwave. After the reaction was completed, the solution was cooled to room temperature, filtered through a celite-filled filter, and washed with water. The aqueous layer obtained through the filtration was acidified with 6N hydrochloric acid until becoming pH 1-2. The acidified aqueous solution was extracted three times with dichloromethane, and the organic layer was dried over anhydrous sodium sulfate and filtered under reduced pressure. The filtered organic layer was concentrated under reduced pressure, and 7 g of the title compound was obtained at 85% yield.

¹H-NMR (300 MHz, DMSO-*d₆*): *δ* 8.42 (s, 1H), 7.15 (s, 1H).

### [Step-3] Preparation of dimethyl 2-hydroxy-5-nitroterephthalate

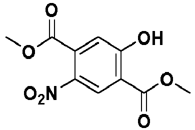

The 2-hydroxy-5-nitroterephthalic acid (7 g, 30.82 mmol) prepared in [Step-2] and sulfuric acid (35 mL, 653.04 mmol) were dissolved in 330 mL of methanol, and the mixture was stirred under reflux at 70°C for 65 hours. After the reaction was completed, the reaction solution was cooled to room temperature and concentrated under reduced pressure. The resultant was extracted three times with ethyl acetate, and an organic layer was dried over anhydrous sodium sulfate and filtered under reduced pressure. The filtered organic layer was concentrated under reduced pressure to obtain 7.9g of the title compound.

¹H-NMR (300 MHz, DMSO-*d₆*): *δ* 8.43 (s, 1H), 6.85 (s, 1H), 3.83 (s, 1H).

### [Step-4] Preparation of dimethyl 2-methoxy-5-nitroterephthalate

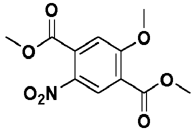

The dimethyl 2-hydroxy-5-nitroterephthalate (7.9 g, 30.82 mmol) obtained in [Step-3], methyl iodide (15.3 mL, 246.56 mmol), and potassium carbonate (34 g, 246.56 mmol) were dissolved in 310 mL of acetone and stirred under reflux at 60°C for 21 hours. After the reaction was completed, the reaction solution was cooled to room temperature and concentrated under reduced pressure. The obtained residue was dissolved in a mixed solution of distilled water and ethyl acetate (1:1 volume ratio) and extracted three times with ethyl acetate to produce an organic layer. The obtained organic layer was dried over anhydrous sodium sulfate. The dried organic layer was filtered under reduced pressure, and the organic layer was concentrated under reduced pressure. The obtained residue was purified by MPLC (ethyl acetate:hexane = 1:5 (v/v) to 1:1 (v/v)) to obtain 6.1 g of the title compound at 73% yield.

¹H-NMR (300 MHz, CDCl₃): *δ* 8.57 (s, 1H), 7.15 (s, 1H), 4.05 (s, 3H), 3.98 (s, 3H), 3.96 (s, 3H).

### [Step-5] Preparation of dimethyl 2-amino-5-methoxy terephthalate

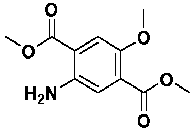

The dimethyl 2-methoxy-5-nitroterephthalate (6.1 g, 22.50 mmol) prepared in [Step-4] above was dissolved in 122 mL of a mixed solution of ethyl acetate and ethanol (1:4 vol/vol), and Pd/C (600 mg, 10 wt%) was added thereto. This reaction solution was stirred at 50°C in a hydrogen gas ambient for 20 hours. After the reaction was completed, the reaction solution was filtered through a celite-filled filter and washed with methanol. The resulting organic layer was concentrated under reduced pressure, and the obtained residue was purified by MPLC (ethyl acetate:hexane =1:5 (v/v) to 1:1 (v/v)) to obtain 5 g of the title compound at 93% yield.

¹H-NMR (300 MHz, CDCl₃): *δ* 7.38 (s, 1H), 7.05 (s, 1H), 5.50 (s, 2H), 3.86 (s, 6H), 3.80 (s, 3H).

### [Step-6] Preparation of 2-amino-5-methoxyterephthalic acid

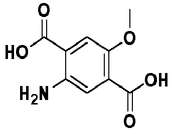

The dimethyl 2-amino-5-methoxyterephthalate (5 g, 20.93 mmol) obtained in [Step-5] was dissolved in 115 mL of tetrahydrofuran, and 115 mL of 4% potassium hydroxide was slowly added dropwise. This was stirred at 70°C for 3 hours. After the reaction was completed, the reaction solution was concentrated under reduced pressure so that an organic layer was removed and an aqueous layer was obtained. The aqueous layer was acidified with 2N hydrochloric acid until becoming pH 1 to 2, and as a result, a solid product was obtained. The obtained solid product was filtered under reduced pressure, and the solid resulting through the filtration was washed with distilled water. The solid resulting through the filtration was dried in an oven dryer at 55°C to obtain 4.3 g of the title compound at 98% yield.

¹H-NMR (300 MHz, DMSO-*d₆*): *δ* 7.30 (s, 1H), 7.01 (s, 1H), 3.70 (s, 3H).

### [Step-7] Preparation of 6-methoxy-2-methyl-4-oxo-1,4-dihydroquinazoline-7-carboxylic acid

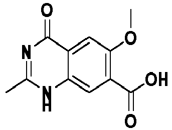

The 2-bromo-5-methoxyterephthalic acid (4.3 g, 20.51 mmol) prepared in [Step-6], acetamide hydrochloride (3.86 g, 41.02 mmol), and sodium acetate (3.38 mg, 40.77 mmol) were dissolved in 86 mL of methoxy ethanol and stirred under reflux at 150°C for 15 hours. After the reaction was completed, the reaction solution was cooled to room temperature, distilled water was added dropwise thereto, and the resulting solution was stirred at 0°C for 0.5 hours to obtain a solid product. The obtained solid product was filtered under reduced pressure, and the solid resulting through the filtration was washed with distilled water. The solid resulting through the filtration was dried in an oven dryer at 55°C to obtain 3.3 g of the title compound at 69% yield.

¹H-NMR (300 MHz, DMSO-*d₆*): *δ* 13.19 (s, 1H), 12.26 (s, 1H), 7.70 (s, 1H), 7.57 (s, 1H), 3.90 (s, 3H), 2.34 (s, 3H).

### [Step-8] Preparation of 6-methoxy-2-methyl-7-(morpholine-4-carbonyl)quinazoline-4(1H)-one;

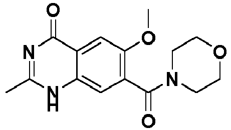

The 6-methoxy-2-methyl-4-oxo-1,4-dihydroquinazoline-7-carboxylic acid (300 mg, 1.28 mmol) obtained in [Step 7], morpholine (0.18 mL, 1.92 mmol), HATU (1.44 g, 3.84 mmol), and DIPEA (1.2 mL, 6.40 mmol) were dissolved in 4.5 mL of DMF, and this solution was stirred under reflux at room temperature for 2.5 hours. After the reaction was completed, the reaction solution was cooled to room temperature to obtain a solid product. The obtained solid product was filtered under reduced pressure, and the solid resulting through the filtration was washed with ethyl acetate to obtain 255 mg of the title compound at 66% yield.

¹H-NMR (300 MHz, DMSO-*d₆*): *δ* 12.24 (s, 1H), 7.55 (s, 1H), 7.41 (s, 1H), 3.90 (s, 3H), 3.68 (m, 4H), 3.52 (m, 2H), 3.11 (m, 2H), 2.51 (s, 3H).

### [Step-9] Preparation of (4-chloro-6-methoxy-2-methylquinazoline-7-yl)(morpholine)methanone

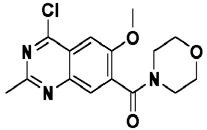

The 6-methoxy-2-methyl-7-(morpholine-4-carbonyl)quinazoline-4(1H)-one (255 mg, 0.84 mmol) obtained in [Step-8] above was dissolved in 14 mL of phosphoryl chloride, and this solution was refluxed at 110°C for 2 hours. After the reaction was completed, the resultant was cooled to room temperature, and a sodium bicarbonate aqueous solution was added dropwise thereto for neutralization. The reaction product was extracted three times with anhydrous sodium sulfate, dried, and then concentrated under reduced pressure. The residue was purified by MPLC (dichloromethane:methanol = 25:1 to 10:1 (v/v)) to obtain 221 mg of the title compound at 82% yield.

¹H-NMR (300 MHz, DMSO-*d₆*): *δ* 7.86 (s, 1H), 7.52 (s, 1H), 4.01 (s, 3H), 3.67 (m, 4H), 3.50 (m, 2H), 3.14 (m, 2H), 2.50 (s, 3H).

### [Step-10] Preparation of (R)-(4-((1-(3-amino-5-(trifluoromethyl)phenyl)ethyl)amino)-6-methoxy-2-methylquinazoline-7-yl)(morpholino)methanone

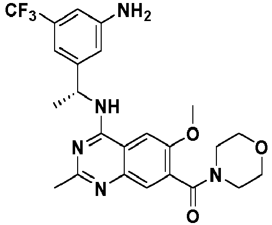

The (4-chloro-6-methoxy-2-methylquinazoline-7-yl) (morpholine)methanone (70 mg, 0.22 mmol) prepared in [Step-9], (*R*)-3-(1-aminoethyl)-5-(trifluoromethyl)aniline hydrochloride (57 mg, 0.24 mmol) synthesized by the method disclosed in WO2018115380, and DIPEA (0.15 mL, 0.88 mmol) were dissolved in 1 mL of DMF, and the mixture was stirred under reflux at 100°C for 13 hours. After the reaction was completed, the reaction solution was cooled to room temperature, followed by dropwise addition of water thereto. The resultant was extracted three times with ethyl acetate, dried over anhydrous sodium sulfate, and concentrated under reduced pressure. The residue was purified by column chromatography (dichloromethane:methanol = 23:1 (v/v)) to obtain 60 mg of the title compound at 56% yield.

¹H-NMR (300 MHz, DMSO-*d₆*): *δ* 8.29 (d, 1H), 7.85 (s, 1H), 7.40 (s, 1H), 6.90 (d, 2H), 6.71 (s, 1H), 5.62 (m, 1H), 5.57 (s, 2H), 3.94 (s, 3H), 3.50 (m, 4H), 3.37 (m, 2H), 3.11 (m, 2H), 2.50 (s, 3H), 1.58 (m, 3H).
MS (ESI+, m/z): 490.2 [M+H]⁺

### Example 2: (6-methoxy-2-methyl-4-((1-(4-(2-((methylamino)methyl)phenyl)thiophen-2-yl)ethyl)amino)quinazoline-7-yl)(morpholino)methanone

### [Step 1] Preparation of (4-((1-(4-bromothiophene-2-yl)ethyl)amino)-6-methoxy-2-methylquinazoline-7-yl)(morpholino)methanone

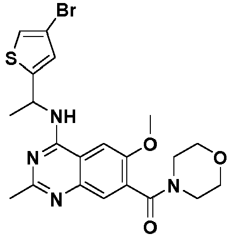

Except for the use of 1-(4-bromothiophene-2-yl)ethane-1-amine (100 mg, 0.47 mmol) instead of the (R)-3-(1-aminoethyl)-5-(triffuoromethyl)aniline hydrochloride and the use of DMAc instead of the DMF in Example 1 [Step-10], the same procedure of Example 1 was repeated to obtain 156 mg of the title compound at 68% yield.

¹H-NMR (300 MHz, DMSO-*d₆*): *δ* 8.40 (d, 1H), 7.77 (s, 1H), 7.51 (s, 1H), 7.43 (s, 1H), 7.10 (s, 1H), 5.90 (m, 1H), 3.91 (s, 3H), 3.65 (m, 4H), 3.48 (m, 2H), 3.11 (m, 2H), 2.49 (m, 3H), 1.72 (m, 3H).

### [Step 2] Preparation of 2-(5-(1-((6-methoxy-2-methyl-7-(morpholine-4-carbonyl)quinazoline-4-yl)amino)ethyl)thiophene-3-yl)benzaldehyde

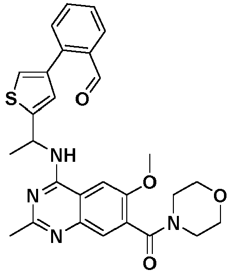

The (4-((1-(4-bromothiophene-2-yl)ethyl)amino)-6-methoxy-2-methylquinazoline-7-yl)(morpholino)methanone (156 mg, 0.32 mmol) prepared in [Step-1] above, (2-formylphenyl)boronic acid (57 mg, 0.38 mmol), Pd(PPh₃)₄ (40 mg, 0.03 mmol), and potassium carbonate (177 mg, 1.28 mmol) were dissolved in 3 mL of a mixed solution of dioxane and water (5:1), and the solution was stirred at 100°C for 5 hours. After the reaction was completed, the resultant was cooled to room temperature, filtered through a celite-filled filter, and washed with dichloromethane. After dropwise addition of water to the collected organic layers, extraction was performed with dichloromethane three times, and the resultant was dried over anhydrous sodium sulfate and concentrated under reduced pressure. The residue was purified by MPLC (dichloromethane:methanol = 100:1 to 10:1 (v/v)) to obtain 143 mg of the title compound at 88% yield.

¹H-NMR(300 MHz, DMSO-*d₆*): *δ* 10.08 (s, 1H), 8.40 (m, 1H), 7.90 (d, 1H), 7.87 (s, 1H), 7.73 (m, 1H), 7.70 (m, 3H), 7.51 (s, 1H), 7.32 (s, 1H), 6.00 (m, 1H), 4.09 (s, 3H), 3.65 (m, 4H), 3.50 (m, 2H), 3.11 (m, 2H), 2.49 (m, 3H), 1.79 (m, 3H).

### [Step 3] Preparation of (6-methoxy-2-methyl-4-((1-(4-(2-((methylamino)methyl)phenyl)thiophene-2-yl)ethyl)amino)quinazoline-7-yl)(morpholino)methanone

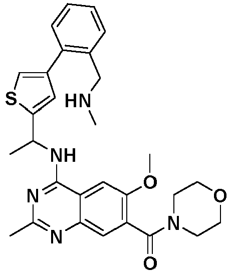

The 2-(5-(1-((6-methoxy-2-methyl-7-(morpholine-4-carbonyl)quinazoline-4-yl)amino)ethyl)thiophene-3-yl)benzaldehyde (143 mg, 0.28 mmol) prepared in [Step-2] above, 2.0M methylamine (0.3 mL, 0.55 mmol), acetic acid (0.03 mL, 0.55 mmol), and sodium triacetoxyborohydride (117 mg, 0.55 mmol) were dissolved in 2 mL of dichloroethane, and this solution was stirred at room temperature for 16 hours. After the reaction was completed, the reaction solution was cooled to room temperature and neutralized by dropwise addition of a sodium bicarbonate aqueous solution. The resultant was extracted with ethyl acetate three times, dried over anhydrous sodium sulfate, and concentrated under reduced pressure. The residue was purified by MPLC (dichloromethane:methanol = 50:1 to 7:1 (v/v)) to obtain 80 mg of the title compound at 56% yield.

¹H-NMR (300 MHz, DMSO-*d₆*): *δ* 8.45 (m, 1H), 7.81 (s, 1H), 7.48 (m, 3H), 7.29 (m, 4H), 6.00 (m, 1H), 4.09 (m, 3H), 3.65 (m, 4H), 3.58 (m, 2H), 3.50 (m, 2H), 3.11 (m, 2H), 2.51 (m, 3H), 2.23 (s, 3H), 1.94 (m, 1H), 1.71 (m, 3H).
MS (ESI+, m/z): 532.2 [M+H]⁺

### Example 3: (4-(((R)-1-(3-amino-5-(trifluoromethyl)phenyl)ethyl)amino)-6-methoxy-2-methylquinazoline-7-yl)((3R, 5S)-3,5-dimethylpiperazin-1-yl)methanone

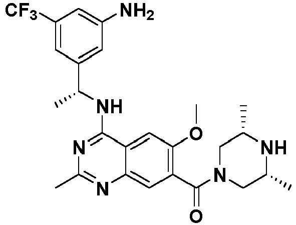

Except for the use of cis-2,6-dimethylpiperazine (270 mg, 2.34 mmol) instead of the morpholine in Example 1 [Step-8], the same procedure of Example 1 was repeated to obtain 14 mg of the title compound at 34% yield.

¹H-NMR (300 MHz, DMSO-*d₆*): *δ* 8.28 (d, 1H), 7.84 (m, 1H), 7.36 (d, 1H), 6.93 (m, 2H), 6.72 (s, 1H), 5.62 (m, 3H), 4.41 (m, 1H), 3.92 (s, 3H), 3.07 (m, 1H), 2.68 (m, 3H), 2.40 (d, 3H), 2.28 (m, 3H), 1.60 (m, 3H), 1.03 (d, 3H), 0.82 (m, 3H).
MS (ESI+, m/z): 517.3 [M+H]⁺

### Example 4: (R)-(4-((1-(3-amino-5-(trifluoromethyl)phenyl)ethyl)amino)-6-methoxy-2-methylquinazoline-7-yl)(thiomorpholino)methanone

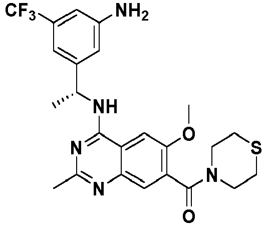

Except for the use of thiomorpholine (0.24 mL, 2.56 mmol) instead of the morpholine in Example 1 [Step-8], the same procedure of Example 1 was repeated to obtain 60 mg of the title compound at 56% yield.

¹H-NMR (300 MHz, DMSO-*d₆*): *δ* 8.27 (d, 1H), 7.85 (d, 1H), 7.41 (s, 1H), 6.88 (m, 2H), 6.71 (s, 1H), 5.59 (m, 3H), 3.88 (m, 5H), 3.36 (m, 2H), 2.73 (m, 2H), 2.50 (m, 2H), 2.38 (s, 3H), 1.58 (m, 3H).
MS (ESI+, m/z): 506.2 [M+H]⁺

### Example 5: (4-(((R)-1-(3-amino-5-(trifluoromethyl)phenyl)ethyl)amino)-6-methoxy-2-methylquinazoline-7-yl)(tetrahydro-1H-furo[3,4-c]pyrrol-5(3H)-yl)methanone

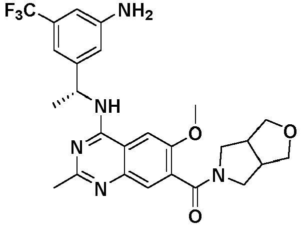

Except for the use of hexahydro-1*H*-furo[3,4-c]pyrrole (159 mg, 1.41 mmol) instead of the morpholine in Example 1 [Step-8], the same procedure of Example 1 was repeated to obtain 9.2 mg of the title compound at 9% yield.

¹H-NMR (300 MHz, CD₃OD): *δ* 7.82 (s, 1H), 7.52 (s, 1H), 7.00 (m, 2H), 6.83 (s, 1H), 5.66 (m, 1H), 4.02 (s, 3H), 3.94 (m, 2H), 3.84 (m, 2H), 3.66 (m, 2H), 3.18 (m, 4H), 2.49 (s, 3H), 1.67 (d, *J* = 6.9 Hz, 3H).
MS (ESI+, m/z): 516.2 [M+H]⁺

### Example 6: (R)-(4-((1-(3-(difluoromethyl)-2-fluorophenyl)ethyl)amino)-6-methoxy-2-methylquinazoline-7-yl)(morpholino)methanone

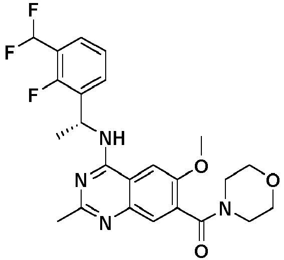

Except for the use of (*R*)-1-(3-(difluoromethyl)-2-fluorophenyl)ethan-1-amine hydrochloride (25 mg, 0.11 mmol) instead of the (R)-3-(1-aminoethyl)-5-(trifluoromethyl)aniline hydrochloride in Example 1 [Step-10], the same procedure of Example 1 was repeated to obtain 8 mg of the title compound at 17% yield.

¹H-NMR (300 MHz, DMSO-*d₆*): *δ* 8.40 (m, 1H), 7.89 (s, 1H), 7.68 (m, 1H), 7.66 (m, 1H), 7.50 (s, 1H), 7.40-7.06 (m, 2H), 5.82 (m, 1H), 3.97 (s, 3H), 3.50 (m, 4H), 3.42 (m, 2H), 3.29 (m, 2H), 2.33 (s, 3H), 1.64 (m, 3H).
MS (ESI+, m/z): 475.2 [M+H]⁺

### Example 7: (R)-(4-((1-(3-amino-5-(trifluoromethyl)phenyl)ethyl)amino)-6-methoxy-2-methylquinazoline-7-yl)(azetidine-1-yl)methanone

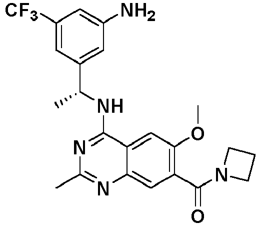

Except for the use of azetidine (0.1 mL, 1.40 mmol) instead of the morpholine in Example 1 [Step-8], the same procedure of Example 1 was repeated to obtain 28 mg of the title compound at 15% yield.

¹H-NMR (300 MHz, DMSO-*d₆*): *δ* 8.28 (d, 1H), 7.83 (s, 1H), 7.43 (s, 1H), 6.90 (d, 2H), 6.71 (s, 1H), 5.64 (m, 3H), 4.06 (m, 2H), 3.95 (s, 3H), 3.88 (m, 2H), 2.42 (s, 3H), 2.28 (m, 2H), 1.59 (d, 3H).
MS (ESI+, m/z): 460.2 [M+H]⁺

### Example 8: (lrmethoay-2-methyl-4-((1-(4-(1,2,3,4-tetrahydroisoquinolin-8-yl)thiophen-2-yl)ethyl)amino)quinazoline-7-yl)(morpholino)methanone

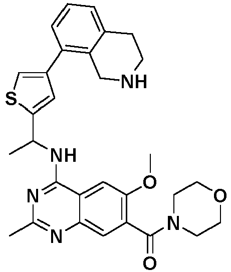

Except for the use of (2-(tert-butoxycarbonyl)-1,2,3,4-tetrahydroisoquinoline-8-yl)boronic acid (74 mg, 0.27 mmol) instead of the (2-formylphenyl)boronic acid in Example 2 [Step-2], the same procedure of Example 2 was repeated to obtain 30 mg of the title compound at 48% yield.

¹H-NMR (300 MHz, CD₃OD): *δ* 7.77 (s, 1H), 7.54 (s, 1H), 7.13 (m, 5H), 6.00 (m, 1H), 4.13 (s, 3H), 3.82 (m, 4H), 3.61 (m, 2H), 3.32 (m, 2H), 3.13 (m, 2H), 2.87 (m, 4H), 2.57 (s, 3H), 1.83 (m, 3H).
MS (ESI+, m/z): 544.2 [M+H]⁺

### Example 9: (R)-(4-((1-(3-amino-5-(trifluoromethyl)phenyl)ethyl)amino)-6-methoxy-2-methylquinazoline-7-yl)(piperazine-1-yl)methanone

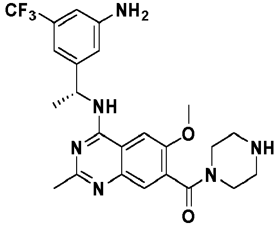

Except for the use of piperazine (26 mg, 0.41 mmol) instead of the morpholine in Example 1 [Step-8], the same procedure of Example 1 was repeated to obtain 5 mg of the title compound at 9% yield.

¹H-NMR (300 MHz, CD₃OD): *δ* 8.09 (s, 1H), 7.61 (s, 1H), 6.98 (m, 2H), 6.84 (s, 2H), 5.79 (m, 1H), 4.06 (m, 5H), 3.54 (m, 2H), 3.34 (m, 2H), 3.20 (m, 2H), 2.65 (s, 3H), 1.74 (d, 3H).
MS (ESI+, m/z): 489.2 [M+H]⁺

### Example 10: (R)-2,2,2-trifluoro-N-(3-(1-((6-methoxy-2-methyl-7-(morpholine-4-carbonyl)quinazoline-4-yl)amino)ethyl)-5-(trifluoromethyl)phenylacetamide

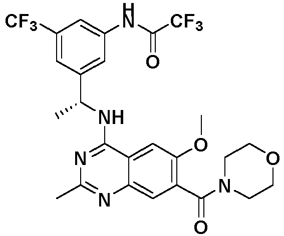

The (R)-(4-((1-(3-amino-5-(trifluoromethyl)phenyl)ethyl)amino)-6-methoxy-2-methylquinazoline-7-yl)(morpholino)methanone (30 mg, 0.061 mmol) prepared in Example 1 [Step-1], trifluoroacetic anhydride (14 mg, 0.067 mmol), and DIPEA (10 mg, 0.078 mmol) were dissolved in 1 mL of dichloromethane, and the solution was stirred at room temperature for 5 hours. After the reaction was completed, an ammonium chloride aqueous solution was added dropwise thereto so that the reaction solution was neutralized. Extraction with dichloromethane was performed three times, and the resultant was dried over anhydrous sodium sulfate and concentrated under reduced pressure. The residue was purified by MPLC (dichloromethane:methanol = 50:1 to 10:1 (v/v)) to obtain 7 mg of the title compound at 20% yield.

¹H-NMR (300 MHz, CD₃OD): *δ* 8.11-8.08 (d, 1H), 7.91-7.88 (d, 1H), 7.88 (s, 1H), 7.66 (s, 1H), 7.52 (s, 1H), 5.74 (m, 1H), 4.86 (s, 6H), 4.03 (s, 3H), 3.79 (m, 4H), 3.63 (m, 2H), 3.32 (m, 2H), 2.47 (s, 3H), 1.76 (d, 3H).
MS (ESI+, m/z): 586.2 [M+H]⁺

### Example 11: (R)-(4-((1-(3-amino-5-(trifluoromethyl)phenyl)ethyl)amino)-6-methoxy-2-methylquinazoline-7-yl)(3-fluorazetidine-1-yl)methanone

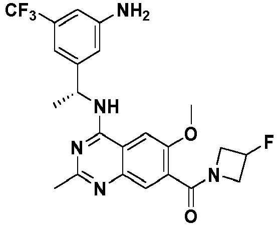

Except for the use of 3-fluoroazetidine (258 mg, 1.07 mmol) instead of the morpholine in Example 1 [Step-8], the same procedure of Example 1 was repeated to obtain 30 mg of the title compound at 9% yield.

¹H-NMR (300 MHz, CD₃OD): *δ* 8.03 (s, 1H), 7.64 (s, 1H), 7.00 (m, 2H), 6.85 (s, 1H), 5.78 (m, 1H), 5.34 (m, 1H), 4.51 (m, 1H), 4.28 (m, 3H), 4.08 (s, 3H), 2.63 (s, 3H), 1.74 (d, 3H)
MS (ESI+, m/z): 478.2 [M+H]⁺

### Example 12: (4-((1-(4-(2-((dimethylamino)methyl)phenyl)thiophen-2-yl)ethyl)amino)-6-methoxy-2-methoxyquinazoline-7-yl)(morpholino)methanone

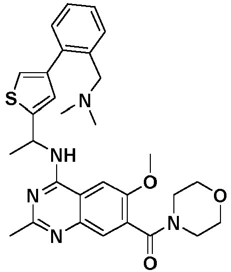

Except for the use 2.0M dimethylamine (0.16 ml, 0.32 mmol) instead of the 2.0M methylamine in Example 2 [Step-3], the same procedure of Example 2 was repeated to obtain 307 mg of the title compound at 8% yield.

¹H-NMR (300 MHz, CD₃OD): *δ* 7.77 (s, 1H), 7.49 (m, 2H), 7.39 (m, 3H), 7.26 (s, 1H), 7.17 (s, 1H), 6.09 (m, 1H), 3.97 (m, 5H), 3.77 (m, 4H), 3.62 (m, 2H), 3.26 (m, 2H), 2.55 (s, 3H), 2.38 (s, 6H), 1.84 (d, 3H)
MS (ESI+, m/z): 546.2 [M+H]⁺

### Example 13: (4-((1-(4-(2-((aminomethyl)phenyl)thiophen-2-yl)ethyl)amino)-6-methoxy-2-methylquinazoline-7-yl)(morpholino)methanone

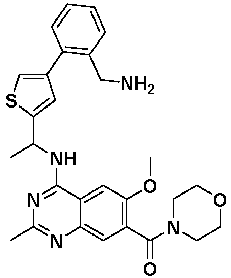

Except for the use 2.0M ammonia (0.22 ml, 0.45 mmol) instead of the 2.0M methylamine in Example 2 [Step-3], the same procedure of Example 2 was repeated to obtain 15 mg of the title compound at 13% yield.

¹H-NMR (300 MHz, CD₃OD): *δ* 7.78 (s, 1H), 7.52 (m, 2H), 7.30 (m, 5H), 6.10 (m, 1H), 4.57 (s, 2H), 3.98 (s, 3H), 3.76 (m, 4H), 3.62 (m, 2H), 3.26 (m, 2H), 2.56 (s, 3H), 1.82 (d, 3H)
MS (ESI+, m/z): 518.2 [M+H]⁺

### Example 14: (4-((1-(4-(2-((hydroxymethyl)phenyl)thiophen-2-yl)ethyl)amino)-6-methoxy-2-methylquinazoline-7-yl)(morpholino)methanone

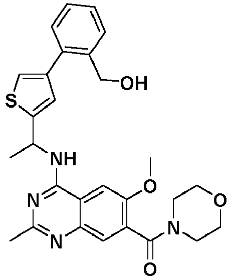

Except for the use 2-(hydroxymethyl)phenylboronic acid (0.14 ml, 0.92 mmol) instead of the (2-formylphenyl)boronic acid in Example 2 [Step-2], the same procedure of Example 2 was repeated to obtain 88 mg of the title compound at 20% yield.

¹H-NMR (300 MHz, CD₃OD): *δ* 7.76 (s, 1H), 7.51 (m, 2H), 7.31 (m, 5H), 6.10 (m, 1H), 4.57 (s, 2H), 3.98 (s, 3H), 3.76 (m, 4H), 3.60 (m, 2H), 3.27 (m, 2H), 2.55 (s, 3H), 1.24 (d, 3H)
MS (ESI+, m/z): 519.2 [M+H]⁺

### Example 15: (R)-(6-methoxy-2-methyl-4-((1-(3-(trifluoromethyl)phenyl)ethyl)amino)quinazolin-7-yl)(morpholino)methanone

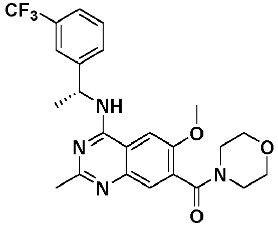

Except for the use of (*R*)-1-(3-(triffuoromethyl)phenyl)ethane-1-amine hydrochloride (33 mg, 0.16 mmol) instead of the (R)-3-(1-aminoethyl)-5-(trifluoromethyl)aniline hydrochloride in Example 1 [Step-10], the same procedure of Example 1 was repeated to obtain 20 mg of the title compound at 27% yield.

¹H-NMR (300 MHz, DMSO-*d₆*): *δ* 8.38 (d, 1H), 7.85 (s, 2H), 7.78 (d, 1H), 7.60 (d, 2H), 7.41 (s, 1H), 5.74 (m, 1H), 3.96 (s, 3H), 3.65 (m, 4H), 3.49 (m, 2H), 3.12 (m, 2H), 2.37 (s, 3H), 1.67 (m,
MS (ESI+, m/z): 475.2 [M+H]⁺

### Example 16: (R)-(4-((1-(5-amino-2-methyl-3-(trifluoromethyl)phenyl)ethyl)amino)-6-methoxy-2-methylquinazolin-7-yl)(morpholino)methanone

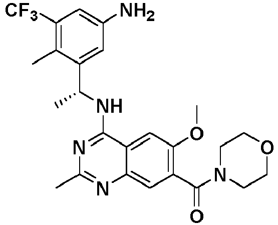

Except for the use of (R)-3-(1-aminoethyl)-4-methyl-5-(trifluoromethyl)aniline hydrochloride (83 mg, 0.31 mmol) instead of the (R)-3-(1-aminoethyl)-5-(trifluoromethyl)aniline hydrochloride in Example 1 [Step-10], the same procedure of Example 1 was repeated to obtain 15 mg of the title compound at 13% yield.

¹H-NMR (300 MHz, DMSO-*d₆*): *δ* 8.36 (d, 1H), 7.91 (s, 1H), 7.39 (s, 1H), 6.92 (d, 2H), 6.79 (s, 1H), 5.68 (m, 1H), 5.25 (s, 2H), 3.96 (s, 3H), 3.65 (m, 4H), 3.51 (m, 2H), 3.12 (m, 2H), 2.38 (d, 6H), 1.55 (m, 3H).
MS (ESI+, m/z): 504.2 [M+H]⁺

### Example 17: (R)-(4-((1-(3-amino-5-(fluorophenyl)ethyl)amino)-6-methoxy-2-methylquinazolin-7-yl)(morpholino)methanone

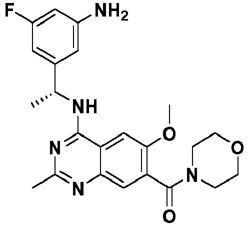

Except for the use of (*R*)-3-(1-aminoethyl)-5-fluoro aniline hydrochloride (46 mg, 0.24 mmol) instead of the (R)-3-(1-aminoethyl)-5-(trifluoromethyl)aniline hydrochloride in Example 1 [Step-10], the same procedure of Example 1 was repeated to obtain 24 mg of the title compound at 25% yield.

¹H-NMR (300 MHz, CD₃OD): *δ* 8.00 (s, 1H), 7.53 (s, 1H), 6.58 (s, 1H), 6.44 (m, 1H), 6.31 (m, 1H), 5.73 (m, 1H), 4.04 (s, 3H), 3.76 (m, 4H), 3.61 (m, 2H), 3.27 (m, 2H), 2.61 (s, 3H), 1.69 (d, 3H)
MS (ESI+, m/z): 440.2 [M+H]⁺

### Example 18: (R)-(4-((1-(3-amino-5-(trifluoromethyl)phenyl)ethyl)amino)-6-methoxy-2-methylquinazolin-7-yl)(1,1-dioxothiomorpholino)methanone

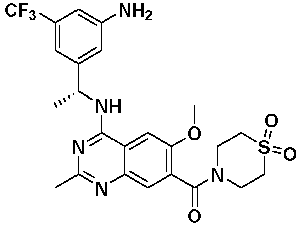

Except for the use of thiomorpholine 1,1-dioxide (30 mg, 0.21 mmol) instead of the morpholine in Example 1 [Step-8], the same procedure of Example 1 was repeated to obtain 23 mg of the title compound at 30% yield.

¹H-NMR (300 MHz, CD₃OD): *δ* 7.85 (s, 1H), 7.58 (s, 1H), 7.01 (m, 2H), 6.83 (s, 1H), 5.67 (m, 1H), 4.26 (m, 2H), 4.03 (s, 3H), 3.68 (m, 2H), 3.24 (m, 2H), 3.13 (m, 2H), 2.50 (s, 3H), 1.67 (d, *J* = 6.9 Hz, 3H).
MS (ESI+, m/z): 538.1 [M+H]⁺

### Example 19: (R)-(4-((1-(3-amino-2-methoxyphenyl)ethyl)amino)-6-methoxy-2-methylquinazolin-7-yl)(morpholino)methanone

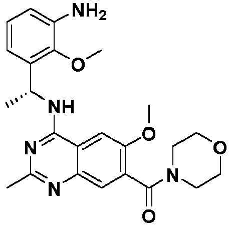

Except for the use of (*R*)-3-(1-aminoethyl)-2-methoxyaniline hydrochloride (49 mg, 0.31 mmol) instead of the (*R*)-3-(1-aminoethyl)-5-(trifluoromethyl)aniline hydrochloride in Example 1 [Step-10], the same procedure of Example 1 was repeated to obtain 30 mg of the title compound at 31% yield.

¹H-NMR (300 MHz, DMSO-*d₆*): *δ* 8.24 (m, 1H), 7.92 (s, 1H), 7.38 (s, 1H), 6.78 (m, 1H), 6.67 (m, 1H), 6.57 (m, 1H), 5.92 (m, 1H), 4.92 (m, 2H), 3.97 (s, 3H), 3.90 (d, 3H), 3.65 (m, 4H), 3.51 (m, 2H), 3.13 (m, 2H), 2.34 (s, 3H), 1.52 (m, 3H).

MS (ESI+, m/z): 452.2 [M+H]⁺

### Example 20: (R)-(4-((1-(3-amino-5-(trifluoromethyl)phenyl)ethyl)amino)-6-methoxy-2-methylquinazolin-7-yl)(thiazolidine-3-yl)methanone

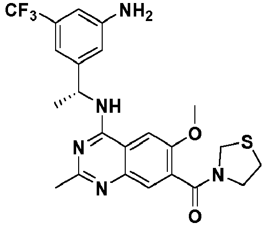

Except for the use of thiazolidine (0.06 mL, 0.70 mmol) instead of the morpholine in Example 1 [Step-8], the same procedure of Example 1 was repeated to obtain 18 mg of the title compound at 56% yield.

¹H-NMR (300 MHz, DMSO-*d₆*): *δ* 8.31 (d, 1H), 7.88 (s, 1H), 7.44 (s, 1H), 6.90 (d, 2H), 6.72 (s, 1H), 5.63 (m, 3H), 4.64 (s, 1H), 4.22 (s, 1H), 3.95 (s, 3H), 3.86 (m, 1H), 3.45 (m, 1H), 3.12 (m, 1H), 2.99 (m, 1H), 2.39 (s, 3H), 1.60 (d, 3H).
MS (ESI+, m/z): 492.2 [M+H]⁺

### Example 21: (R)-(4-((1-(3-amino-5-methylphenyl)ethyl)amino)-6-methoxy-2-methylquinazolin-7-yl)(morpholino)methanone

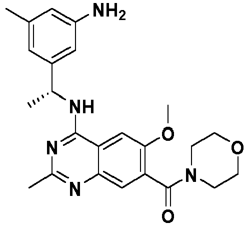

Except for the use of (*R*)-3-(1-aminoethyl)-5-methylaniline hydrochloride (45 mg, 0.31 mmol) instead of the (*R*)-3-(1-aminoethyl)-5-(trifluoromethyl)aniline hydrochloride in Example 1 [Step-10], the same procedure of Example 1 was repeated to obtain 15 mg of the title compound at 16% yield.

¹H-NMR (300 MHz, DMSO-*d₆*): *δ* 8.20 (m, 1H), 7.87 (s, 1H), 7.39 (s, 1H), 6.63 (m, 2H), 6.48 (m, 1H), 5.60 (m, 1H), 4.92 (m, 2H), 3.94 (d, 3H), 3.65 (s, 4H), 3.54 (m, 2H), 3.12 (m, 2H), 2.40 (d, 3H), 2.20 (d, 3H), 1.55 (m, 3H).
MS (ESI+, m/z): 436.2 [M+H]⁺

### Example 22: (R)-3-amino-5-(1-((6-methoxy-2-methyl-7-(morpholine-4-carbonyl)quinazolin-4-yl)amino)ethyl)benzonitrile

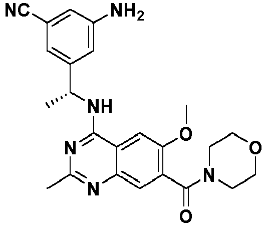

Except for the use of (*R*)-3-amino-5-(1-aminoethyl)benzonitrile hydrochloride (65 mg, 0.33 mmol) instead of the (*R*)-3-(1-aminoethyl)-5-(trifluoromethyl)aniline hydrochloride in Example 1 [Step-10], the same procedure of Example 1 was repeated to obtain 6 mg of the title compound at 6% yield.

¹H-NMR (300 MHz, CD₃OD): *δ* 7.80 (s, 1H), 7.49 (s, 1H), 7.02 (m, 2H), 6.80 (s, 1H), 5.58 (m, 1H), 4.01 (s, 3H), 3.76 (m, 4H), 3.61 (m, 2H), 3.27 (m, 2H), 2.47 (s, 3H), 1.64 (d, 3H)
MS (ESI+, m/z): 447.2 [M+H]⁺

### Example 23: (R)-(4-((1-(2,3-dihydro-1H-inden-4-yl)ethyl)amino)-6-methoxy-2-methylquinazolin-7-yl)(morpholino)methanone

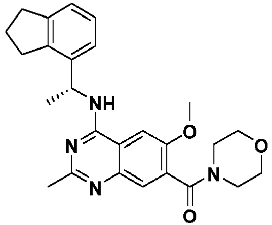

Except for the use of (R)-1-(2,3-dihydro-1H-inden-4-yl)ethane-1-amine (45 mg, 0.27 mmol) instead of the (R)-3-(1-aminoethyl)-5-(trifluoromethyl)aniline hydrochloride in Example 1 [Step-10], the same procedure of Example 1 was repeated to obtain 35 mg of the title compound at 31% yield.

¹H-NMR (300 MHz, DMSO-*d₆*): *δ* 8.29 (m, 1H), 7.86 (s, 1H), 7.37 (s, 1H), 7.27 (m, 1H), 7.09 (s, 1H), 5.63 (m, 1H), 3.95 (s, 3H), 3.64 (m, 4H), 3.48 (m, 2H), 3.32 (m, 2H), 2.95 (m, 2H), 2.36 (s, 3H), 2.09 (m, 2H), 1.58 (m, 3H)
MS (ESI+, m/z): 447.2 [M+H]⁺

### Example 24: (R)-(4-((1-(3-amino-5-cyclopropylphenyl)ethyl)amino)-6-methoxy-2-methylquinazolin-7-yl)(morpholino)methanone

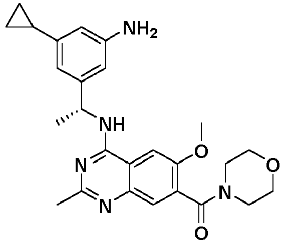

Except for the use of (*R*)-3-(1-aminoethyl)-5-cyclopropylaniline hydrochloride (57 mg, 0.27 mmol) instead of the (*R*)-3-(1-aminoethyl)-5-(trifluoromethyl)aniline hydrochloride in Example 1 [Step-10], the same procedure of Example 1 was repeated to obtain 23 mg of the title compound at 20% yield.

¹H-NMR (300 MHz, DMSO-*d₆*): *δ* 8.18 (d, 1H), 7.86 (s, 1H), 7.38 (s, 1H), 6.41 (s, 2H), 6.10 (s, 1H), 5.55 (m, 1H), 4.89 (s, 2H), 3.95 (s, 3H), 3.65 (m, 4H), 3.50 (m, 2H), 3.38 (m, 2H), 3.11 (s, 2H), 2.39 (s, 3H), 1.83 (m, 1H), 1.53 (m, 3H), 0.83 (m, 2H), 0.55 (m, 2H)
MS (ESI+, m/z): 462.2 [M+H]⁺

### Example 25: (R)-(4-((1-(5-amino-2-fluoro-3-(trifluoromethyl)phenyl)ethyl)amino)-6-methoxy-2-methylquinazolin-7-yl)(morpholino)methanone

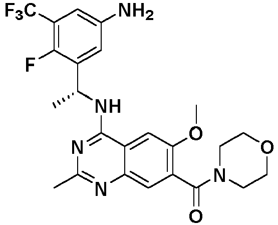

Except for the use of (*R*)-3-(1-aminoethyl)-4-fluoro-5-trifluoromethyl)aniline hydrochloride (62 mg, 0.24 mmol) instead of the (*R*)-3-(1-aminoethyl)-5-(trifluoromethyl)aniline hydrochloride in Example 1 [Step-10], the same procedure of Example 1 was repeated to obtain 40 mg of the title compound at 36% yield.

¹H-NMR (300 MHz, CD₃OD): *δ* 7.84 (m, 1H), 7.47 (s, 1H), 6.92 (m, 1H), 6.77 (m, 1H), 5.72 (m, 1H), 4.00 (s, 3H), 3.78 (m, 4H), 3.57 (m, 2H), 3.22 (m, 2H), 2.39 (m, 3H), 1.64 *(d, J=* 7.4 Hz, 3H).
MS (ESI+, m/z): 508.1 [M+H]⁺

### Example 26: (R)-(4-((1-(5-amino-3-(difluoromethyl)-2-fluorophenyl)ethyl)amino)-6-methoxy 2-methylquinazolin-7-yl)(morpholino)methanone

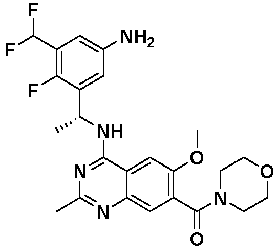

Except for the use of (*R*)-3-(1-aminoethyl)-2-methoxyaniline hydrochloride (58 mg, 0.31 mmol) instead of the (*R*)-3-(1-aminoethyl)-5-(trifluoromethyl)aniline hydrochloride in Example 1 [Step-10], the same procedure of Example 1 was repeated to obtain 33 mg of the title compound at 31% yield.

¹H-NMR (300 MHz, DMSO-*d₆*): *δ* 8.30 (m, 1H), 7.92 (s, 1H), 7.41 (s, 1H), 7.09 (m, 1H), 6.79 (d, 1H), 6.63 (m, 1H), 5.78 (m, 1H), 5.20 (s, 2H), 3.97 (s, 3H), 3.66 (s, 4H), 3.54 (m, 2H), 3.13 (m, 2H), 2.35 (s, 3H), 1.60 (m, 3H).
MS (ESI+, m/z): 490.2 [M+H]⁺

### Example 27: (4-(((R)-1-(3-amino-5-(trifluoromethyl)phenyl)ethyl)amino)-2-methyl-6-(((S)-tetrahydrofuran-3-yl)oxy)quinazolin-7-yl)(morpholino)methanone

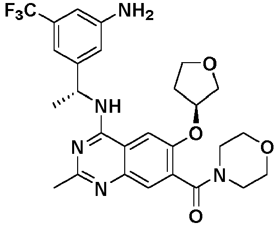

Except for the use of tetrahydrofuran-3-yl 4-methylbenzenesulfonate (1.11 g, 4.6 mmol) instead of the methyl iodide in Example 1 [Step-4], the same procedure of Example 1 was repeated to obtain 25 mg of the title compound at 18% yield.

¹H-NMR (300 MHz, CD₃OD): *δ* 7.79 (m, 1H), 7.58 (s, 1H), 7.52 (s, 1H), 7.01 (m, 1H), 6.83 (s, 1H), 5.67 (m, 1H), 5.25 (m, 1H), 3.94 (m, 4H), 3.81 (m, 4H), 3.62 (m, 2H), 3.34 (m, 2H), 2.49 (s, 3H), 2.38 (m, 2H), 1.68 (d, *J* = 5.4 Hz, 3H).
MS (ESI+, m/z): 546.2 [M+H]⁺

### Example 28: (R)-(4-((1-(3-amino-5-(furan-3-yl)phenyl)ethyl)amino)-6-methoxy-2-methylquinazolin-7-yl)(morpholino)methanone

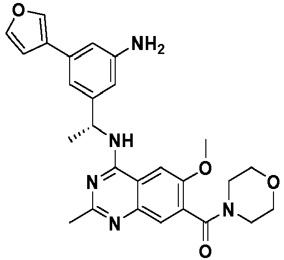

Except for the use of (*R*)-3-(1-aminoethyl)-5-(furan-3-yl)aniline hydrochloride (62 mg, 0.26 mmol) instead of the (*R*)-3-(1-aminoethyl)-5-(trifluoromethyl)aniline hydrochloride in Example 1 [Step-10], the same procedure of Example 1 was repeated to obtain 10 mg of the title compound at 9% yield.

¹H-NMR (300 MHz, CD₃OD): *δ* 8.04 (s, 1H), 7.80 (s, 1H), 7.51 (m, 2H), 6.97 (s, 1H), 6.83 (s, 1H), 6.74 (m, 2H), 5.80 (m, 1H), 4.04 (s, 3H), 3.78 (m, 4H), 3.61 (m, 2H), 3.27 (m, 2H), 2.64 (s, 3H), 1.75 (d, 3H)
MS (ESI+, m/z): 488.2 [M+H]⁺

### Example 29: (R)-(4-((1-(3-amino-5-(difluoromethyl)phenyl)ethyl)amino)-6-methoxy-2-methylquinazolin-7-yl)(morpholino)methanone

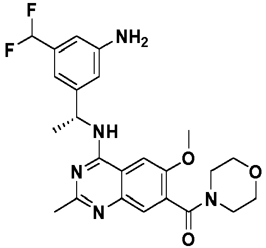

Except for the use of (*R*)-3-(1-aminoethyl)-5-(difluoromethyl)aniline hydrochloride (58 mg, 0.26 mmol) instead of the (*R*)-3-(1-aminoethyl)-5-(trifluoromethyl)aniline hydrochloride in Example 1 [Step-10], the same procedure of Example 1 was repeated to obtain 10 mg of the title compound at 10% yield.
¹H-NMR (300 MHz, CD₃OD): *δ* 7.82 (s, 1H), 7.49 (s, 1H), 6.91 (s, 2H), 6.58 (s, 1H), 6.39 (m, 1H), 5.68 (m, 1H), 4.00 (s, 3H), 3.76 (m, 4H), 3.60 (m, 2H), 3.27 (m, 2H), 2.49 (s, 3H), 1.67 (d, 3H)
MS (ESI+, m/z): 472.2 [M+H]⁺

### Example 30: (R)-(4-((1-(3-amino-5-(thiazole-5-yl)phenyl)ethyl)amino)-6-methoxy-2-methylquinazolin-7-yl)(morpholino)methanone

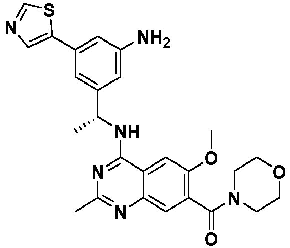

Except for the use of (*R*)-3-(1-aminoethyl)-5-(thiazol-5-yl)aniline hydrochloride (67 mg, 0.26 mmol) instead of the (*R*)-3-(1-aminoethyl)-5-(trifluoromethyl)aniline hydrochloride in Example 1 [Step-10], the same procedure of Example 1 was repeated to obtain 4 mg of the title compound at 4% yield.
¹H-NMR (300 MHz, CD₃OD): *δ* 8.89 (s, 1H), 8.06 (d, 1H), 7.81 (s, 1H), 7.49 (s, 1H), 7.06 (s, 1H), 6.87 (s, 1H), 6.84 (m, 1H), 5.65 (m, 1H), 4.00 (s, 3H), 3.78 (m, 4H), 3.61 (m, 2H), 3.27 (m, 2H), 2.48 (s, 3H), 1.69 (d, 3H)
MS (ESI+, m/z): 505.2 [M+H]⁺

### Example 31: (R)-(4-((1-(3-(ethylamino)-5-(trifluoromethyl)phenyl)ethyl)amino)-6-methoxy-2-methylquinazolin-7-yl)(morpholino)methanone

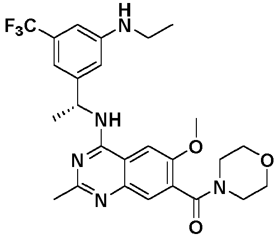

After the (*R*)-(4-((1-(3-amino-5-(trifluoromethyl)phenyl)ethyl)amino)-6-methoxy-2-methylquinazoline-7-yl)(morpholino)methanone (50 mg, 0.10 mmol) obtained in Example 1 [Step-10] was dissolved in 1 mL of dichloromethane, acetaldehyde (5.4 mg, 0.12 mmol) and 1.0M titanium tetrachloride dichloromethane solution were added to the reaction solution (0.01 mL, 0.01 mmol), and then sodium cyanobromine hydride (26 mg, 0.41 mmol) was added, followed by stirring at room temperature overnight. After the reaction was completed, the reaction product was extracted three times with ethyl acetate, dried over anhydrous sodium sulfate, and then concentrated under reduced pressure. The residue was purified by column chromatography (dichloromethane:methanol = 20:1 (v/v)) to obtain 16 mg of the title compound at 31% yield.

¹H-NMR (300 MHz, CD₃OD): *δ* 7.82 (d, 1H), 7.51 (s, 1H), 6.98 (d, 2H), 6.71 (s, 1H), 5.68 (m, 1H), 4.02 (s, 3H), 3.77 (m, 4H), 3.63 (m, 2H), 3.35 (m, 2H), 3.28 (m, 2H), 2.49 (s, 3H), 1.68 (d, *J* = 6.9 Hz, 3H), 1.25 (m, 3H).
MS (ESI+, m/z): 518.2 [M+H]⁺

### Example 32: methyl (R)-(4-((1-(3-amino-5-(trifluoromethyl)phenyl)ethyl)amino)-6-(2-methoxyethoxy)-2-methylquinazolin-7-yl)(morpholino)methanone

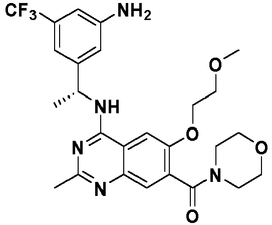

Except for the use of 2-methoxyethyl 4-methylbenzenesulfonate (3.2 g, 14.11 mmol) instead of the methyl iodide in Example 1 [Step-4], the same procedure of Example 1 was repeated to obtain 7 mg of the title compound at 6% yield.

¹H-NMR (300 MHz, DMSO-*d₆*): *δ* 8.31 (m, 1H), 7.86 (s, 1H), 7.41 (s, 1H), 6.89 (m, 2H), 6.71 (s, 1H), 5.57 (m, 3H), 4.28 (m, 2H), 3.94-3.50 (m, 8H), 3.37 (m, 3H), 3.16 (m, 2H), 2.39 (s, 3H), 1.59 (m, 3H).
MS (ESI+, m/z): 534.2 [M+H]⁺

### Example 33: (R)-(4-((1-(3-amino-5-(trifluoromethyl)phenyl)ethyl)amino)-2-(fluoromethyl)-6-methoxyquinazolin-7-yl)(morpholino)methanone

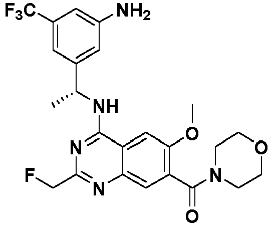

Except for the use of fluoroacetonitrile (7.1 mL, 125 mmol) instead of the acetonitrile in Example 1 [Step-7], the same procedure of Example 1 was repeated to obtain 32 mg of the title compound at 34% yield.

¹H-NMR (300 MHz, CD₃OD): *δ* 7.92 (s, 1H), 7.68 (s, 1H), 7.00 (m, 2H), 6.83 (s, 1H), 5.68 (m, 1H), 5.51 (m, 1H), 5.39 (m, 1H), 4.01 (s, 3H), 3.79 (m, 4H), 3.63 (m, 2H), 3.23 (m, 2H), 1.69 *(d, J= 7.2 Hz, 3H).*
MS (ESI+, m/z): 508.2 [M+H]⁺

### Example 34: (R)-N-(1-(3-amino-5-(trifluoromethyl)phenyl)ethyl)-6-methoxy-2-methyl-7-(morpholinomethyl)quinazoline-4-amine

### [Step-1] Preparation of methyl 2-bromo-5-methoxy-methylbenzoate

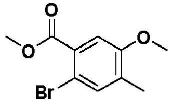

Methyl 3-methoxy-4-methylbenzoate (5 g, 27.74 mmol) was mixed with 40 mL of acetic acid and 40 mL of water, and bromine (1.5 mL, 30.52 mmol) was added dropwise thereto. After the dropwise addition was completed, stirring and refluxing were performed at 60°C for 1 hour. After the reaction was completed, the resultant was cooled to room temperature, and a sodium bicarbonate aqueous solution was added dropwise thereto. The aqueous solution was extracted three times with a hexane/ether (8:3) solution, an organic layer was dried over anhydrous sodium sulfate and filtered under reduced pressure, and the filtered solution was concentrated under reduced pressure to obtain 6.92 g of the title compound at 96% yield.

¹H-NMR (300 MHz, CDCl₃): *δ* 7.39 (s, 1H), 7.26 (s, 1H), 3.92 (s, 3H), 3.84 (s, 3H), 2.21 (s, 3H).

### [Step-2] Preparation of methyl 2-bromo-4-(bromomethyl)-5-methoxybenzoate

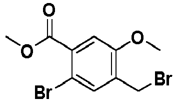

The methyl 2-bromo-5-methoxy-methylbenzoate (6.92 g, 26.70 mmol) obtained in [Step-1], *N*-bromosuccinimide (4.28 g, 24.60 mmol), and azobisisobutyronitrile (853 mg, 5.19 mmol) were dissolved in 130 mL of chloroform, and this solution was stirred under reflux at 70°C for 2 hours. After the reaction was completed, the resultant was cooled to room temperature, and a sodium bicarbonate aqueous solution was added dropwise thereto. After extraction with ethyl acetate three times, an obtained organic layer was dried over anhydrous sodium sulfate. The dried organic layer was filtered under reduced pressure, and the organic layer was concentrated under reduced pressure. The obtained residue was purified by MPLC (ethyl acetate:hexane = 1:5 (v/v) to 1:1 (v/v)) to obtain 5.98 g of the title compound at 66% yield.

¹H-NMR (300 MHz, CDCl₃): *δ* 7.59 (s, 1H), 7. 30 (s, 1H), 4.45 (s, 2H), 3.93 (s, 3H), 3.91 (s, 3H).

### [Step-3] Preparation of methyl 2-bromo-5-methoxy-4- (morpholinomethyl) benzoate

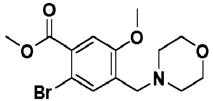

The methyl 2-bromo-4-(bromomethyl)-5-methoxybenzoate (1.2 g, 3.55 mmol) obtained in [Step-2], morpholine (0.34 mL, 3.90 mmol), and potassium carbonate (981 mg, 7.10 mmol) were dissolved in 20 mL of acetonitrile, and this solution was stirred at room temperature for 18 hours. After the reaction was completed, the resultant was cooled to room temperature, and a sodium bicarbonate aqueous solution was added dropwise thereto. After extraction with ethyl acetate three times, an obtained organic layer was dried over anhydrous sodium sulfate. The dried organic layer was filtered under reduced pressure, and the organic layer was concentrated under reduced pressure. The obtained residue was purified by MPLC (ethyl acetate:hexane=1:5 (v/v) to 1:1 (v/v)) to obtain 1.0 g of the title compound at 82% yield.

¹H-NMR (300 MHz, CDCl₃): *δ* 7.68 (s, 1H), 7.28 (s, 1H), 3.93 (s, 3H), 3.84 (s, 3H), 3.73 (m, 4H), 3.51 (m, 2H), 2.49 (m, 4H).

### [Step-4] Preparation of methyl 2-((tert-butoxycarbonyl)amino)-5-methoxy-4-(morpholinomethyDbenzoate

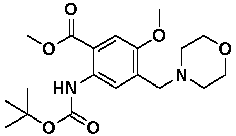

The methyl 2-bromo-5-methoxy-4- (morpholino methyl)benzoate (1.0 g, 2.90 mmol) obtained in [Step-3], *tert*-butyl carbamate (566 mg, 3.19 mmol), xantphos (336 mg, 0.58 mmol), Pd₂(dba)₃dba (266 mg, 0.29 mmol), and cesium carbonate (2.83 g, 8.71 mmol) were dissolved in 25 mL of 1,4-dioxane, and this solution was stirred at 110°C for 2 hours. After the reaction was completed, the resultant was cooled to room temperature, filtered through a celite-filled filter, and washed with ethyl acetate. The filtered organic layer was concentrated under reduced pressure, and the obtained residue was purified by MPLC (ethyl acetate:hexane =1:5 (v/v) to 1:1 (v/v)) to obtain a target compound.

¹H-NMR (300 MHz, CDCl₃): *δ* 8.39 (s, 1H), 7.41 (s, 1H), 3.91 (s, 3H), 3.81 (s, 3H), 3.73 (m, 4H), 3.56 (s, 2H), 2.51 (m, 4H), 1.52 (s, 9H).

### [Step-8] Preparation of 6-methoxy-2-methyl-7-(morpholinomethyl)quinazoline-4-ol

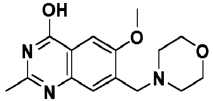

The methyl 2-((tert-butoxycarbonyl)amino)-5-methoxy-4-(morpholinomethyl)benzoate (950 mg, 2.49 mmol) obtained in [Step-4] was dissolved in 10 mL of acetonitrile, and 10 mL of a 4N hydrogen chloride dioxane solution was added dropwise thereto. This solution was stirred under reflux at 80°C for 2 hours. After the reaction was completed, the resultant was cooled to room temperature, and a sodium bicarbonate aqueous solution was added dropwise thereto for neutralization. The reaction product was extracted three times with dichloromethane, dried under anhydrous sodium sulfate, and concentrated under reduced pressure. The residue was solidified with ethyl acetate and filtered under reduced pressure. A solid resulting through the filtration was dried to produce 680 mg of the title compound at 94% yield.

¹H-NMR (300 MHz, CD₃OD): *δ* 7.70 (s, 1H), 7.59 (s, 1H), 3.95 (s, 3H), 3.73 (m, 4H), 3.67 (s, 2H), 2.55 (m, 4H), 2.43 (s, 3H).

### Preparation of (R)-N-(1-(3-amino-5-(trifluoromethyl)phenyl)ethyl)-6-methoxy-2-methyl-7-(morpholinomethyl)quinazoline-4-amine

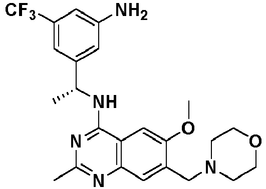

The 6-methoxy-2-methyl-7-(morpholino methyl)quinazoline-4-ol (100 mg, 0.34 mmol) obtained in [Step-8], (R)-3-(1-aminoethyl)-5-(trifluoromethyl)aniline hydrochloride (125 mg, 0.51 mmol), PyBOP (269 mg, 0.51 mmol), and DBU (0.13 mL 0.86 mmol) were dissolved in 3 mL of acetonitrile and stirred at 80°C for 5 hours. After the reaction was completed, the reaction solution was cooled to room temperature, followed by dropwise addition of water thereto. The resultant was extracted three times with ethyl acetate, dried over anhydrous sodium sulfate, and concentrated under reduced pressure. The residue was purified by column chromatography (dichloromethane methanol = 23:1 (v/v)) to obtain 14 mg of the title compound at 8% yield.
¹H-NMR (300 MHz, DMSO-*d₆*): *δ* 8.11 (d, 1H), 7.71 (s, 1H), 7.57 (s, 1H), 6.85 (m, 2H), 6.69 (s, 1H), 5.57 (m, 3H), 3.92 (s, 3H), 3.61 (m, 4H), 3.57(s, 2H), 2.44 (m, 4H), 2.36 (s, 3H), 1.56 (d, 3H)
MS (ESI+, m/z): 476.2 [M+H]⁺

### Example 35: (R)-N-(1-(5-amino-3-(difluoromethyl)-2-fluorophenyl)ethyl)-6-methoxy-2-methyl-7-(morpholinomethyl)quinazoline-4-amine

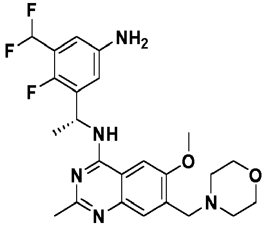

Except for the use of (R)-3-(1-aminoethyl)-5-(difluoromethyl)-4-fluoroaniline hydrochloride (125 mg, 0.51 mmol) instead of the (R)-3-(1-aminoethyl)-5-(trifluoromethyl)aniline hydrochloride in Example 39 [Step-6], the same procedure of Example 39 was repeated to obtain 9 mg of the title compound at 5% yield.
¹H-NMR (300 MHz, DMSO-*d₆*): *δ* 8.11 (d, 1H), 7.76 (s, 1H), 7.55 (s, 1H), 7.06 (t, 1H), 6.74 (m, 1H), 6.58 (m, 1H), 5.72 (m, 3H), 5.16 (s, 2H), 3.94 (s, 3H), 3.60 (m, 4H), 3.56(s, 2H), 2.42 (m, 4H), 2.31 (s, 3H), 1.55 (d, 3H)
MS (ESI+, m/z): 476.2 [M+H]⁺

### Example 36: (R)-N-(1-(3-amino-5-(trifluoromethylphenyl)ethyl)-6-methoxy-2-methyl-7-((tetrahydro-2H-pyran-4-yl)oxy)quinazolin-4-amine

### [Step-1] Preparation of methyl 4-hydroxy-5-methoxy-2-nitrobenzoate

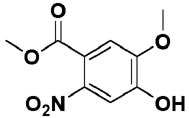

Methyl 4-(benzyloxy)-5-methoxy-2-nitrobenzoate (11.7 g, 36.9 mmol) was dissolved in 250 mL of methanol, and Pd/C (1.2 g, 10 wt%) was added thereto. This reaction solution was stirred at 50°C in a hydrogen gas ambient for 20 hours. After the reaction was completed, the reaction solution was filtered through a celite-filled filter and washed with methanol. The resulting organic layer was concentrated under reduced pressure, and the obtained residue was purified by MPLC (ethyl acetate:hexane =1:5 (v/v) to 1:1 (v/v)) to obtain 7 g of the title compound at 96% yield.

¹H-NMR (300 MHz, CD₃OD): *δ* 7.29 (s, 1H), 6.24 (s, 1H), 6.84 (m, 1H), 4.87 (bs, 2H), 3.91 (s, 3H), 3.81 (s, 3H).

### [Step-2] Preparation of 7-hydroxy-6-methoxy-2-methylquinazoline-4(1H)-one

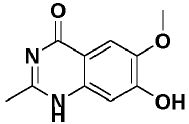

The methyl 4-hydroxy-5-methoxy-2-mitrobenzoate (7.0 g, 35.5 mmol) obtained in [Step-1] and a 4N hydrogen chloride dioxane solution (71 mL, 284 mmol) were dissolved in 20 mL of 355 mmol acetonitrile, and the solution was stirred under reflux at 70°C for 15 hours. After the reaction was completed, the reaction solution was cooled to room temperature, and 30 mL of dichloromethane was added thereto. After that, the mixture was stirred for 0.5 hours to obtain a solid product. The obtained solid was filtered under reduced pressure and dried in an oven dryer at 55°C to obtain 9 g of the title compound at 82% yield.

¹H-NMR (300 MHz, DMSO-*d*₆): *δ* 11.2 (s, 1H), 9.38 (s, 1H), 8.24 (s, 1H), 7.49 (s, 1H), 6.86 (s, 1H), 3.85 (s, 3H), 3.79 (s, 3H).

### [Step-3] Preparation of 6-methoxy-2-methyl-7-((tetrahydro-2H-pyran-4-yl)oxy)quinazoline-4(1H)-one

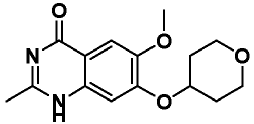

The 7-hydroxy-6-methoxy-2-methylquinazoline-4(1H)-one (1.8 g, 8.7 mmol) obtained in [Step-2] was dissolved in 20 mL ofDMF, and tetrahydro-2*H*-pyran-4-yl methane sulfonate (1.7 g, 9.5 mmol) and cesium carbonate (3.4 g, 10.4 mmol) were added thereto. This was stirred at 100°C for 15 hours. After the reaction was completed, water was added to the reaction solution, and the reaction product was extracted three times with ethyl acetate, dried over anhydrous sodium sulfate, and concentrated under reduced pressure. After 10 mL of acetone was added thereto and stirred, the precipitated crystals was filtered to obtain 250 mg of the title compound at 10% yield.

¹H-NMR (300 MHz, CD₃OD): *δ* 7.84 (s, 1H), 7.58 (s, 1H), 4.77 (m, 1H), 4.13 (m, 2H), 3.96 (s, 3H), 3.64 (m, 2H), 2.44 (s, 3H), 2.22 (m, 2H), 2.09 (m, 2H).

### [Step-4] Preparation of 4-chloro-6-methoxy-2-methyl-7-((tetrahydro-2H-pyran-4-yl)oxy)quinazoline

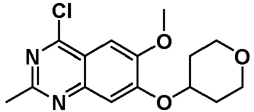

The 6-methoxy-2-methyl-7-((tetrahydro-2H-pyran-4-yl)oxy)quinazoline-4(1H)-one (250 mg, 0.86 mmol) obtained in [Step-3] above was dissolved in 14 mL of phosphoryl chloride, and this solution was refluxed at 1 10°C for 2 hours. After the reaction was completed, the reaction solution was cooled to room temperature and neutralized by dropwise addition of a sodium bicarbonate aqueous solution. The resultant was extracted with ethyl acetate three times, dried over anhydrous sodium sulfate, and concentrated under reduced pressure. This was used in the next step without being purified.

### [Step-5] Preparation of (R)-N-(1-(3-amino-5-(trifluoromethylphenyl)ethyl)-6-methoxy-2-methyl-7-((tetrahydro-2H-pyran-4-yl)oxy)quinazoline-4-amine

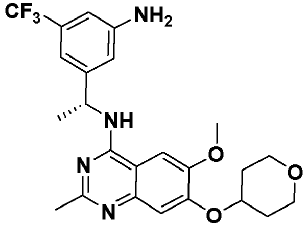

The 4-chloro-6-methoxy-2-methyl-7-((tetrahydro-2H-pyran-4-yl)oxy)quinazoline (100 mg, 0.33 mmol) obtained in [Step-4] above was dissolved in 1 mL of DMF, and (R)-3-(1-aminoethyl)-5-(trifluoromethyl)aniline hydrochloride (92 mg, 0.36 mmol) and DIPEA (0.17 mL, 0.97 mmol) were added thereto. The mixture was stirred at 100°C for 13 hours. After the reaction was completed, the reaction solution was cooled to room temperature, followed by dropwise addition of water thereto. The resultant was extracted three times with ethyl acetate, dried over anhydrous sodium sulfate, and concentrated under reduced pressure. The residue was purified by column chromatography(dichloromethane:methanol = 23:1 (v:methanol = 23:1 (v/v)) to obtain 21 mg of the title compound at 13% yield.

¹H-NMR (300 MHz, CD₃OD): *δ* 7.67 (s, 1H), 7.06 (s, 1H), 6.99 (m, 2H), 6.82 (s, 1H), 5.64 (m, 1H), 4.74 (m, 1H), 4.12 (m, 5H), 3.66 (m, 2H), 2.46 (s, 3H), 2.16 (m, 2H), 1.86 (m, 2H), 1.67 (d, 3H).
MS (ESI+, m/z): 477.2 [M+H]⁺

### Example 37: (R)-N-(1-(5-amino-3-(difluoromethyl)-2-fluorophenyl)ethyl)-6-methoxy-2-methyl-7-((tetrahydro-2H-pyran-4-yl)oxy)quinazoline-4-amine

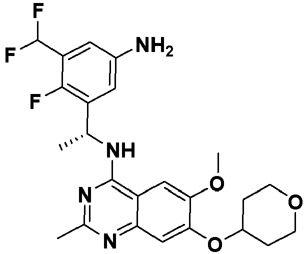

Except for the use of (R)-3-(1-aminoethyl)-5-(difluoromethyl)-4-fluoroaniline hydrochloride (69 mg, 0.28 mmol) instead of the (R)-3-(1-aminoethyl)-5-(trifluoromethyl)aniline hydrochloride in Example 44 [Step-5], the same procedure of Example 44 was repeated to obtain 5.7 mg of the title compound at 4.6% yield.

¹H-NMR (300 MHz, CD₃OD): *δ* 7.73 (s, 1H), 7.09 (s, 1H), 6.79 (m, 3H), 5.81 (m, 1H), 4.59 (m, 1H), 3.99 (m, 5H), 3.66 (m, 2H), 2.42 (s, 3H), 2.17 (m, 2H), 1.73 (m, 2H), 1.62 (d, 3H).
MS (ESI+, m/z): 477.2 [M+H]⁺

### Example 38: (R)-N-(1-(3-amino-5-(trifluoromethyl)phenyl)ethyl)-6-methoxy-2-methyl-7-((tetrahydro-2H-pyran-4-yl)methoxy)quinazoline-4-amine

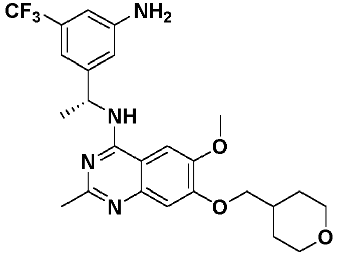

Except for the use of (tetrahydro-2*H*-pyran-4-yl)methyl 4-methylbenzenesulfonate (752 mg, 2.78 mmol) instead of the tetrahydro-2*H*-pyran-4-yl methanesulfonate in Example 44 [Step-3], the same procedure of Example 44 was repeated to obtain 15 mg of the title compound at 3.5% yield.

¹H-NMR (300 MHz, CD₃OD): *δ* 7.46 (s, 1H), 7.00 (s, 3H), 6.83 (s, 1H), 5.67(q, 1H), 4.08~4.10 (m, 7H), 3.73(m, 2H), 3.50 (t, 2h\H), 2.54 (s, 3H), 1.89(m, 2H), 1.68(d, 3H), 1.57(m, 2H).
MS (ESI+, m/z): 491.2 [M+H]⁺

### Example 39: (R)-N-(1-(3-amino-5-(trifluoromethyl)phenyl)ethyl)-6-methoxy-2-methyl-7-(oxetane-3-ylmethoxy)quinazoline-4-amine

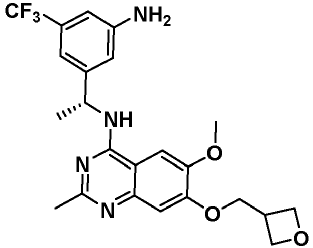

Except for the use of oxetane-3-ylmethyl 4-methylbenzenesulfonate (530 mg, 2.17 mmol) instead of the tetrahydro-2*H*-pyran-4-yl methanesulfonate in Example 44 [Step-3], the same procedure of Example 44 was repeated to obtain 20 mg of the title compound at 15% yield.

¹H-NMR (300 MHz, DMSO-*d*₆): *δ* 8.00 (d, 1H), 7.71 (s, 1H), 7.10 (s, 1H), 6.89 (d, 2H), 6.70 (s, 1H), 5.59 (m, 3H), 4.76 (m, 2H), 4.47 (m, 2H), 4.34 (m, 2H), 3.90 (s, 3H), 3.50 (m, 1H), 2.36 (s, 3H), 1.57 (d, 3H).
MS (ESI+, m/z): 463.2 [M+H]⁺

### Example 40: (R)-(4-((1-(3-amino-5-(trifluoromethyl)phenyl)ethyl)amino)-2-methyl-6-(methylamino)quinazoline-7-yl)(morpholino)methanone

### [Step-1] Preparation of 2-bromo-5-nitroterephthalic acid

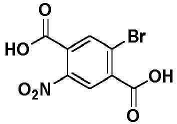

2-bromoterephthalic acid (75 g, 306.12 mmol) was dissolved in 490 mL of sulfuric acid, and 98 mL of nitric acid was slowly added dropwise thereto at 0°C. After the dropwise addition was completed, stirring and refluxing were performed at room temperature for 17 hours. After the reaction was completed, the reaction solution was slowly added dropwise to ice water and the mixture was stirred at room temperature for 1 hour. After the stirring, the resultant solid was filtered under reduced pressure and washed with distilled water to obtain 61 g of the title compound at 69% yield.

¹H-NMR (300 MHz, DMSO-*d₆*): *δ* 8.34 (s, 1H), 8.17 (s, 1H).

### [Step-4] Preparation of dimethyl 2-bromo-5-nitroterephthalate

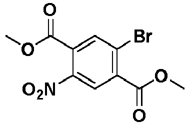

The 2-bromo-5-nitroterephthalic acid (61 g, 210.32 mmol) obtained in [Step-1] above was dissolved in 2.2 L of methanol, and 240 mL of sulfuric acid was slowly added dropwise thereto at 0°C. After the dropwise addition was completed, stirring and refluxing were performed at 90°C for 17 hours. After the reaction was completed, the reaction solution was cooled to room temperature and concentrated under reduced pressure, 600 mL of distilled water was added dropwise thereto at 0°C, and the mixture was stirred at room temperature for 0.5 hours. After the stirring, the resultant was filtered under reduced pressure to obtain 61 g of the title compound at 91% yield.

¹H-NMR (300 MHz, DMSO-*d₆*): *δ* 8.47 (s, 1H), 8.28 (s, 1H), 3.93-3.89 (m, 6H).

### [Step-3] Preparation of dimethyl 2-(methylamino)-5-nitroterephthalate

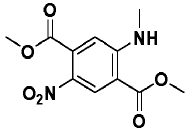

The dimethyl 2-bromo-5-nitroterephthalate (60 g, 188.63 mmol) obtained in [Step-2], methylamine hydrochloride (63.6 g, 941.94 mmol), and DIPEA (492 g, 2829.48 mmol) were dissolved in 900 mL of DMF, and the mixture were stirred under reflux at 100°C for 1 hour. After the reaction was completed, the solution was cooled to 0°C, 1.8 L of distilled water was added dropwise to the solution, and the solution was stirred at room temperature for 0.5 hours. After the stirring, the resultant was filtered under reduced pressure to obtain 48.6 g of the title compound at 96% yield.

¹H-NMR (300 MHz, DMSO-*d₆*): *δ* 8.59 (s, 2H), 6.95 (s, 1H), 3.88 (s, 3H), 3.86 (s, 3H), 3.01-2.99 (d, 3H).

### [Step-4] Preparation of dimethyl 2-amino-5-(methylamino)terephthalate

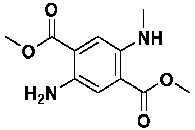

The dimethyl 2-(methylamino)-5-nitroterephthalate (45.5 g, 169.63 mmol) obtained in [Step-3] and zinc dust (39.4 g, 593.71 mmol) were dissolved in 460 mL of a mixed solution of dioxane and distilled water (4:1), and the mixture was stirred under reflux at room temperature for 0.5 hours. After the stirring, the reaction solution was cooled to 0°C, and ammonium chloride (45.4 g, 848.76 mmol) was slowly added dropwise. After the dropwise addition was completed, stirring and refluxing were performed at room temperature for 2 hours. After the reaction was completed, the reaction solution was filtered through a celite-filled filter and washed with ethyl acetate. Distilled water was added dropwise to an organic layer that was obtained (in a volume ratio of 1:1), and extraction was performed with ethyl acetate three times. The obtained organic layer was then dried over anhydrous sodium sulfate. The dried organic layer was filtered under reduced pressure and concentrated under reduced pressure, and the obtained residue was purified by column chromatography (ethyl acetate:hexane = 1:8(v/v) to 1:4(v/v)) to obtain 31 g of the title compound at 77% yield.

¹H-NMR (300 MHz, DMSO-*d₆*): *δ* 7.39 (s, 1H), 7.02 (s, 1H), 6.55 (m, 1H), 5.88 (s, 2H), 3.83-3.81 (m, 6H), 2.78-2.76 (d, 3H).

### [Step-5] Preparation of methyl 4-hydroxy-2-methyl-6-(methylamino)quinazoline-7-carboxylate

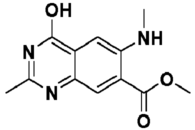

The dimethyl 2-amino-5-(methylamino)terephthalate (31 g, 130.12 mmol) obtained in [Step-4] and acetonitrile (68 mL, 1301.20 mmol) were dissolved in 260 mL of 4N hydrochloric acid, and the mixture was stirred using a sealed tube under reflux at 90°C for 3 hours. After the reaction was completed, the reaction solution was cooled to room temperature, filtered through a filter, and washed with hexane. The solid resulting through the filtration was neutralized with a sodium bicarbonate aqueous solution, filtered under reduced pressure, and washed with distilled water to obtain 30 g of the title compound at 93.2% yield.

¹H-NMR (300 MHz, DMSO-*d₆*): *δ* 12.03 (m, 1H), 8.02 (s, 1H), 7.41 (m, 1H), 7.16 (s, 1H), 3.87 (s, 3H), 2.91 (d, 3H), 2.28 (s, 3H).

### [Step-6] Preparation of methyl 4-chloro-2-methyl-6-(methylamino)quinazoline-7-carboxylate

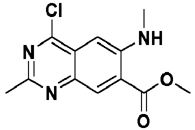

The methyl 4-hydroxy-2-methyl-6-(methylamino)quinazoline-7-carboxylate (6.1 g, 24.69 mmol) prepared in [Step-5] was dissolved in 150 mL of phosphoryl chloride, and the mixture was stirred under reflux at 120°C for 3 hours. When the reaction was completed, the reaction solution was cooled to room temperature and concentrated under reduced pressure, and the obtained residue was dissolved in dichloromethane and neutralized with dichloromethane at low temperature. The organic layer was washed with distilled water and dried over anhydrous sodium sulfate. The dried organic layer was filtered under reduced pressure and concentrated under reduced pressure, and the obtained residue was purified by column chromatography (dichloromethane:ethyl acetate = 45:55(v/v)) to obtain 1.6 g of the title compound at 24% yield.

¹H-NMR (300 MHz, DMSO-*d₆*): *δ* 8.36 (s, 1H), 7.57 (m, 1H), 6.92 (s, 1H), 3.93 (s, 3H), 2.94 (d, 3H), 2.66 (s, 3H).

### [Step-7] Preparation of methyl (R)-(4-((1-(3-amino-5-(trifluoromethyl)phenyl)ethyl)amino)-2-methyl-6-(methylamino)quinazoline-7-carboxylate

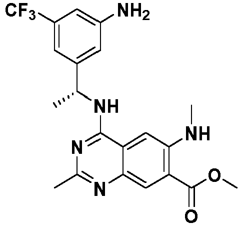

The methyl 4-chloro-2-methyl-6-(methylamino)quinazoline-7-carboxylate (600 mg, 2.26 mmol) prepared in [Step-6], (*R*)-3-(1-aminoethyl)-5-(trifluoromethyl)aniline hydrochloride (705 mg, 2.93 mmol) synthesized by the method disclosed in WO2018115380, and DIPEA (1.21 mL, 6.78 mmol) were dissolved in 30 mL of DMF, and the mixture was stirred under reflux at 90°C for 13 hours. After the reaction was completed, the reaction solution was cooled to room temperature, followed by dropwise addition of water thereto. The resultant was extracted three times with ethyl acetate, dried over anhydrous sodium sulfate, and concentrated under reduced pressure. The residue was purified by column chromatography (dichloromethane:methanol = 23:1 (v/v)) to obtain 280 mg of the title compound at 29% yield.

¹H-NMR (300 MHz, CDCl₃): *δ* 8.46 (s, 1H), 7.12 (s, 1H), 6.94 (s, 1H), 6.83 (s, 1H), 6.47 (s, 1H), 5.62 (m, 1H), 3.93 (s, 3H), 2.93 (s, 3H), 2.55 (s, 3H).

### [Step-8] Preparation of (R)-(4-((1-(3-amino-5-(trifluoromethyl)phenyl)ethyl)amino)-2-methyl-6-(methylamino)quinazoline-7-yl)(morpholino)methanone

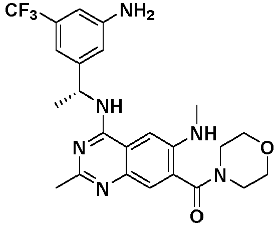

The (*R*)-4-((1-(3-amino-5-(trifluoromethyl)phenyl)ethyl)amino)-2-methyl-6-(methylamino)quinazoline-7-carboxylate (280 mg, 0.64 mmol) obtained in [Step-7] was dissolved in 20 mL of a mixed solution of tetrahydrofuran, methanol, and water (= 2:1: 1), followed by addition of sodium hydroxide (129 mg, 3.23 mmol). Next, the mixture was stirred under reflux at room temperature for 2 hours. After the reaction was completed, a 2N HCl aqueous solution was added dropwise to adjust the pH to 5 to 6, and then the resultant was washed with ethyl acetate. The obtained organic layer was dried under anhydrous sodium sulfate and concentrated under reduced pressure to produce (*R*)-(4-((1-(3-amino-5-(trifluoromethyl)phenyl)ethyl)amino)-2-methyl-6-(methylamino)quinazoline-7-yl)-carboxylic acid, without performing purification. The obtained title compound, morpholine (0.62 mL, 0.72 mmol), HATU (272 g, 0.18 mmol), and DIPEA (0.26 mL, 1.43 mmol) were dissolved in 5 mL of DMF, and the mixture was stirred under reflux at room temperature for 2.5 hours. After the reaction was completed, the reaction product was extracted three times with ethyl acetate, dried over anhydrous sodium sulfate, and concentrated under reduced pressure. The obtained residue was purified by column chromatography (dichloromethane:methanol = 20:1 (v/v)) to obtain 30 mg of the title compound at 20% yield.

¹H-NMR (300 MHz, CD₃OD): *δ* 7.38 (s, 1H), 7.28 (s, 1H), 6.99 (m, 2H), 6.82 (s, 1H), 5.67 (m, 1H), 3.73 (m, 8H), 2.96 (s, 3H), 2.46 (s, 3H), 1.67 (d, J= 7.2 Hz, 3H).
MS (ESI+, m/z): 489.2 [M+H]⁺

### Example 41: (R)-(4-((1-(3-amino-5-(trifluoromethyl)phenyl)ethyl)amino)-6-(dimethylamino)-2-methylquinazoline-7-yl)(morpholino)methanone

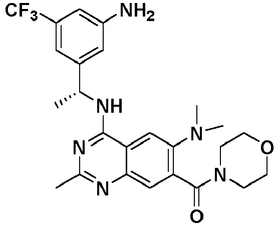

Except for the use of dimethylamine hydrochloride (2.3 g, 28 mmol) instead of the methylamine hydrochloride in Example 49 [Step-3], the same procedure of Example 49 was repeated to obtain 40 mg of the title compound at 12% yield.

¹H-NMR (300 MHz, DMSO-*d₆*): *δ* 7.77 (d, 1H), 7.38 (s, 1H), 6.90 (d, 2H), 6.74 (s, 1H), 5.68 (m, 3H), 3.77 (m, 6H), 3.11 (m, 2H), 2.87 (d, 6H), 2.47 (s, 3H), 1.63 (m, 3H).
MS (ESI+, m/z): 503.2 [M+H]⁺

### Example 42: (R)-(4-((1-(3-amino-5-(trifluoromethyl)phenyl)ethyl)amino)-2-methyl-6-(pyrrolidin-1-yl)quinazoline-7-yl)(morpholino)methanone

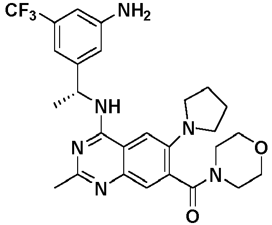

Except for the use of pyrrolidine (2.0 g, 28 mmol) instead of the methylamine hydrochloride in Example 49 [Step-3], the same procedure of Example 49 was repeated to obtain 60 mg of the title compound at 19% yield.

¹H-NMR (300 MHz, DMSO-*d₆*): *δ* 8.17 (d, 1H), 7.34 (m, 2H), 6.90 (d, 2H), 6.70 (s, 1H), 5.65 (m, 3H), 3.76 (m, 6H), 3.28 (m, 6H), 2.35 (d, 3H), 1.98 (m, 4H), 1.58 (m, 3H).
MS (ESI+, m/z): 529.3 [M+H]⁺

### Example 43: (R)-(4-((1-(5-amino-3-(difluoromethyl)-2-fluorophenyl)ethyl)amino)-2-methyl-6-(methylamino)quinazoline-7-yl)(morpholino)methanone

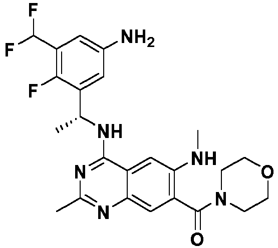

Except for the use of (*R*)-3-(1-aminoethyl)-5-(difluoromethyl)-4-fluoroaniline hydrochloride (235 mg, 0.98 mmol) instead of the (*R*)-3-(1-aminoethyl)-5-(trifluoromethyl)aniline hydrochloride in Example 49 [Step-7], the same procedure of Example 49 was repeated to obtain 24 mg of the title compound at 38% yield.

¹H-NMR (300 MHz, CD₃OD): *δ* 7.38 (s, 1H), 7.32 (s, 1H), 7.09 (m, 2H), 5.78 (m, 1H), 3.67 (m, 8H), 2.98 (s, 3H), 2.42 (s, 3H), 1.67 (d, *J* = 7.2 Hz, 3H).
MS (ESI+, m/z): 489.2 [M+H]⁺

### Example 44: (R)-(4-((1-(3-amino-5-(trifluoromethyl)phenyl)ethyl)amino)-lr((2-methoxyethyl)amino)-2-methylquinazoline-7-yl)(morpholino)methanone

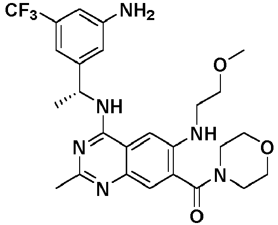

Except for the use of 2-methoxyethan-1-amine (3.5 g, 47.2 mmol) instead of the methylamine hydrochloride in Example 49 [Step-3], the same procedure of Example 49 was repeated to obtain 12 mg of the title compound at 11% yield.
¹H-NMR (300 MHz, CD₃OD): *δ* 7.39 (m, 2H), 6.99 (m, 1H), 6.83 (s, 1H), 5.65 (m, 1H), 3.76 (m, 8H), 3.68 (m, 4H), 3.66 (s, 3H), 2.52 (s, 3H), 1.66 (d, *J*= 6.9 Hz, 3H)
MS (ESI+, m/z): 533.2 [M+H]⁺

### Example 45: (R)-(4-((1-(3-amino-5-(trifluoromethyl)phenyl)ethyl)amino)-6-(cyclopentylamino)-2-methylquinazoline-7-yl)(morpholino)methanone

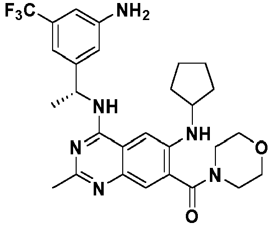

Except for the use of cyclopentanamine (4.7 g, 47.2 mmol) instead of the methylamine hydrochloride in Example 49 [Step-3], the same procedure of Example 49 was repeated to obtain 22 mg of the title compound at 6% yield.

¹H-NMR (300 MHz, CD₃OD): *δ* 7.44 (s, 1H), 7.38 (s, 1H), 6.99 (m, 2H), 6.84 (m, 1H), 5.73 (m, 1H), 4.08 (m, 1H), 3.67 (m, 8H), 2.52 (s, 3H), 1.81 (m, 4H), 1.73 (m, 3H), 1.53 (m, 4H).
MS (ESI+, m/z): 543.2 [M+H]⁺

### Example 46: (R)-(4-((1-(3-amino-5-(trifluoromethyl)phenyl)ethyl)amino)-6-(ethylamino)-2-methylquinazoline-7-yl)(morpholino)methanone

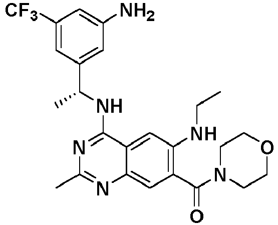

Except for the use of ethylamine (23 g, 47 mmol) dissolved in tetrahydrofuran instead of the methylamine hydrochloride in Example 49 [Step-3], the same procedure of Example 49 was repeated to obtain 120 mg of the title compound at 53% yield.

¹H-NMR (300 MHz, DMSO-*d₆*): *δ* 8.74 (s, 1H), 7.37 (d, 2H), 6.89 (d, 2H), 6.73 (s, 1H), 5.76 (m, 4H), 3.65 (m, 6H), 3.33 (m, 4H), 2.43 (s, 3H), 1.62 (d, 3H), 1.26 (m, 3H).
MS (ESI+, m/z): 503.2 [M+H]⁺

### Example 47: (R)-(4-((1-(3-amino-5-(trifluoromethyl)phenyl)ethyl)amino)-6-(isopropylamino)-2-methylquinazoline-7-yl)(morpholino)methanone

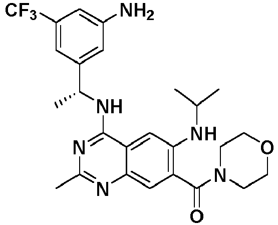

Except for the use of isopropylamine (3.86 mL, 47.1 mmol) instead of the methylamine hydrochloride in Example 49 [Step-3], the same procedure of Example 49 was repeated to obtain 3 mg of the title compound at 1% yield.

¹H-NMR (300 MHz, CD₃OD): *δ* 7.36 (m, 2H), 6.98 (m, 2H), 6.80 (s, 1H), 5.64 (m, 1H), 3.92 (m, 1H), 3.69 (m, 8H), 2.42 (s, 3H), 1.64 (d, 3H), 1.27 (m, 6H).
MS (ESI+, m/z): 517.2 [M+H]⁺

### Example 48: (R)-(4-((1-(3-amino-5-(trifluoromethyl)phenyl)ethyl)amino)-2-methyl-6-((tetrahydro-2H-pyran-4-yl)amino)quinazoline-7-yl)(morpholino)methanone

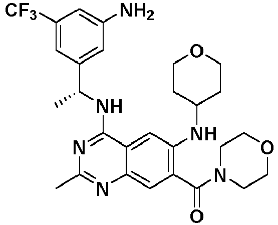

Except for the use of 4-aminotetrahydropyran hydrochloride (3.89 g, 28.29 mmol) instead of the methylamine hydrochloride in Example 49 [Step-3], the same procedure of Example 49 was repeated to obtain 130 mg of the title compound at 56% yield.

¹H-NMR (300 MHz, DMSO-*d₆*): *δ* 7.42 (s, 1H), 7.34 (s, 1H), 6.88 (s, 1H), 6.85 (s, 1H), 6.42 (s, 1H), 5.68 (p, 1H), 5.57(br, 2H), 5.15(d, 1H), 3.90 (d, 2H), 3.82-3.48 (br, 10H), 2.41 (s, 3H), 1.92(d, 2H), 1.62 (d, 3H), 1.57-1.48(m, 2H).
MS (ESI+, m/z): 559.2 [M+H]⁺

### Example 49: (R)-N⁴-(1-(3-amino-5-(trifluoromethyl)phenyl)ethyl)-N⁶,2-dimethyl-7-(morpholinomethyl)quinazoline-4,6-diamine

### [Step-1] Preparation of ethyl 2-(tert-butoxycarbonyl)amino)-4-methylbenzoate

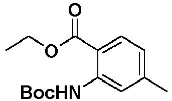

Methyl 2-amino-4-methylbenzoate (6.2 g, 34.5 mmol), di-tert-butyl decarbonate (20 mL, 86.2 mmol), triethylamine (12 mL, 86.2 mmol), and 4-dimethylaminopyridine (4.2 g, 34.5 mmol) were dissolved in 100 mL of tetrahydrofuran, and the mixture was stirred under reflux at room temperature for 15 hours. After the reaction was completed, water was added dropwise. Then, the reaction product was extracted three times with ethyl acetate, dried over anhydrous sodium sulfate, and concentrated under reduced pressure. The obtained residue was purified by column chromatography (hexane:ethyl acetate = 5:1 (v/v)) to obtain 4.7 g of the title compound at 49% yield.

¹H-NMR (300 MHz, CDCl₃): *δ* 10.35 (s, 1H), 8.30 (s, 1H), 7.93 (d, 1H), 6.84 (m, 1H), 4.41 (m, 2H), 2.40 (s, 3H), 1.55 (s, 9H), 1.44 (m, 3H).

### [Step-2] Preparation of ethyl 4-(bromomethyl)-2-((tert-butoxycarbonyl)amino)benzoate

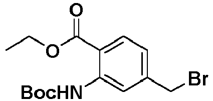

The ethyl 2-(tert-butoxycarbonyl)amino)-4-methylbenzoate (4.7 g, 16.8 mmol) obtained in [Step-1], N-bromosuccinimide (3.0 g, 16.8 mmol), and azobisisobutyronitrile (0.55 mg, 3.3 mmol) were dissolved in 100 mL of chloroform, and the mixture was stirred under reflux at 70°C for 1.5 hours. After the reaction was completed, the solution was cooled to room temperature and concentrated under reduced pressure to produce 4.3 g of the title compound. Without purification, the next reaction was performed.

### [Step-3] Preparation of ethyl 2-((tert-butoxycarbonyl)amino)-4-(morpholinomethxl)benzoate

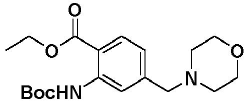

The ethyl 4-(bromomethyl)-2-((tert-butoxycarbonyl)amino)benzoate (4.3 g, 11.9 mmol) obtained in [Step-2], morpholine (2.0 mL, 23.8 mmol), and potassium carbonate (6.6 mg, 47.6 mmol) were dissolved in 40 mL of acetonitrile, and the mixture was stirred under reflux at room temperature for 1 hour. After the reaction was completed, water was added dropwise. Then, the reaction product was extracted three times with ethyl acetate, dried over anhydrous sodium sulfate, and concentrated under reduced pressure. The residue was purified by column chromatography (hexane:ethyl acetate = 5:1 (v/v)) to produce 2.1 g of the title compound at 34% yield.

¹H-NMR (300 MHz, CDCl₃): *δ* 10.34 (s, 1H), 8.39 (d, 1H), 8.00 (d, 1H), 7.08 (m, 1H), 4.42 (m, 2H), 3.75 (m, 4H), 3.54 (m, 2H), 2.49 (m, 4H), 1.55 (s, 9H), 1.45 (m, 3H).

### [Step-4] Preparation of 2-methyl-7-(morpholinomethyl)quinazoline-4-ol

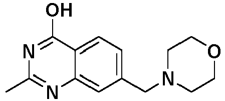

The ethyl 2-((tert-butoxycarbonyl)amino)-4-(morpholino methyl)benzoate (2.1 g, 5.73 mmol) obtained in [Step-3] and acetonitrile (21 mL, 402.5 mmol) were dissolved in 21 mL of 4N hydrochloric acid dissolved in dioxane, and the mixture was stirred using a sealed tube under reflux at 90°C for 3 hours. After the reaction was completed, the reaction solution was cooled to room temperature, filtered through a filter, and washed with hexane. The solid resulting through the filtration was neutralized with a sodium bicarbonate aqueous solution, filtered under reduced pressure, and washed with distilled water to obtain 1.2 g of the title compound at 81% yield.

¹H-NMR (300 MHz, DMSO-*d₆*): *δ* 12.17 (m, 1H), 8.04 (d, 1H), 7.49 (s, 1H), 7.42 (m, 1H), 3.61 (m, 6H), 2.52 (m, 4H), 2.39 (m, 3H).

### [Step-5] Preparation of 2-methyl-7-(morpholinomethyl)-6-nitroquinazoline-4-ol

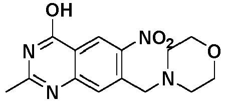

The 2-methyl-7-(morpholinomethyl)quinazoline-4-ol (1.2 g, 4.62 mmol) prepared in [Step-4] was dissolved in 12 mL of sulfuric acid, and 2.4 mL of nitric acid was slowly added dropwise thereto at 0°C. The mixture was stirred under reflux at 70°C for 3 hours. After the reaction was completed, the reaction solution was cooled to room temperature and concentrated under reduced pressure, and the obtained residue was dissolved in dichloromethane and neutralized with a sodium hydroxide aqueous solution at low temperature. The obtained organic layer was washed with distilled water and dried over anhydrous sodium sulfate. The dried organic layer was filtered under reduced pressure and concentrated under reduced pressure to produce 1.2 g of the title compound at 85% yield.

¹H-NMR (300 MHz, DMSO-*d₆*): *δ* 12.57 (m, 1H), 8.49 (s, 1H), 7.79 (s, 1H), 3.86 (s, 2H), 3.55 (m, 4H), 2.52 (m, 3H), 2.39 (m, 4H).

### [Step-6] Preparation of 6-amino-2-methyl-7-(morpholinomethyl)quinazoline-4-ol

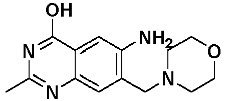

The 2-methyl-7-(morpholino methyl)-6-nitroquinazoline-4-ol (450 g, 1.47 mmol) obtained in [Step-5] and zinc dust (340 g, 5.14 mmol) were dissolved in 5 mL of a mixed solution of dioxane and distilled water (4:1), and the mixture was stirred under reflux at room temperature for 0.5 hours. After the stirring, the reaction solution was cooled to 0°C, and ammonium chloride (400 g, 7.35 mmol) was slowly added dropwise. After the dropwise addition was completed, stirring and refluxing were performed at room temperature for 2 hours. After the reaction was completed, the reaction solution was filtered through a celite-filled filter and washed with ethyl acetate. Distilled water was added to the obtained organic layer (in a volume ratio of 1:1), extraction was performed with ethyl acetate three times, and the obtained organic layer was dried over anhydrous sodium sulfate. The dried organic layer was filtered under reduced pressure and concentrated under reduced pressure, and the obtained residue was purified by column chromatography (dichloromethane:methanol = 10:1 (v/v)) to produce 330 g of the title compound at 81% yield.

¹H-NMR (300 MHz, DMSO-*d₆*): δ 11.76 (m, 1H), 7.26 (d, 2H), 5.62 (s, 2H), 3.60 (m, 6H), 2.38 (m, 4H), 2.26 (s, 3H).

### [Step-7] Preparation of 2-methyl-6-(methylamino)-7-(morpholinomethyl)quinazoline-4-ol

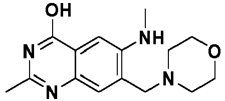

The 6-amino-2-methyl-7-(morpholino methyl)quinazoline-4-ol (155 mg, 0.56 mmol) obtained in [Step-6], methyl iodide (70 mg, 0.50 mmol), and calcium carbonate (84 mg, 0.84 mmol) were dissolved in 2 mL of dimethylformamide, and the mixture was stirred under reflux at 50°C for 12 hours. After the reaction was completed, the reaction solution was cooled to room temperature, followed by dropwise addition of water thereto. The resultant was extracted three times with ethyl acetate, dried over anhydrous sodium sulfate, and concentrated under reduced pressure. The residue was purified by column chromatography(dichloromethane:methanol = 13:1 (v/v)) to produce 30 mg of the title compound at 18% yield.

¹H-NMR (300 MHz, CDCl₃): *δ* 9.75 (s, 1H), 7.38 (s, 1H), 7.27 (s, 1H), 6.41 (d, 1H), 3.73 (m, 6H), 2.98 (d, 3H), 2.50 (s, 3H), 2.47 (m, 4H).

### [Step-8] Preparation of 2-methyl-6-(methylamino)-7-(morpholinomethyl)quinazoline-4-yl 2,4,6-triisopropylbenzenesulfonate

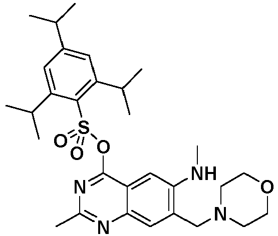

The 2-methyl-6-(methylamino)-7-(morpholino methyl)quinazoline-4-ol (30 mg, 0.10 mmol) obtained in [Step-7], 2,4,6-triisopropylbenzenesulfonyl chloride (48 mg, 0.12 mmol), 4-dimethylaminopyridine (3 mg, 0.01 mmol), and triethylamine (0.04 mL, 0.30 mmol) were dissolved in 2 mL of dichloromethane, and the mixture was stirred under reflux at room temperature for 18 hours. After the reaction was completed, the resultant was concentrated under reduced pressure, and the residue was purified by column chromatography (dichloromethane:methanol = 15:1 (v/v)) to produce 25 mg of the title compound at 43% yield.

### [Step-9] Preparation of (R)-N⁴-(1-(3-amino-5-(trifluoromethyl)phenyl)ethyl)-N⁶,2-dimethyl-7-(morpholinomethyl)quinazoline-4,6-diamine

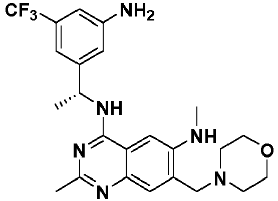

The 2-methyl-6-(methylamino)-7-(morpholino methyl)quinazoline-4-yl 2,4,6-triisopropylbenzenesulfonate (25 mg, 0.04 mmol) prepared in [Step-8], (*R*)-3-(1-aminoethyl)-5-(trifluoromethyl)aniline hydrochloride (15 mg, 0.05 mmol), and triethylamine (0.025 mL, 0.18 mmol) were dissolved in 2 mL of DMF, and the mixture was stirred under reflux at 90°C for 22 hours. After the reaction was completed, water was added dropwise. The reaction product was extracted with ethyl acetate, dried over anhydrous sodium sulfate, and concentrated under reduced pressure. The residue was purified by column chromatography (dichloromethane:methanol = 15:1 (v/v)) to produce 3 mg of the title compound at 14% yield.

¹H-NMR (300 MHz, CDCl₃): δ 7.52 (s, 1H), 7.11 (s, 1H), 7.00 (m, 1H), 6.82 (s, 1H), 6.49 (m, 2H), 5.71 (m, 1H), 3.89 (s, 2H), 3.71 (m, 6H), 2.97 (s, 3H), 2.59 (s, 3H), 2.44 (m, 4H), 1.71 (d, 3H).
MS (ESI+, m/z): 475.2 [M+H]⁺

### Example 50: (R)-(4-((1-(5-amino-2-fluoro-3-(trifluoromethyl)phenyl)ethyl)amino)-2-methyl-6-(methylamino)quinazoline-7-yl)(morpholino)methanone

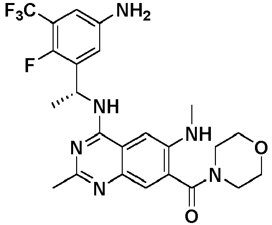

Except for the use of (*R*)-3-(1-aminoethyl)-4-fluoro-5-(trifluoromethyl)aniline hydrochloride (235 mg, 0.98 mmol) instead of the (R)-3-(1-aminoethyl)-5-(trifluoromethyl)aniline hydrochloride in Example 49 [Step-7], the same procedure of Example 49 was repeated to obtain 23 mg of the title compound at 15% yield.

¹H-NMR (300 MHz, CD₃OD): δ 7.38 (s, 1H), 7.32 (s, 1H), 7.09 (m, 2H), 6.95 (m, 1H), 6.81 (m, 1H), 5.75 (m, 1H), 3.68 (m, 8H), 2.98 (s, 3H), 2.41 (s, 3H), 1.68 (d, J = 6.9 Hz, 3H).
MS (ESI+, m/z): 507.2 [M+H]⁺

### Example 51: (R)-(4-((1-(3-amino-5-(difluoromethyl)phenyl)ethyl)amino)-2-methyl-6-(methylamino)quinazoline-7-yl)(morpholino)methanone

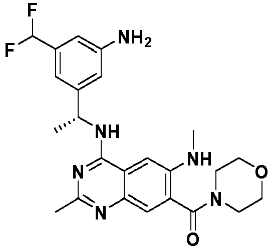

Except for the use of (R)-3-(1-aminoethyl)-5-(difluoromethyl)aniline hydrochloride (270 mg, 1.20 mmol) instead of the (R)-3-(1-aminoethyl)-5-(trifluoromethyl)aniline hydrochloride in Example 49 [Step-7], the same procedure of Example 49 was repeated to obtain 70 mg of the title compound at 41% yield.

¹H-NMR (300 MHz, DMSO-*d₆*): δ 9.15 (d, 1H), 7.34 (d, 2H), 7.03-6.66 (m, 3H), 6.62 (s, 1H), 5.81 (d, 1H), 5.73 (m, 1H), 5.45 (d, 2H), 3.68-3.51(m, 6H), 3.32(s, 2H), 2.88 (d, 3H), 2.48 (s, 3H), 1.62 (d, 3H).
MS (ESI+, m/z): 471.2 [M+H]⁺

### Example 52: (R)-(4-((1-(5-amino-3-(difluoromethyl)-2-fluorophenyl)ethyl)amino)-6-(ethylamino)-2-methylquinazoline-7-yl)(morpholino)methanone

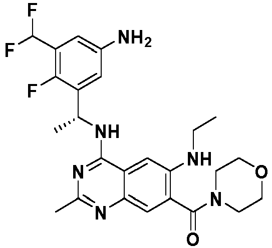

Except for the use of 2.0M ethylamine (23 mL, 47 mmol) dissolved in tetrahydrofuran, instead of methylamine hydrochloride in Example 49 [Step-3] and the use of (R)-3-(1-aminoethyl)-5-(difluoromethyl)-4-fluoroaniline hydrochloride (230 mg, 0.96 mmol) instead of the (R)-3-(1-aminoethyl)-5-(trifluoromethyl)aniline hydrochloride in Example 49 [Step-7], the same procedure of Example 49 was repeated to obtain 55 mg of the title compound at 41% yield.

¹H-NMR (300 MHz, DMSO-*d₆*): δ 8.53 (s, 1H), 7.41 (d, 2H), 7.27-6.90 (m, 1H), 6.76 (d, 1H), 6.63 (m, 1H), 5.81 (m, 1H), 5.36 (s, 1H), 5.27 (d, 2H), 3.64(s, 6H), 3.32(m, 4H), 2.37 (s, 3H), 1.60 (d, 3H), 1.28 (m, 3H).
MS (ESI+, m/z): 503.2 [M+H]⁺

### Example 53: (R)-(4-((1-(3-amino-5-(trifluoromethyl)phenyl)ethyl)amino)-2-methyl-6-(methylamino)quinazoline-7-yl)(thiazolidine-3-yl)methanone

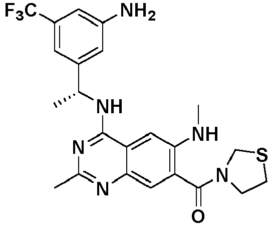

Except for the use of thiazolidine (24 mg, 0.26 mmol) instead of the morpholine in Example 49 [Step-8], the same procedure of Example 49 was repeated to obtain 16 mg of the title compound at 24% yield.

¹H-NMR (300 MHz, DMSO-*d₆*): δ 8.10 (d, 1H), 7.36 (s, 1H), 7.22 (s, 1H), 6.90 (d, 2H), 6.70 (s, 1H), 5.61-5.53 (m, 4H), 4.55 (m, 2H), 3.72 (m, 2H), 3.04 (m, 2H), 2.87 (m, 3H), 2.34 (s, 3H), 1.58 (d, 3H).
MS (ESI+, m/z): 491.2 [M+H]⁺

### Example 54: (R)-(4-((1-(3-amino-5-(furan-3-yl)phenyl)ethyl)amino)-2-methyl-6-(methylamino)quinazolin-7-yl)(morpholino)methanone

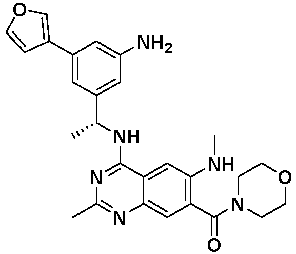

Except for the use of (*R*)-3-(1-aminoethyl)-5-(furan-3-yl)aniline hydrochloride (233 mg, 0.98 mmol) instead of the (*R*)-3-(1-aminoethyl)-5-(trifluoromethyl)aniline hydrochloride in Example 49 [Step-7], the same procedure of Example 49 was repeated to obtain 15 mg of the title compound at 32% yield.

¹H-NMR (300 MHz, CD₃OD): δ 7.80 (s, 1H), 7.52 (s, 1H), 7.38 (s, 1H), 7.29 (s, 1H), 7.01 (s, 1H), 6.77 (s, 1H), 6.75 (s, 1H), 6.72 (s, 1H), 5.67 (m, 1H), 3.66 (m, 8H), 2.96 (s, 3H), 2.46 (s, 3H), 1.67 (d, J = 7.2 Hz, 3H).
MS (ESI+, m/z): 487.2 [M+H]⁺

### Example 55: (R)-4-((1-(5-amino-3-(difluoromethyl)-2-fluorophenyl)ethyl)amino)-6-(isopropylamino)-2-methylquinazoline-7-(yl)(morpholino)methanone

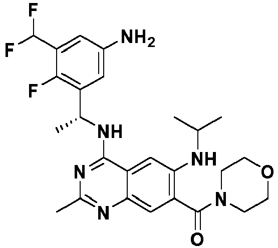

Except for the use of isopropylamine (1.7 mL, 3.14 mmol) instead of the methyleamine hydrochloride in Example 49 [Step-3] and the use of (*R*)-3-(1-aminoethyl)-5-(difluoromethyl)-4-fluoroaniline hydrochloride (224 mg, 0.92 mmol) instead of the (*R*)-3-(1-aminoethyl)-5-(trifluoromethyl)aniline hydrochloride in [Step-7], the same procedure of Example 49 was repeated to obtain 20 mg of the title compound at 23% yield.

¹H-NMR (300 MHz, DMSO-*d₆*): δ 8.03 (d, 1H), 7.40 (s, 1H), 7.27 (s, 1H), 7.07 (t, 1H), 6.73 (m, 1H), 6.58 (m, 1H), 5.71 (m, 1H), 5.17 (s, 2H), 4.80 (m, 1H), 3.94 (m, 1H), 3.72 (m, 8H), 2.28 (s, 3H), 1.55 (d, 3H), 1.23 (m, 6H).
MS (ESI+, m/z): 517.2 [M+H]⁺

### Example 56: (R)-(4-((1-(5-amino-3-(difluoromethyl)-2-fluorophenyl)ethyl)amino)-6-((2-methoxyethyl)amino)-2-methylquinazolin-7-yl)(morpholino)methanone

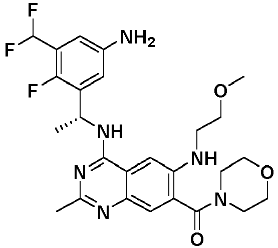

Except for the use of 2-methoxyethan-1-amine (3.5 g, 47.2 mmol) instead of the methylamine hydrochloride in Example 49 [Step-3] and the use of (*R*)-3-(1-aminoethyl)-5-(difluoromethyl)-4-fluoroaniline hydrochloride (235 mg, 0.98 mmol) instead of the (*R*)-3-(1-aminoethyl)-5-(trifluoromethyl)aniline hydrochloride in [Step-7], the same procedure of Example 49 was repeated to obtain 35 mg of the title compound at 30% yield.

¹H-NMR (300 MHz, DMSO-*d₆*): δ 8.06 (d, 1H), 7.40 (s, 1H), 7.30 (s, 1H), 7.07 (t, 1H), 6.74 (m, 1H), 6.59 (m, 1H), 5.72 (m, 1H), 5.21 (m, 3H), 3.60 (m, 8H), 3.42 (m, 4H), 3.31 (s, 3H), 2.29 (s, 3H), 1.55 (d, 3H).
MS (ESI+, m/z): 533.2 [M+H]⁺

### Example 57: (R)-(4-((1-(3-amino-5-(trifluoromethyl)phenyl)ethyl)amino)-2-methyl-6-(methylamino)quinazoline-7-yl)(4-methylpiperazin-1-yl)methanone

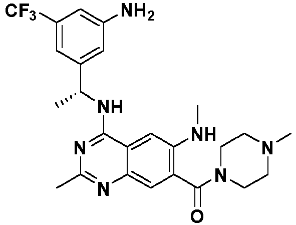

Except for the use of 1-methylpiperazine (0.03 mL, 0.25 mmol) instead of the morpholine in Example 49 [Step-8], the same procedure of Example 49 was repeated to obtain 20 mg of the title compound at 16% yield.

¹H-NMR (300 MHz, DMSO-*d₆*): δ 8.32 (s, 1H), 7.26 (d, 2H), 6.90 (d, 2H), 6.71 (s, 1H), 5.64-5.46 (m, 4H), 3.74 (m, 4H), 2.87 (d, 3H), 2.38-2.26 (m, 10H), 1.60 (d, 3H).
MS (ESI+, m/z): 502.2 [M+H]⁺

### Example 58: (4-(((R)-1-(3-amino-5-(trifluoromethyl)phenyl)ethyl)amino)-2-methyl-6-(methylamino)quinazoline-7-yl)(tetrahydro-1H-furo[3,4-c]pyrrol-5(3H)-yl)methanone

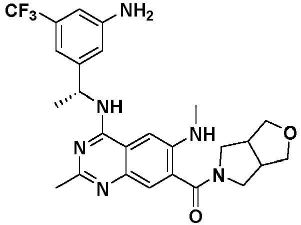

Except for the use of hexahydro-1*H-*furo[3,4-c]pyrrole (30 mg, 0.25 mmol) instead of the morpholine in Example 49 [Step-8], the same procedure of Example 49 was repeated to obtain 38 mg of the title compound at 31% yield.

¹H-NMR (300 MHz, DMSO-*d₆*): δ 9.12 (m, 1H), 7.36 (d, 2H), 6.90 (d, 2H), 6.74 (s, 1H), 5.88-5.60 (m, 4H), 3.80-3.45 (m, 8H), 2.92 (m, 5H), 2.48 (s, 3H), 1.60 (d, 3H).
MS (ESI+, m/z): 515.2 [M+H]⁺

### Example 59: (R)-(4-((1-(3-amino-5-(trifluoromethyl)phenyl)ethyl)amino)-2-methyl-6-(methylamino)quinazolin-7-yl)(1,1-dioxothiomorpholino)methanone

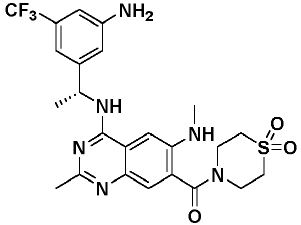

Except for the use of thiomorpholino 1,1-dioxide (35 mg, 0.26 mmol) instead of the morpholine in Example 49 [Step-8], the same procedure of Example 49 was repeated to obtain 5 mg of the title compound at 4% yield.

¹H-NMR (300 MHz, CD₃OD): δ 7.42 (s, 1H), 7.27 (s, 1H), 6.99 (m, 2H), 6.80 (m, 1H), 5.64 (m, 1H), 3.22 (m, 8H), 2.94 (s, 3H), 2.44 (s, 3H), 1.65 (d, 3H).
MS (ESI+, m/z): 537.2 [M+H]⁺

### Example 60: (R)-(4-((1-(3-amino-5-(trifluoromethyl)phenyl)ethyl)amino)-2-methyl-6-(methylamino)quinazolin-7-yl)(thiomorpholino)methanone

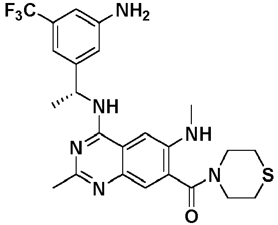

Except for the use of thiomorpholine (0.03 mL, 0.26 mmol) instead of the morpholine in Example 49 [Step-8], the same procedure of Example 49 was repeated to obtain 22 mg of the title compound at 18% yield.

¹H-NMR (300 MHz, DMSO-*d₆*): δ 8.07 (d, 1H), 7.25 (s, 1H), 7.19 (s, 1H), 6.90 (d, 2H), 6.70 (s, 1H), 5.60-5.53 (m, 3H), 5.36 (m, 1H), 3.89 (m, 2H), 3.50 (m, 2H), 2.85 (m, 3H), 2.73 (m, 2H), 2.53 (m, 2H), 2.34 (s, 3H), 1.55 (d, 3H).
MS (ESI+, m/z): 505.2 [M+H]⁺

### Example 61: (R)-(4-((1-(3-amino-5-(trifluoromethyl)phenyl)ethyl)amino)-2-methyl-6-(methylamino)quinazoline-7-yl)(piperazine-1-yl)methanone

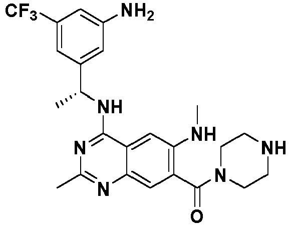

Except for the use of piperazine(100 mg, 0.50 mmol) instead of the morpholine in Example 49 [Step-8], the same procedure of Example 49 was repeated to obtain 30 mg of the title compound at 13% yield.

¹H-NMR (300 MHz, DMSO-*d₆*): *δ* 8.06 (d, 1H), 7.21 (d, 2H), 6.90 (d, 2H), 6.70 (s, 1H), 5.64-5.33 (m, 4H), 3.61 (m, 2H), 3.18 (m, 2H), 2.86 (d, 3H), 2.78-2.55 (m, 4H), 2.34 (s, 3H), 1.58(d, 3H).
MS (ESI+, m/z): 488.2 [M+H]⁺

### Example 62: (R)-(4-((1-(3-amino-5-(trifluoromethyl)phenyl)ethyl)amino)-2-methyl-6-(methylamino)quinazoline-7-yl)(azetidine-1-yl)methanone

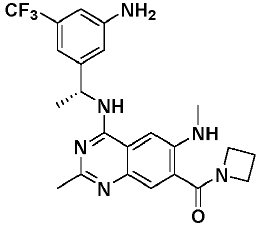

Except for the use of azetidine hydrochloride (24 mg, 0.26 mmol) instead of the morpholine in Example 49 [Step-8], the same procedure of Example 49 was repeated to obtain 12 mg of the title compound at 11% yield.

¹H-NMR (300 MHz, DMSO-*d₆*): *δ* 8.16 (m, 1H), 7.42 (s, 1H), 7.20 (s, 1H), 6.88 (m, 2H), 6.69 (s, 1H), 6.35 (m, 1H), 5.56 (m, 3H), 4.12 (m, 2H), 4.04 (m, 2H) 2.87 (m, 3H), 2.34 (s, 3H), 2.22 (m, 2H), 1.56 (d, 3H).
MS (ESI+, m/z): 459.2 [M+H]⁺

### Example 63: (4-(((R)-1-(5-amino-3-(difluoromethyl)-2-fluorophenyl)ethyl)amino)-2-methyl-6-(methylamino)quinazoline-7-yl)(tetrahydro-1H-furo[3,4-c]pyrrol-5(3H)-yl)methanone

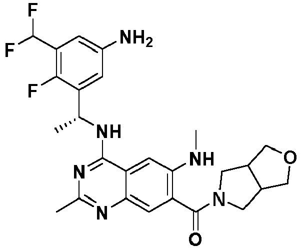

Except for the use of (R)-3-(1-aminoethyl)-5-(diffuoromethyl)-4-fluoroaniline hydrochloride (230 mL, 0.96 mmol) instead of the (R)-3-(1-aminoethyl)-5-(trifluoromethyl)aniline hydrochloride in Example 49 [Step-7] and the use of hexahydro-1*H*furo[3,4-c]pyrrole (30 mg, 0.25 mmol) instead of the mmorpholine in Example 49 [Step-8], the same procedure of Example 49 was repeated to obtain 40 mg of the title compound at 32% yield.

¹H-NMR (300 MHz, DMSO-*d₆*): *δ* 9.56 (m, 1H), 7.44 (d, 2H), 7.27-6.91 (m, 1H), 6.80 (d, 1H), 6.67 (m, 1H), 6.06-5.32 (m, 4H), 3.80-3.43 (m, 8H), 2.91 (m, 5H), 2.50 (s, 3H), 1.65 (d, 3H).
MS (ESI+, m/z): 515.2 [M+H]⁺

### Example 64: (R)-(4-((1-(5-amino-3-(difluoromethyl)-2-fluorophenyl)ethyl)amino)-2-methyl-6-(methylamino)quinazoline-7-yl)(1,1-dioxothiomorpholino)methanone

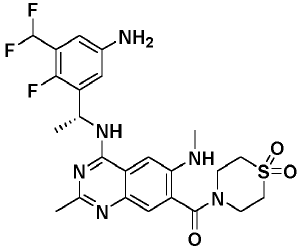

Except for the use of (*R*)-3-(1-aminoethyl)-5-(difluoromethyl)-4-fluoroaniline hydrochloride (235 mL, 0.98 mmol) instead of the (*R*)-3-(1-aminoethyl)-5-(trifluoromethyl)aniline hydrochloride in Example 49 [Step-7] and the use of thiomorpholino 1,1-dioxide (35 mg, 0.26 mmol) instead of the mmorpholine in [Step-8], the same procedure of Example 49 was repeated to obtain 8 mg of the title compound at 6% yield.

¹H-NMR (300 MHz, DMSO-*d₆*): *δ* 8.09 (m, 1H), 7.41 (s, 1H), 7.26 (t, 1H), 6.75 (m, 2H), 6.59 (m, 1H), 5.72 (m, 1H), 5.19 (m, 2H), 4.07 (m, 2H), 3.58 (m, 2H), 3.31 (m, 2H), 3.19 (m, 2H), 2.87 (m, 3H), 2.29 (s, 3H), 1.55 (d, 3H).
MS (ESI+, m/z): 537.2 [M+H]⁺

### Example 65: (R)-(4-((1-(3-amino-5-methylphenyl)ethyl)amino)-2-methyl-6-(methylamino)quinazolin-7-yl)(morpholino)methanone

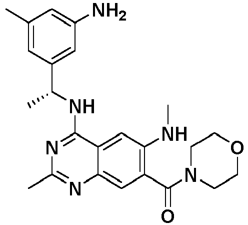

Except for the use of (R)-3-(1-aminoethyl)-5-methylaniline hydrochloride (165 mg, 0.98 mmol) instead of the (*R*)-3-(1-aminoethyl)-5-(trifluoromethyl)aniline hydrochloride in Example 49 [Step-7], the same procedure of Example 49 was repeated to obtain 3.5 mg of the title compound at 10% yield.

¹H-NMR (300 MHz, CD₃OD): *δ* 7.37 (s, 1H), 7.28 (s, 1H), 6.66 (m, 2H), 6.47 (s, 1H), 5.65 (m, 1H), 3.98 (m, 8H), 2.96 (s, 3H), 2.48 (s, 3H), 2.24 (s, 3H), 1.66 (s, 3H).
MS (ESI+, m/z): 435.2 [M+H]⁺

### Example 66: (4-(((R)-1-(5-amino-3-(difluoromethyl)-2-fluorophenyl)ethyl)amino)-6-((2-methoxyethyl)amino)-2-methylquinazoline-7-yl)(tetrahydro-1H-furo[3,4-c]pyrrol-5(3H)-yl)methanone

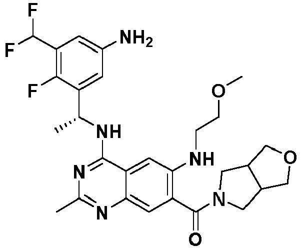

Except for the use of 2-methoxyethylamine (4.1 mL, 47.15 mmol) instead of the methylamine hydrochloride in Example 49 [Step-3] and the use of (*R*)-3-(1-aminoethyl)-5-(difluoromethyl)-4-fluoroaniline hydrochloride (190 mg, 0.76 mmol) instead of the (*R*)-3-(1-aminoethyl)-5-(trifluoromethyl)aniline hydrochloride in Example 49 [Step-7], the same procedure of Example 49 was repeated to obtain 40 mg of the title compound at 36% yield.

¹H-NMR (300 MHz, DMSO-*d₆*): *δ* 8.63 (m, 1H), 7.46 (d, 2H), 7.09-6.91 (m, 1H), 6.76 (d, 1H), 6.63 (m, 1H), 5.78-5.24 (m, 4H), 4.11-3.32 (m, 15H), 2.91 (m, 2H), 2.37 (s, 3H), 1.60 (d, 3H).
MS (ESI+, m/z): 559.2 [M+H]⁺

### Example 67: (R)-(4-((1-(5-amino-3-(difluoromethyl)-2-fluorophenyl)ethyl)amino)-6-((2-methoxyethyl)amino)-2-methylquinazoline-7-yl)(1,1-dioxothiomorpholino)methanone

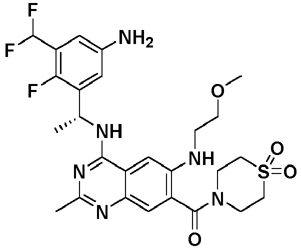

Except for the use of 2-methoxyethane-1-amine (3.5 g, 47.2 mmol) instead of the methylamine hydrochloride in Example 49 [Step-3] and the use of (*R*)-3-(1-aminoethyl)-5-(difluoromethyl)-4-fluoroaniline hydrochloride (235 mg, 0.21 mmol) instead of the (*R*)-3-(1-aminoethyl)-5-(trifluoromethyl)aniline hydrochloride in [Step-8], the same procedure of Example 49 was repeated to obtain 2 mg of the title compound at 2% yield.

¹H-NMR (300 MHz, CD₃OD): *δ* 7.40 (s, 1H), 7.38 (s, 1H), 6.88 (t, 1H), 6.85 (m, 1H), 6.77 (m, 1H), 5.77 (m, 1H), 4.59 (m, 4H), 3.69 (m, 6H), 3.48 (m, 2H), 3.42 (s, 3H), 2.39 (s, 3H), 1.65 (d, 3H).
MS (ESI+, m/z): 581.2 [M+H]⁺

### Example 68: (R)-(4-((1-(3-amino-5-(trifluoromethyl)phenyl)ethyl)amino)-lr((2-methoxyethyl)amino)-2-methylquinazoline-7-yl)(1,1-dioxothiomorpholino)methanone

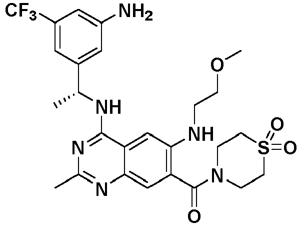

Except for the use of 2-methoxyethane-1-amine (3.5 g, 47.2 mmol) instead of the methylamine hydrochloride in Example 49 [Step-3] and the use of thiomorpholino 1,1-dioxide (55 mg, 0.40 mmol) instead of the morpholine in [Step-8], the same procedure of Example 49 was repeated to obtain 18 mg of the title compound at 8% yield.

¹H-NMR (300 MHz, DMSO-*d₆*): *δ* 8.07 (m, 1H), 7.42 (s, 1H), 7.32 (s, 1H), 6.87 (m, 2H), 6.69 (s, 1H), 5.56 (m, 3H), 5.31 (m, 1H), 3.59 (m, 2H), 3.41 (m, 4H), 3.29 (m, 9H), 2.33 (s, 3H), 1.55 (d, 3H).
MS (ESI+, m/z): 581.2 [M+H]⁺

### Example 69: (R)-N⁴-(1-(3-amino-5-(trifluoromethyl)phenyl)ethyl)-6-methoxy-2-methyl-N⁷-(tetrahydro-2H-pyran-4-yl)quinazoline-4,7-diamine

### [Step-1] Preparation of methyl 5-methoxy-2-nitro-4((tetrahydro-2H-pyran-4-yl) amino) benzoate

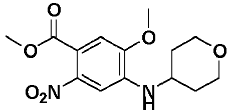

Methyl-bromo-5-methoxy-2-nitrobenzoate (800 mg, 2.75 mmol), 4-aminotetrahydropyran hydrochloride (455 mg, 3.30 mmol), Pd₂(OAc)₂ (43 mg, 0.19 mmol), (±) BINAP (120 mg, 0.19 mmol), and cesium carbonate (3.14 g, 9.64 mmol) were dissolved in 16 mL of 1,4-dioxane, and the mixture was stirred at 100°C for 24 hours. After the reaction was completed, the reaction solution was cooled to room temperature, followed by dropwise addition of water thereto. Next, the resultant was extracted with dichloromethane three times, dried over anhydrous sodium sulfate, and concentrated under reduced pressure. The obtained residue was purified by MLPC (ethyl acetate:hexane =1:1 (v/v)) to produce 970 mg of the title compound at 91% yield.

¹H-NMR (300 MHz, CDCl₃): *δ* 7.07 (s, 1H), 6.89 (s, 1H), 4.69 (m, 1H), 4.01 (m, 2H), 3.95 (s, 3H), 3.85 (s, 3H), 3.54 (m, 2H), 2.03 (m, 2H), 1.54 (m, 2H).

### [Step-2] Preparation of methyl 2-amino-5-methoxy-4((tetrahydro-2H-pyran-4-yl) amino) benzoate

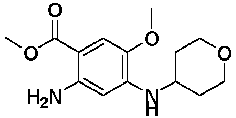

The methyl 5-methoxy-2-nitro-4((tetrahydro-2*H*-pyran-4-yl) amino) benzoate (970 g, 3.12 mmol) obtained in [Step-1] and iron (715 mg, 10.94 mmol) were were dissolved in 10 mL of a mixed solution of 1,4-dioxane and distilled water (4:1), and the mixture was cooled to 0°C. The mixture was added to ammonium chloride (636 mg, 15.62 mmol) and stirred at room temperature for 1 hour. After the reaction was completed, the reaction solution was filtered through a celite-filled filter and washed with dichloromethane. After dropwise addition of water to the filtrate, extraction was performed with dichloromethane three times, and the resultant was dried over anhydrous sodium sulfate and concentrated under reduced pressure. The obtained residue was purified by MLPC (ethyl acetate:hexane =1:1 (v/v)) to produce 850 mg of the title compound at 97% yield.

¹H-NMR (300 MHz, CDCl₃): *δ* 6.88 (s, 1H), 5.53 (s, 1H), 5.24 (s, 2H), 3.35 (m, 1H), 3.72 (m, 2H), 3.54 (m, 6H), 3.26 (m, 2H), 1.74 (m, 2H), 1.26 (m, 2H).

### [Step-3] Preparation of 6-methoxy-2-methyl-7-((tetrahydro-2H-pyran-4-yl)amino)quinazoline-4-ol

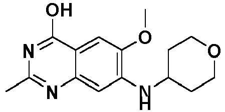

The methyl 2-amino-5-methoxy-4((tetrahydro-2*H*-pyran-4-yl) amino) benzoate (850 mg, 3.03 mmol) obtained in [Step-2] was dissolved in 3 mL of acetonitrile, and 6 mL of 4N hydrochloric acid dissolved in dioxane was added dropwise thereto. This solution was stirred under reflux at 80°C for 2 hours. After the reaction was completed, the resultant was cooled to room temperature, and a sodium bicarbonate aqueous solution was added dropwise thereto for neutralization. The reaction product was extracted three times with dichloromethane, dried under anhydrous sodium sulfate, and concentrated under reduced pressure. The residue was solidified with ethyl acetate and filtered under reduced pressure. A solid resulting through the filtration was dried to produce 830 mg of the title compound at 95% yield.

¹H-NMR (300 MHz, DMSO-*d₆*): *δ* 7.23 (s, 1H), 6.62 (s, 1H), 5.52 (m, 1H), 3.85 (m, 5H), 3.63 (m, 1H), 3.49 (m, 1H), 2.25 (s, 3H), 1.89 (m, 2H), 1.54 (m, 2H).

### [Step-4] Preparation of 4-chloro-6-methoxy-2-methyl-N-(tetrahydro-2H-pyran-4-yl)quinazoline-7-amine

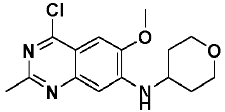

The 6-methoxy-2-methyl-7-((tetrahydro-2*H*-pyran-4-yl)amino)quinazoline-4-ol (200 mg, 0.69 mmol) obtained in [Step-3] was dissolved in 2 mL of phosphoryl chloride, and the mixture was refluxed at 100°C for 1 hour. After the reaction was completed, the reaction solution was cooled to room temperature and neutralized by dropwise addition of a sodium bicarbonate aqueous solution. The resultant was extracted with ethyl acetate three times, dried over anhydrous sodium sulfate, and concentrated under reduced pressure. Without purification, the concentrate underwent the next process.

### [Step-5] Preparation of (R)-N⁴-(3-amino-5-(trifluoromethyl)phenyl)ethyl)-6-methoxy-2-methyl-N⁷-(tetrahydro-2H-pyran-4-yl)quinazoline-4,7-diamine

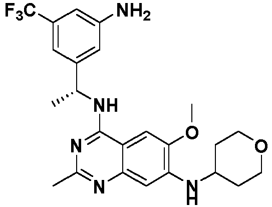

The 4-chloro-6-methoxy-2-methyl-N-(tetrahydro-2H-pyran-4-yl) quinazoline-7-amine (130 mg, 0.42 mmol) obtained in [Step-4] above was dissolved in 2 mL of DMF, and (*R*)-3-(1-aminoethyl)-5-(difluoromethyl)-4-fluoroaniline (163 mg, 0.67 mmol) and DIPEA (0.23 mL, 1.26 mmol) were added thereto. The mixture was stirred at 90°C for 13 hours. After the reaction was completed, the reaction solution was cooled to room temperature, followed by dropwise addition of water thereto. The resultant was extracted three times with ethyl acetate, dried over anhydrous sodium sulfate, and concentrated under reduced pressure. The residue was purified by column chromatography (dichloromethane methanol = 23:1 (v/v)) to produce 2 mg of the title compound at 1% yield.

¹H-NMR (300 MHz, CD₃OD): *δ* 7.52 (s, 1H), 6.96 (m, 2H), 6.80 (m, 1H), 6.61 (s, 1H), 5.63 (m, 1H), 3.98 (m, 5H), 3.68 (m, 1H), 3.58 (m, 2H), 2.44 (s, 3H), 2.04 (m, 2H), 1.67 (m, 5H).
MS (ESI+, m/z): 476.2 [M+H]⁺

**[Table 1]**

| Compound Number | Structure | Name | MS[M+H]+ |
|---|---|---|---|
| | 1H-NMR spectrum (300 MHz) | | |
| 1 | 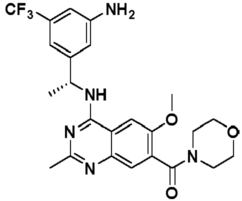 | (*R*)-(4-((1-(3-amino-5-(trifluoromethyl)phenyl)ethyl)amin o)-6-methoxy-2-methylquinazoline-7-yl)(morpholino)methanone | 490.2 |
| | ¹H-NMR (300 MHz, DMSO-*d₆*): *δ* 8.29 (d, 1H), 7.85 (s, 1H), 7.40 (s, 1H), 6.90 (d, 2H), 6.71 (s, 1H), 5.62 (m, 1H), 5.57 (s, 2H), 3.94 (s, 3H), 3.50 (m, 4H), 3.37 (m, 2H), 3.11 (m, 2H), 2.50 (s, 3H), 1.58 (m, 3H). | | |
| 2 | 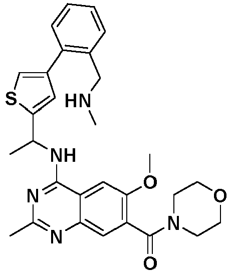 | (6-methoxy-2-methyl-4-((1-(4-(2-((methylamino)methyl)phenyl)thio phen-2-yl)ethyl)amino)quinazoline-7-yl)(morpholino)methanone | 532.2 |
| | ¹H-NMR (300 MHz, DMSO-*d₆*): *δ* 8.45 (m, 1H), 7.81 (s, 1H), 7.48 (m, 3H), 7.29 (m, 4H), 6.00 (m, 1H), 4.09 (m, 3H), 3.65 (m, 4H), 3.58 (m, 2H), 3.50 (m, 2H), 3.11 (m, 2H), 2.51 (m, 3H), 2.23 (s, 3H), 1.94 (m, 1H), 1.71 (m, 3H). | | |
| 3 | 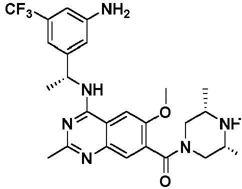 | (4-(((R)-1-(3-amino-5-(trifluoromethyl)phenyl)ethyl)amin o)-6-methoxy-2-methylquinazoline-7-yl)((3R, 5*S*)-3,5-dimethylpiperazine-1-yl)methanone | 517.3 |
| | ¹H-NMR (300 MHz, DMSO-*d₆*): *δ* 8.28 (d, 1H), 7.84 (m, 1H), 7.36 (d, 1H), 6.93 (m, 2H), 6.72 (s, 1H), 5.62 (m, 3H), 4.41 (m, 1H), 3.92 (s, 3H), 3.07 (m, 1H), 2.68 (m, 3H), 2.40 (d, 3H), 2.28 (m, 3H), 1.60 (m, 3H), 1.03 (d, 3H), 0.82 (m, 3H). | | |
| 4 | 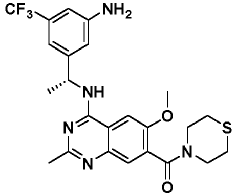 | (*R*)-(4-((1-(3-amino-5-(trifluoromethyl)phenyl)ethyl)amin o)-6-methoxy-2-methylquinazoline-7-yl)(thiomorpholino)methanone | 506.2 |
| | ¹H-NMR (300 MHz, DMSO-*d₆*): *δ* 8.27 (d, 1H), 7.85 (d, 1H), 7.41 (s, 1H), 6.88 (m, 2H), 6.71 (s, 1H), 5.59 (m, 3H), 3.88 (m, 5H), 3.36 (m, 2H), 2.73 (m, 2H), 2.50 (m, 2H), 2.38 (s, 3H), 1.58 (m, 3H). | | |
| 5 | 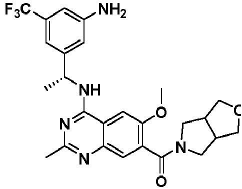 | (4-(((*R*)-1-(3-amino-5-(trifluoromethyl)phenyl)ethyl)amin o)-6-methoxy-2-methylquinazoline-7-yl)(tetrahydro-1*H*-furo[3,4-*c*]pyrrol-5(3*H*)-yl)methanone | 516.2 |
| | ¹H-NMR (300 MHz, CD₃OD): *δ* 7.82 (s, 1H), 7.52 (s, 1H), 7.00 (m, 2H), 6.83 (s, 1H), 5.66 (m, 1H), 4.02 (s, 3H), 3.94 (m, 2H), 3.84 (m, 2H), 3.66 (m, 2H), 3.18 (m, 4H), 2.49 (s, 3H), 1.67 (d, *J* = 6.9 Hz, 3H). | | |
| 6 | 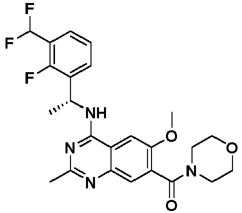 | (*R*)-(4-((1-(3-(difluoromethyl)-2-fluorophenyl)ethyl)amino)-6-methoxy-2-methylquinazoline-7-yl)(morpholino)methanone | 475.2 |
| | ¹H-NMR (300 MHz, DMSO-*d₆*): *δ* 8.40 (m, 1H), 7.89 (s, 1H), 7.68 (m, 1H), 7.66 (m, 1H), 7.50 (s, 1H), 7.40-7.06 (m, 2H), 5.82 (m, 1H), 3.97 (s, 3H), 3.50 (m, 4H), 3.42 (m, 2H), 3.29 (m, 2H), 2.33 (s, 3H), 1.64 (m, 3H). | | |
| 7 | 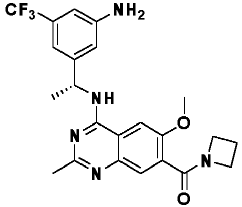 | (*R*)-(4-((1-(3-amino-5-(trifluoromethyl)phenyl)ethyl)amin o)-6-methoxy-2-methylquinazoline-7-yl)(azetidine-1-yl)methanone | 460.2 |
| | ¹H-NMR (300 MHz, DMSO-*d₆*): *δ* 8.28 (d, 1H), 7.83 (s, 1H), 7.43 (s, 1H), 6.90 (d, 2H), 6.71 (s, 1H), 5.64 (m, 3H), 4.06 (m, 2H), 3.95 (s, 3H), 3.88 (m, 2H), 2.42 (s, 3H), 2.28 (m, 2H), 1.59 (d, 3H). | | |
| 8 | 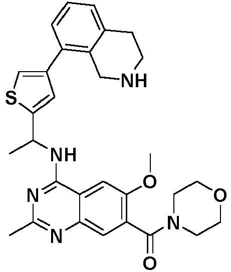 | (6-methoxy-2-methyl-4-((1-(4-(1,2,3,4-tetrahydroisoquinolin-8-yl)thiophen-2-yl)ethyl)amino)quinazoline-7-yl)(morpholino)methanone | 544.2 |
| | ¹H-NMR (300 MHz, CD₃OD): *δ* 7.77 (s, 1H), 7.54 (s, 1H), 7.13 (m, 5H), 6.00 (m, 1H), 4.13 (s, 3H), 3.82 (m, 4H), 3.61 (m, 2H), 3.32 (m, 2H), 3.13 (m, 2H), 2.87 (m, 4H), 2.57 (s, 3H), 1.83 (m, 3H). | | |
| 9 | 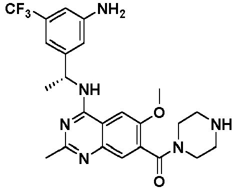 | (*R*)-(4-((1-(3-amino-5-(trifluoromethyl)phenyl)ethyl)amin o)-6-methoxy-2-methylquinazoline-7-yl)(piperazine-1-yl)methanone | 489.2 |
| | ¹H-NMR (300 MHz, CD₃OD): *δ* 8.09 (s, 1H), 7.61 (s, 1H), 6.98 (m, 2H), 6.84 (s, 2H), 5.79 (m, 1H), 4.06 (m, 5H), 3.54 (m, 2H), 3.34 (m, 2H), 3.20 (m, 2H), 2.65 (s, 3H), 1.74 (d, 3H). | | |
| 10 | 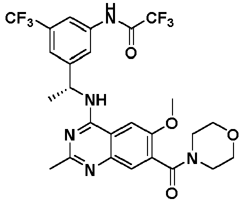 | (*R*)-2,2,2-trifluoro-*N*-(3-(1-((6-methoxy-2-methyl-7-(morpholine-4-carbonyl)quinazoline-4-yl)amino)ethyl)-5-(trifluoromethyl)phenylacetamide | 586.2 |
| | ¹H-NMR (300 MHz, CD₃OD): *δ* 8.11-8.08 (d, 1H), 7.91-7.88 (d, 1H), 7.88 (s, 1H), 7.66 (s, 1H), 7.52 (s, 1H), 5.74 (m, 1H), 4.86 (s, 6H), 4.03 (s, 3H), 3.79 (m, 4H), 3.63 (m, 2H), 3.32 (m, 2H), 2.47 (s, 3H), 1.76 (d, 3H). | | |
| 11 | 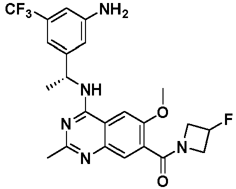 | (*R*)-(4-((1-(3-amino-5-(trifluoromethyl)phenyl)ethyl)amin o)-6-methoxy-2-methylquinazoline-7-yl)(3-fluorazetidine-1-yl)methanone | 478.2 |
| | ¹H-NMR (300 MHz, CD₃OD): *δ* 8.03 (s, 1H), 7.64 (s, 1H), 7.00 (m, 2H), 6.85 (s, 1H), 5.78 (m, 1H), 5.34 (m, 1H), 4.51 (m, 1H), 4.28 (m, 3H), 4.08 (s, 3H), 2.63 (s, 3H), 1.74 (d, 3H). | | |
| 12 | 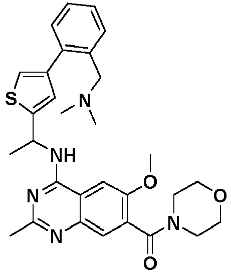 | (4-((1-(4-(2-((dimethylamino)methyl)phenyl)thi ophen-2-yl)ethyl)amino)-6-methoxy-2-methoxyquinazoline-7-yl)(morpholino)methanone | 546.2 |
| | ¹H-NMR (300 MHz, CD₃OD): *δ* 7.77 (s, 1H), 7.49 (m, 2H), 7.39 (m, 3H), 7.26 (s, 1H), 7.17 (s, 1H), 6.09 (m, 1H), 3.97 (m, 5H), 3.77 (m, 4H), 3.62 (m, 2H), 3.26 (m, 2H), 2.55 (s, 3H), 2.38 (s, 6H), 1.84 (d, 3H). | | |
| 13 | 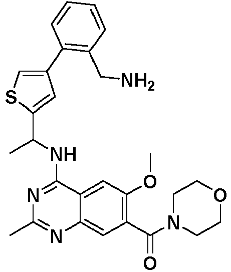 | (4-((1-(4-(2-((aminomethyl)phenyl)thiophen-2-yl)ethyl)amino)-6-methoxy-2-methylquinazoline-7-yl)(morpholino)methanone | 518.2 |
| | ¹H-NMR (300 MHz, CD₃OD): *δ* 7.78 (s, 1H), 7.52 (m, 2H), 7.30 (m, 5H), 6.10 (m, 1H), 4.57 (s, 2H), 3.98 (s, 3H), 3.76 (m, 4H), 3.62 (m, 2H), 3.26 (m, 2H), 2.56 (s, 3H), 1.82 (d, 3H). | | |
| 14 | 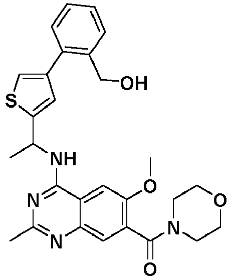 | (4-((1-(4-(2-((hydroxymethyl)phenyl)thiophen-2-yl)ethyl)amino)-6-methoxy-2-methylquinazoline-7-yl)(morpholino)methanone | 519.2 |
| | ¹H-NMR (300 MHz, CD₃OD): *δ* 7.76 (s, 1H), 7.51 (m, 2H), 7.31 (m, 5H), 6.10 (m, 1H), 4.57 (s, 2H), 3.98 (s, 3H), 3.76 (m, 4H), 3.60 (m, 2H), 3.27 (m, 2H), 2.55 (s, 3H), 1.24 (d, 3H). | | |
| 15 | 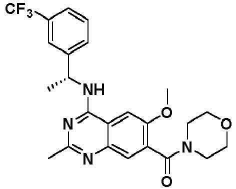 | (*R*)-(6-methoxy-2-methyl-4-((1-(3-(trifluoromethyl)phenyl)ethyl)amin o)quinazolin-7-yl)(morpholino)methanone | 475.2 |
| | ¹H-NMR (300 MHz, DMSO-*d₆*): *δ* 8.38 (d, 1H), 7.85 (s, 2H), 7.78 (d, 1H), 7.60 (d, 2H), 7.41 (s, 1H), 5.74 (m, 1H), 3.96 (s, 3H), 3.65 (m, 4H), 3.49 (m, 2H), 3.12 (m, 2H), 2.37 (s, 3H), 1.67 (m, 3H). | | |
| 16 | 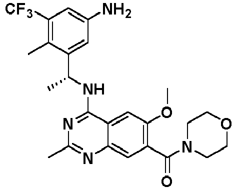 | (*R*)-(4-((1-(5-amino-2-methyl-3-(trifluoromethyl)phenyl)ethyl)amin o)-6-methoxy-2-methylquinazolin-7-yl)(morpholino)methanone | 504.2 |
| | ¹H-NMR (300 MHz, DMSO-*d₆*): *δ* 8.36 (d, 1H), 7.91 (s, 1H), 7.39 (s, 1H), 6.92 (d, 2H), 6.79 (s, 1H), 5.68 (m, 1H), 5.25 (s, 2H), 3.96 (s, 3H), 3.65 (m, 4H), 3.51 (m, 2H), 3.12 (m, 2H), 2.38 (d, 6H), 1.55 (m, 3H). | | |
| 17 | 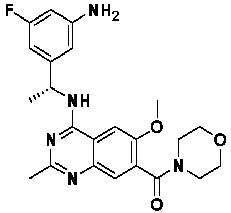 | (*R*)-(4-((1-(3-amino-5-(fluorophenyl)ethyl)amino)-6-methoxy-2-methylquinazolin-7-yl)(morpholino)methanone | 440.2 |
| | ¹H-NMR (300 MHz, CD₃OD): *δ* 8.00 (s, 1H), 7.53 (s, 1H), 6.58 (s, 1H), 6.44 (m, 1H), 6.31 (m, 1H), 5.73 (m, 1H), 4.04 (s, 3H), 3.76 (m, 4H), 3.61 (m, 2H), 3.27 (m, 2H), 2.61 (s, 3H), 1.69 (d, 3H). | | |
| 18 | 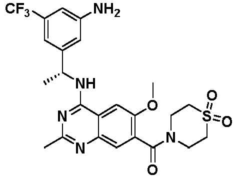 | (*R*)-(4-((1-(3-amino-5-(trifluoromethyl)phenyl)ethyl)amin o)-6-methoxy-2-methylquinazolin-7-yl)(1,1-dioxothiomorpholino)methanone | 538.1 |
| | ¹H-NMR (300 MHz, CD₃OD): *δ* 7.85 (s, 1H), 7.58 (s, 1H), 7.01 (m, 2H), 6.83 (s, 1H), 5.67 (m, 1H), 4.26 (m, 2H), 4.03 (s, 3H), 3.68 (m, 2H), 3.24 (m, 2H), 3.13 (m, 2H), 2.50 (s, 3H), 1.67 (d, *J* = 6.9 Hz, 3H). | | |
| 19 | 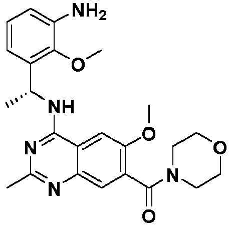 | (*R*)-(4-((1-(3-amino-2-methoxyphenyl)ethyl)amino)-6-methoxy-2-methylquinazolin-7-yl)(morpholino)methanone | 452.2 |
| | ¹H-NMR (300 MHz, DMSO-*d₆*): *δ* 8.24 (m, 1H), 7.92 (s, 1H), 7.38 (s, 1H), 6.78 (m, 1H), 6.67 (m, 1H), 6.57 (m, 1H), 5.92 (m, 1H), 4.92 (m, 2H), 3.97 (s, 3H), 3.90 (d, 3H), 3.65 (m, 4H), 3.51 (m, 2H), 3.13 (m, 2H), 2.34 (s, 3H), 1.52 (m, 3H). | | |
| 20 | 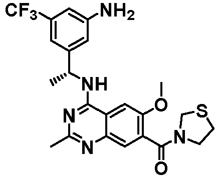 | (R)-(4-((1-(3-amino-5-(trifluoromethyl)phenyl)ethyl)amin o)-6-methoxy-2-methylquinazolin-7-yl)(thiazolidine-3-yl)methanone | 492.2 |
| | ¹H-NMR (300 MHz, DMSO-*d₆*): *δ* 8.31 (d, 1H), 7.88 (s, 1H), 7.44 (s, 1H), 6.90 (d, 2H), 6.72 (s, 1H), 5.63 (m, 3H), 4.64 (s, 1H), 4.22 (s, 1H), 3.95 (s, 3H), 3.86 (m, 1H), 3.45 (m, 1H), 3.12 (m, 1H), 2.99 (m, 1H), 2.39 (s, 3H), 1.60 (d, 3H). | | |
| 21 | 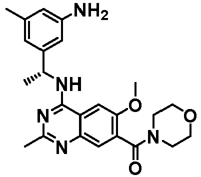 | (R)-(4-((1-(3-amino-5-methylphenyl)ethyl)amino)-6-methoxy-2-methylquinazolin-7-yl)(morpholino)methanone | 436.2 |
| | ¹H-NMR (300 MHz, DMSO-*d₆*): *δ* 8.20 (m, 1H), 7.87 (s, 1H), 7.39 (s, 1H), 6.63 (m, 2H), 6.48 (m, 1H), 5.60 (m, 1H), 4.92 (m, 2H), 3.94 (d, 3H), 3.65 (s, 4H), 3.54 (m, 2H), 3.12 (m, 2H), 2.40 (d, 3H), 2.20 (d, 3H), 1.55 (m, 3H). | | |
| 22 | 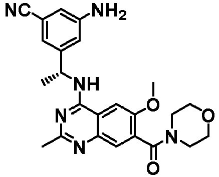 | (*R*)-3-amino-5-(1-((6-methoxy-2-methyl-7-(morpholine-4-carbonyl)quinazolin-4-yl)amino)ethyl)benzonitrile | 447.2 |
| | ¹H-NMR (300 MHz, CD₃OD): *δ* 7.80 (s, 1H), 7.49 (s, 1H), 7.02 (m, 2H), 6.80 (s, 1H), 5.58 (m, 1H), 4.01 (s, 3H), 3.76 (m, 4H), 3.61 (m, 2H), 3.27 (m, 2H), 2.47 (s, 3H), 1.64 (d, 3H) | | |
| 23 | 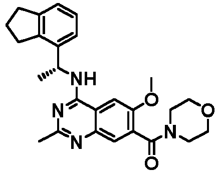 | (*R*)-(4-((1-(2,3-dihydro-1*H-*indene-4-yl)ethyl)amino)-6-methoxy-2-methylquinazoline-7-yl)(morpholino)methanone | 447.2 |
| | ¹H-NMR (300 MHz, DMSO-*d₆*): *δ* 8.29 (m, 1H), 7.86 (s, 1H), 7.37 (s, 1H), 7.27 (m, 1H), 7.09 (s, 1H), 5.63 (m, 1H), 3.95 (s, 3H), 3.64 (m, 4H), 3.48 (m, 2H), 3.32 (m, 2H), 2.95 (m, 2H), 2.36 (s, 3H), 2.09 (m, 2H), 1.58 (m, 3H) | | |
| 24 | 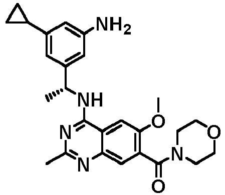 | (*R*)-(4-((1-(3-amino-5-cyclopropylphenyl)ethyl)amino)-6-methoxy-2-methylquinazoline-7-yl)(morpholino)methanone | 462.2 |
| | ¹H-NMR (300 MHz, DMSO-*d₆*): *δ* 8.18 (d, 1H), 7.86 (s, 1H), 7.38 (s, IH), 6.41 (s, 2H), 6.10 (s, 1H), 5.55 (m, 1H), 4.89 (s, 2H), 3.95 (s, 3H), 3.65 (m, 4H), 3.50 (m, 2H), 3.38 (m, 2H), 3.11 (s, 2H), 2.39 (s, 3H), 1.83 (m, 1H), 1.53 (m, 3H), 0.83 (m, 2H), 0.55 (m, 2H) | | |
| 25 | 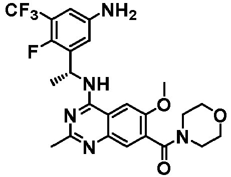 | (*R*)-(4-((1-(5-amino-2-ffuoro-3-(trifluoromethyl)phenyl)ethyl)amin o)-6-methoxy-2-methylquinazoline-7-yl)(morpholino)methanone | 508.1 |
| | ¹H-NMR (300 MHz, CD₃OD): *δ* 7.84 (m, 1H), 7.47 (s, 1H), 6.92 (m, 1H), 6.77 (m, 1H), 5.72 (m, 1H), 4.00 (s, 3H), 3.78 (m, 4H), 3.57 (m, 2H), 3.22 (m, 2H), 2.39 (m, 3H), 1.64 *(d, J=* 7.4 Hz, 3H). | | |
| 26 | 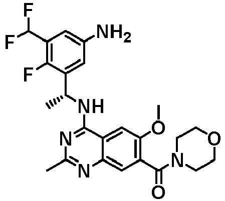 | (*R*)-(4-((1-(5-amino-3-(difluoromethyl)-2-fluorophenyl)ethyl)amino)-6-methoxy 2-methylquinazolin-7-yl)(morpholino)methanone | 490.2 |
| | ¹H-NMR (300 MHz, DMSO-*d₆*): *δ* 8.30 (m, 1H), 7.92 (s, 1H), 7.41 (s, 1H), 7.09 (m, 1H), 6.79 (d, 1H), 6.63 (m, 1H), 5.78 (m, 1H), 5.20 (s, 2H), 3.97 (s, 3H), 3.66 (s, 4H), 3.54 (m, 2H), 3.13 (m, 2H), 2.35 (s, 3H), 1.60 (m, 3H). | | |
| 27 | 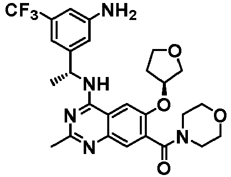 | (4-(((*R*)-1-(3-amino-5-(trifluoromethyl)phenyl)ethyl)amin o)-2-methyl-6-(((,S)-tetrahydrofuran-3-yl)oxy)quinazoline-7-yl)(morpholino)methanone | 546.2 |
| | ¹H-NMR (300 MHz, CD₃OD): *δ* 7.79 (m, 1H), 7.58 (s, 1H), 7.52 (s, 1H), 7.01 (m, 1H), 6.83 (s, 1H), 5.67 (m, 1H), 5.25 (m, 1H), 3.94 (m, 4H), 3.81 (m, 4H), 3.62 (m, 2H), 3.34 (m, 2H), 2.49 (s, 3H), 2.38 (m, 2H), 1.68 (d, *J* = 5.4 Hz, 3H). | | |
| 28 | 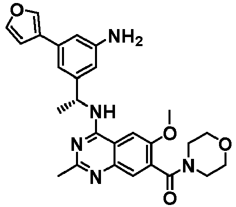 | (*R*)-(4-((1-(3-amino-5-(furan-3-yl)phenyl)ethyl)amino)-6-methoxy-2-methylquinazoline-7-yl)(morpholino)methanone | 488.2 |
| | ¹H-NMR (300 MHz, CD₃OD): *δ* 8.04 (s, 1H), 7.80 (s, 1H), 7.51 (m, 2H), 6.97 (s, 1H), 6.83 (s, 1H), 6.74 (m, 2H), 5.80 (m, 1H), 4.04 (s, 3H), 3.78 (m, 4H), 3.61 (m, 2H), 3.27 (m, 2H), 2.64 (s, 3H), 1.75 (d, 3H) | | |
| 29 | 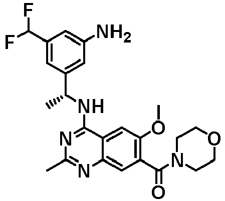 | (*R*)-(4-((1-(3-amino-5-(difluoromethyl)phenyl)ethyl)amin o)-6-methoxy-2-methylquinazoline-7-yl)(morpholino)methanone | 472.2 |
| | ¹H-NMR (300 MHz, CD₃OD): *δ* 7.82 (s, 1H), 7.49 (s, 1H), 6.91 (s, 2H), 6.58 (s, 1H), 6.39 (m, 1H), 5.68 (m, 1H), 4.00 (s, 3H), 3.76 (m, 4H), 3.60 (m, 2H), 3.27 (m, 2H), 2.49 (s, 3H), 1.67 (d, 3H) | | |
| 30 | 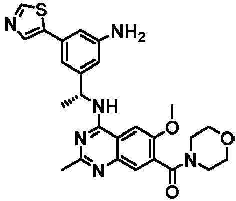 | (*R*)-(4-((1-(3-amino-5-(thiazole-5-yl)phenyl)ethyl)amino)-6-methoxy-2-methylquinazoline-7-yl)(morpholino)methanone | 505.2 |
| | ¹H-NMR (300 MHz, CD₃OD): *δ* 8.89 (s, 1H), 8.06 (d, 1H), 7.81 (s, 1H), 7.49 (s, 1H), 7.06 (s, 1H), 6.87 (s, 1H), 6.84 (m, 1H), 5.65 (m, 1H), 4.00 (s, 3H), 3.78 (m, 4H), 3.61 (m, 2H), 3.27 (m, 2H), 2.48 (s, 3H), 1.69 (d, 3H) | | |
| 31 | 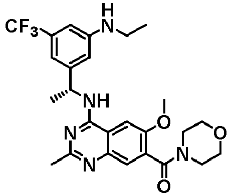 | (*R*)-(4-((1-(3-(ethylamino)-5-(trifluoromethyl)phenyl)ethyl)amin o)-6-methoxy-2-methylquinazoline-7-yl)(morpholino)methanone | 518.2 |
| | ¹H-NMR (300 MHz, CD₃OD): *δ* 7.82 (d, 1H), 7.51 (s, 1H), 6.98 (d, 2H), 6.71 (s, 1H), 5.68 (m, 1H), 4.02 (s, 3H), 3.77 (m, 4H), 3.63 (m, 2H), 3.35 (m, 2H), 3.28 (m, 2H), 2.49 (s, 3H), 1.68 (d, *J* = 6.9 Hz, 3H), 1.25 (m, 3H). | | |
| 32 | 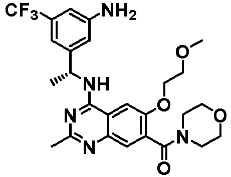 | Methyl (*R*)-(4-((1-(3-amino-5-(trifluoromethyl)phenyl)ethyl)amin o)-6-(2-methoxyethoxy)-2-methylquinazolin-7-yl)(morpholino)methanone | 534.2 |
| | ¹H-NMR (300 MHz, DMSO-*d₆*): *δ* 8.31 (m, 1H), 7.86 (s, 1H), 7.41 (s, 1H), 6.89 (m, 2H), 6.71 (s, 1H), 5.57 (m, 3H), 4.28 (m, 2H), 3.94-3.50 (m, 8H), 3.37 (m, 3H), 3.16 (m, 2H), 2.39 (s, 3H), 1.59 (m, 3H). | | |
| 33 | 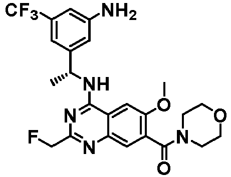 | (*R*)-(4-((1-(3-amino-5-(trifluoromethyl)phenyl)ethyl)amin o)-2-(fluoromethyl)-6-methoxyquinazolin-7-yl)(morpholino)methanone | 508.2 |
| | ¹H-NMR (300 MHz, CD₃OD): *δ* 7.92 (s, 1H), 7.68 (s, 1H), 7.00 (m, 2H), 6.83 (s, 1H), 5.68 (m, 1H), 5.51 (m, 1H), 5.39 (m, 1H), 4.01 (s, 3H), 3.79 (m, 4H), 3.63 (m, 2H), 3.23 (m, 2H), 1.69 (d, *J* = 7.2 Hz, 3H). | | |
| 34 | 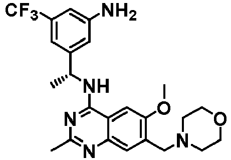 | (*R*)-*N*-(1-(3-amino-5-(trifluoromethyl)phenyl)ethyl)-6-methoxy-2-methyl-7-(morpholinomethyl)quinazoline-4-amine | 476.2 |
| | ¹H-NMR (300 MHz, DMSO-*d₆*): *δ* 8.11 (d, 1H), 7.71 (s, 1H), 7.57 (s, 1H), 6.85 (m, 2H), 6.69 (s, 1H), 5.57 (m, 3H), 3.92 (s, 3H), 3.61 (m, 4H), 3.57(s, 2H), 2.44 (m, 4H), 2.36 (s, 3H), 1.56 (d, 3H) | | |
| 35 | 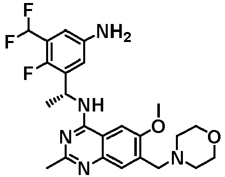 | (*R*)-*N*-(1-(5-amino-3-(difluoromethyl)-2-fluorophenyl)ethyl)-6-methoxy-2-methyl-7-(morpholinomethyl)quinazoline-4-amine | 476.2 |
| | ¹H-NMR (300 MHz, DMSO-*d₆*): *δ* 8.11 (d, 1H), 7.76 (s, 1H), 7.55 (s, 1H), 7.06 (t, 1H), 6.74 (m, 1H), 6.58 (m, 1H), 5.72 (m, 3H), 5.16 (s, 2H), 3.94 (s, 3H), 3.60 (m, 4H), 3.56(s, 2H), 2.42 (m, 4H), 2.31 (s, 3H), 1.55 (d, 3H) | | |
| 36 | 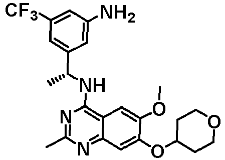 | (*R*)-*N*-(1-(3-amino-5-(trifluoromethylphenyl)ethyl)-6-methoxy-2-methyl-7-((tetrahydro-2*H*-pyran-4-yl)oxy)quinazolin-4-amine | 477.2 |
| | ¹H-NMR (300 MHz, CD₃OD): *δ* 7.67 (s, 1H), 7.06 (s, 1H), 6.99 (m, 2H), 6.82 (s, 1H), 5.64 (m, 1H), 4.74 (m, 1H), 4.12 (m, 5H), 3.66 (m, 2H), 2.46 (s, 3H), 2.16 (m, 2H), 1.86 (m, 2H), 1.67 (d, 3H) | | |
| 37 | 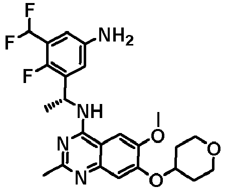 | (*R*) N (1-(5-amino-3-(difluoromethyl)-2-fluorophenyl)ethyl)-6-methoxy-2-methyl-7-((tetrahydro-2*H*-pyran-4-yl)oxy)quinazolin-4-amine | 477.2 |
| | ¹H-NMR (300 MHz, CD₃OD): *δ* 7.73 (s, 1H), 7.09 (s, 1H), 6.79 (m, 3H), 5.81 (m, 1H), 4.59 (m, 1H), 3.99 (m, 5H), 3.66 (m, 2H), 2.42 (s, 3H), 2.17 (m, 2H), 1.73 (m, 2H), 1.62 (d, 3H) | | |
| 38 | 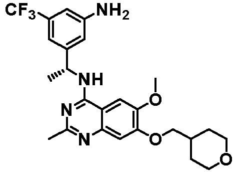 | (*R*)-*N*-(1-(3-amino-5-(trifluoromethyl)phenyl)ethyl)-6-methoxy-2-methyl-7-((tetrahydro-2*H*-pyran-4-yl)methoxy)quinazolin-4-amine | 491.2 |
| | ¹H-NMR (300 MHz, CD₃OD): *δ* 7.46 (s, 1H), 7.00 (s, 3H), 6.83 (s, 1H), 5.67(q, 1H), 4.08~4.10 (m, 7H), 3.73(m, 2H), 3.50 (t, 2h\H), 2.54 (s, 3H), 1.89(m, 2H), 1.68(d, 3H), 1.57(m, 2H) | | |
| 39 | 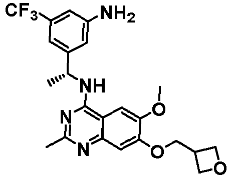 | (*R*)-*N*-(1-(3-amino-5-(trifluoromethyl)phenyl)ethyl)-6-methoxy-2-methyl-7-(oxetan-3-ylmethoxy)quinazoline-4-amine | 463.2 |
| | ¹H-NMR (300 MHz, DMSO-*d₆*): *δ* 8.00 (d, 1H), 7.71 (s, 1H), 7.10 (s, 1H), 6.89 (d, 2H), 6.70 (s, 1H), 5.59 (m, 3H), 4.76 (m, 2H), 4.47 (m, 2H), 4.34 (m, 2H), 3.90 (s, 3H), 3.50 (m, 1H), 2.36 (s, 3H), 1.57 (d, 3H) | | |
| 40 | 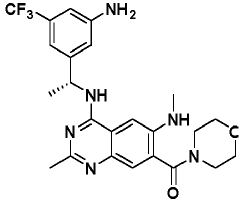 | (R)-(4-((1-(3-amino-5-(trifluoromethyl)phenyl)ethyl)amin o)-2-methyl-6-(methylamino)quinazolin-7-yl)(morpholino)methanone | 489.2 [M+H]⁺ |
| | ¹H-NMR (300 MHz, CD₃OD): *δ* 7.38 (s, 1H), 7.28 (s, 1H), 6.99 (m, 2H), 6.82 (s, 1H), 5.67 (m, 1H), 3.73 (m, 8H), 2.96 (s, 3H), 2.46 (s, 3H), 1.67 (d, *J=* 7.2 Hz, 3H). | | |
| 41 | 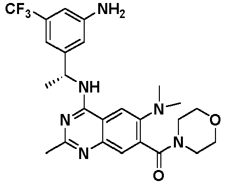 | (*R*)-(4-((1-(3-amino-5-(trifluoromethyl)phenyl)ethyl)amin o)-6-(dimethylamino)-2-methylquinazolin-7-yl)(morpholino)methanone | 503.2 [M+H]⁺ |
| | ¹H-NMR (300 MHz, DMSO-*d₆*): *δ* 7.77 (d, 1H), 7.38 (s, 1H), 6.90 (d, 2H), 6.74 (s, 1H), 5.68 (m, 3H), 3.77 (m, 6H), 3.11 (m, 2H), 2.87 (d, 6H), 2.47 (s, 3H), 1.63 (m, 3H). | | |
| 42 | 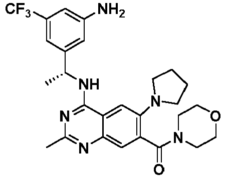 | (*R*)-(4-((1-(3-amino-5-(trifluoromethyl)phenyl)ethyl)amin o)-2-methyl-6-(pyrrolidin-1 - yl)quinazoline-7-yl)(morpholino)methanone | 529.3 [M+H]⁺ |
| | ¹H-NMR (300 MHz, DMSO-*d₆*): *δ* 8.17 (d, 1H), 7.34 (m, 2H), 6.90 (d, 2H), 6.70 (s, 1H), 5.65 (m, 3H), 3.76 (m, 6H), 3.28 (m, 6H), 2.35 (d, 3H), 1.98 (m, 4H), 1.58 (m, 3H). | | |
| 43 | 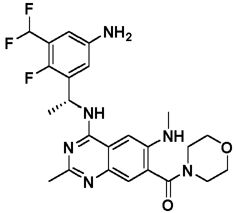 | (*R*)-(4-((1-(5-amino-3-(difluoromethyl)-2-fluorophenyl)ethyl)amino)-2-methyl-6-(methylamino)quinazoline-7-yl)(morpholino)methanone | 489.2 [M+H]⁺ |
| | ¹H-NMR (300 MHz, CD₃OD): *δ* 7.38 (s, 1H), 7.32 (s, 1H), 7.09 (m, 2H), 5.78 (m, 1H), 3.67 (m, 8H), 2.98 (s, 3H), 2.42 (s, 3H), 1.67 (d, *J* = 7.2 Hz, 3H). | | |
| 44 | 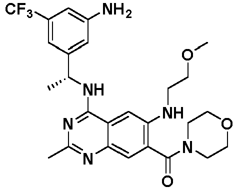 | (*R*)-(4-((1-(3-amino-5-(trifluoromethyl)phenyl)ethyl)amin o)-6-((2-methoxyethyl)amino)-2-methylquinazoline-7-yl)(morpholino)methanone | 533.2 [M+H]⁺ |
| | ¹H-NMR (300 MHz, CD₃OD): *δ* 7.39 (m, 2H), 6.99 (m, 1H), 6.83 (s, 1H), 5.65 (m, 1H), 3.76 (m, 8H), 3.68 (m, 4H), 3.66 (s, 3H), 2.52 (s, 3H), 1.66 (d, *J* = 6.9 Hz, 3H). | | |
| 45 | 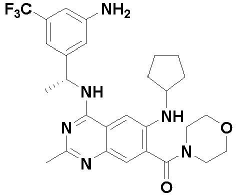 | (*R*)-(4-((1-(3-amino-5-(trifluoromethyl)phenyl)ethyl)amin o)-6-(cyclopentylamino)-2-methylquinazoline-7-yl)(morpholino)methanone | 543.2 [M+H]⁺ |
| | ¹H-NMR (300 MHz, CD₃OD): *δ* 7.44 (s, 1H), 7.38 (s, 1H), 6.99 (m, 2H), 6.84 (m, 1H), 5.73 (m, 1H), 4.08 (m, 1H), 3.67 (m, 8H), 2.52 (s, 3H), 1.81 (m, 4H), 1.73 (m, 3H), 1.53 (m, 4H). | | |
| 46 | 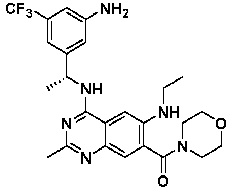 | (*R*)-(4-((1-(3-amino-5-(trifluoromethyl)phenyl)ethyl)amin o)-6-(ethylamino)-2-methylquinazoline-7-yl)(morpholino)methanone | 503.2 [M+H]⁺ |
| | ¹H-NMR (300 MHz, DMSO-*d₆*): *δ* 8.74 (s, 1H), 7.37 (d, 2H), 6.89 (d, 2H), 6.73 (s, 1H), 5.76 (m, 4H), 3.65 (m, 6H), 3.33 (m, 4H), 2.43 (s, 3H), 1.62 (d, 3H), 1.26 (m, 3H). | | |
| 47 | 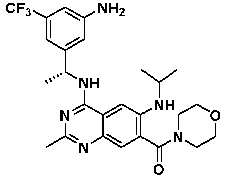 | (*R*)-(4-((1-(3-amino-5-(trifluoromethyl)phenyl)ethyl)amin o)-6-(isopropylamino)-2-methylquinazoline-7-yl)(morpholino)methanone | 517.2 [M+H]⁺ |
| | ¹H-NMR (300 MHz, CD₃OD): *δ* 7.36 (m, 2H), 6.98 (m, 2H), 6.80 (s, 1H), 5.64 (m, 1H), 3.92 (m, 1H), 3.69 (m, 8H), 2.42 (s, 3H), 1.64 (d, 3H), 1.27 (m, 6H). | | |
| 48 | 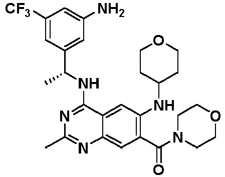 | (*R*)-(4-((1-(3-amino-5-(trifluoromethyl)phenyl)ethyl)amin o)-2-methyl-6-((tetrahydro-2*H-*pyran-4-yl) amino)quinazoline-7-yl)(morpholino)methanone | 559.2 [M+H]⁺ |
| | ¹H-NMR (300 MHz, DMSO-*d₆*): *δ* 7.42 (s, 1H), 7.34 (s, 1H), 6.88 (s, 1H), 6.85 (s, 1H), 6.42 (s, 1H), 5.68 (p, 1H), 5.57(br, 2H), 5.15(d, 1H), 3.90 (d, 2H), 3.82-3.48 (br, 10H), 2.41 (s, 3H), 1.92(d, 2H), 1.62 (d, 3H), 1.57-1.48(m, 2H). | | |
| 49 | 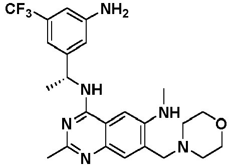 | (*R*)-*N*⁴-(1-(3-amino-5-(trifluoromethyl)phenyl)ethyl)-N⁶,2-dimethyl-7-(morpholinomethyl)quinazoline-4,6-diamine | 475.2 [M+H]⁺ |
| | ¹H-NMR (300 MHz, CDCl₃): *δ* 7.52 (s, 1H), 7.11 (s, 1H), 7.00 (m, 1H), 6.82 (s, 1H), 6.49 (m, 2H), 5.71 (m, 1H), 3.89 (s, 2H), 3.71 (m, 6H), 2.97 (s, 3H), 2.59 (s, 3H), 2.44 (m, 4H), 1.71 (d, 3H). | | |
| 50 | 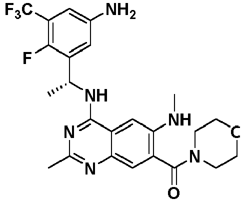 | (*R*)-(4-((1-(5-amino-2-ffuoro-3-(trifluoromethyl)phenyl)ethyl)amin o)-2-methyl-6-(methylamino)quinazoline-7-yl)(morpholino)methanone | 507.2 [M+H]⁺ |
| | ¹H-NMR (300 MHz, CD₃OD): δ 7.38 (s, 1H), 7.32 (s, 1H), 7.09 (m, 2H), 6.95 (m, 1H), 6.81 (m, 1H), 5.75 (m, 1H), 3.68 (m, 8H), 2.98 (s, 3H), 2.41 (s, 3H), 1.68 (d, J = 6.9 Hz, 3H). | | |
| 51 | 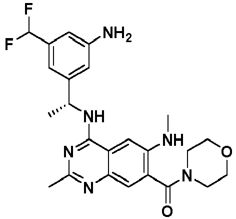 | (*R*)-(4-((1-(3-amino-5-(difluoromethyl)phenyl)ethyl)amin o)-2-methyl-6-(methylamino)quinazoline-7-yl)(morpholino)methanone | 471.2 [M+H]⁺ |
| | ¹H-NMR (300 MHz, DMSO-*d₆*): δ 9.15 (d, 1H), 7.34 (d, 2H), 7.03-6.66 (m, 3H), 6.62 (s, 1H), 5.81 (d, 1H), 5.73 (m, 1H), 5.45 (d, 2H), 3.68-3.51(m, 6H), 3.32(s, 2H), 2.88 (d, 3H), 2.48 (s, 3H), 1.62 (d, 3H). | | |
| 52 | 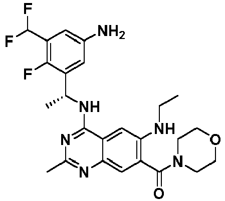 | (*R*)-(4-((1-(5-amino-3-(difluoromethyl)-2-fluorophenyl)ethyl)amino)-6-(ethylamino)-2-methylquinazoline-7-yl)(morpholino)methanone | 503.2 [M+H]⁺ |
| | ¹H-NMR (300 MHz, DMSO-*d₆*): δ 8.53 (s, 1H), 7.41 (d, 2H), 7.27-6.90 (m, 1H), 6.76 (d, 1H), 6.63 (m, 1H), 5.81 (m, 1H), 5.36 (s, 1H), 5.27 (d, 2H), 3.64(s, 6H), 3.32(m, 4H), 2.37 (s, 3H), 1.60 (d, 3H), 1.28 (m, 3H). | | |
| 53 | 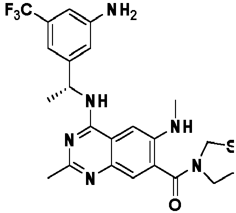 | (*R*)-(4-((1-(3-amino-5-(trifluoromethyl)phenyl)ethyl)amin o)-2-methyl-6-(methylamino)quinazoline-7-yl)(thiazolidine-3-yl)methanone | 491.2 [M+H]⁺ |
| | ¹H-NMR (300 MHz, DMSO-*d₆*): *δ* 8.10 (d, 1H), 7.36 (s, 1H), 7.22 (s, 1H), 6.90 (d, 2H), 6.70 (s, 1H), 5.61-5.53 (m, 4H), 4.55 (m, 2H), 3.72 (m, 2H), 3.04 (m, 2H), 2.87 (m, 3H), 2.34 (s, 3H), 1.58 (d, 3H). | | |
| 54 | 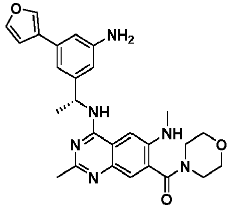 | (*R*)-(4-((1-(3-amino-5-(furan-3-yl)phenyl)ethyl)amino)-2-methyl-6-(methylamino)quinazolin-7-yl)(morpholino)methanone | 487.2 [M+H]⁺ |
| | ¹H-NMR (300 MHz, CD₃OD): δ 7.80 (s, 1H), 7.52 (s, 1H), 7.38 (s, 1H), 7.29 (s, 1H), 7.01 (s, 1H), 6.77 (s, 1H), 6.75 (s, 1H), 6.72 (s, 1H), 5.67 (m, 1H), 3.66 (m, 8H), 2.96 (s, 3H), 2.46 (s, 3H), 1.67 (d, J = 7.2 Hz, 3H). | | |
| 55 | 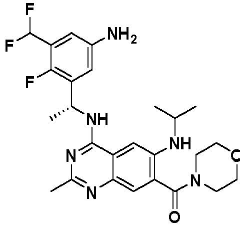 | (*R*)-4-((1-(5-amino-3-(difluoromethyl)-2-fluorophenyl)ethyl)amino)-6-(isopropylamino)-2-methylquinazoline-7-(yl)(morpholino)methanone | 517.2 [M+H]⁺ |
| | ¹H-NMR (300 MHz, DMSO-*d₆*): δ 8.03 (d, 1H), 7.40 (s, 1H), 7.27 (s, 1H), 7.07 (t, 1H), 6.73 (m, 1H), 6.58 (m, 1H), 5.71 (m, 1H), 5.17 (s, 2H), 4.80 (m, 1H), 3.94 (m, 1H), 3.72 (m, 8H), 2.28 (s, 3H), 1.55 (d, 3H), 1.23 (m, 6H). | | |
| 56 | 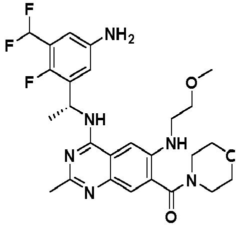 | (*R*)-(4-((1-(5-amino-3-(difluoromethyl)-2-fluorophenyl)ethyl)amino)-6-((2-methoxyethyl)amino)-2-methylquinazolin-7-yl)(morpholino)methanone | 533.2 [M+H]⁺ |
| | ¹H-NMR (300 MHz, DMSO-*d₆*): δ 8.06 (d, 1H), 7.40 (s, 1H), 7.30 (s, 1H), 7.07 (t, 1H), 6.74 (m, 1H), 6.59 (m, 1H), 5.72 (m, 1H), 5.21 (m, 3H), 3.60 (m, 8H), 3.42 (m, 4H), 3.31 (s, 3H), 2.29 (s, 3H), 1.55 (d, 3H). | | |
| 57 | 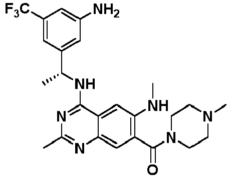 | (*R*)-(4-((1-(3-amino-5-(trifluoromethyl)phenyl)ethyl)amin o)-2-methyl-6-(methylamino)quinazoline-7-yl)(4-methylpiperazin-1-yl)methanone | 502.2 [M+H]⁺ |
| | ¹H-NMR (300 MHz, DMSO-*d₆*): δ 8.32 (s, 1H), 7.26 (d, 2H), 6.90 (d, 2H), 6.71 (s, 1H), 5.64-5.46 (m, 4H), 3.74 (m, 4H), 2.87 (d, 3H), 2.38-2.26 (m, 10H), 1.60 (d, 3H). | | |
| 58 | 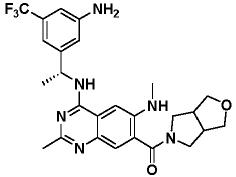 | (4-(((R)-1-(3-amino-5-(trifluoromethyl)phenyl)ethyl)amin o)-2-methyl-6-(methylamino)quinazolin-7-yl)(tetrahydro-1*H*-furo[3,4-c]pyrrol-5(3*H*)-yl)methanone | 515.2 [M+H]⁺ |
| | ¹H-NMR (300 MHz, DMSO-*d₆*): δ 9.12 (m, 1H), 7.36 (d, 2H), 6.90 (d, 2H), 6.74 (s, 1H), 5.88-5.60 (m, 4H), 3.80-3.45 (m, 8H), 2.92 (m, 5H), 2.48 (s, 3H), 1.60 (d, 3H). | | |
| 59 | 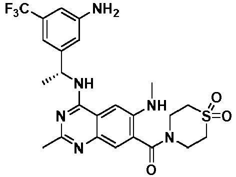 | (*R*)-(4-((1-(3-amino-5-(trifluoromethyl)phenyl)ethyl)amin o)-2-methyl-6-(methylamino)quinazolin-7-yl)(1,1-dioxothiomorpholino)methanone | 537.2 [M+H]⁺ |
| | ¹H-NMR (300 MHz, CD₃OD): δ 7.42 (s, 1H), 7.27 (s, 1H), 6.99 (m, 2H), 6.80 (m, 1H), 5.64 (m, 1H), 3.22 (m, 8H), 2.94 (s, 3H), 2.44 (s, 3H), 1.65 (d, 3H). | | |
| 60 | 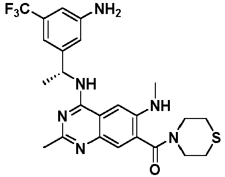 | (R)-(4-((1-(3-amino-5-(trifluoromethyl)phenyl)ethyl)amin o)-2-methyl-6-(methylamino)quinazolin-7-yl)(thiomorpholino)methanone | 505.2 [M+H]⁺ |
| | ¹H-NMR (300 MHz, DMSO-*d₆*): *δ* 8.07 (d, 1H), 7.25 (s, 1H), 7.19 (s, 1H), 6.90 (d, 2H), 6.70 (s, 1H), 5.60-5.53 (m, 3H), 5.36 (m, 1H), 3.89 (m, 2H), 3.50 (m, 2H), 2.85 (m, 3H), 2.73 (m, 2H), 2.53 (m, 2H), 2.34 (s, 3H), 1.55 (d, 3H). | | |
| 61 | 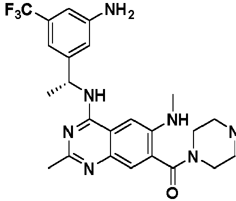 | (*R*)-(4-((1-(3-amino-5-(trifluoromethyl)phenyl)ethyl)amin o)-2-methyl-6-(methylamino)quinazoline-7-yl)(piperazine-1-yl)methanone | 488.2 [M+H]⁺ |
| | ¹H-NMR (300 MHz, DMSO-*d₆*): *δ* 8.06 (d, 1H), 7.21 (d, 2H), 6.90 (d, 2H), 6.70 (s, 1H), 5.64-5.33 (m, 4H), 3.61 (m, 2H), 3.18 (m, 2H), 2.86 (d, 3H), 2.78-2.55 (m, 4H), 2.34 (s, 3H), 1.58(d, 3H). | | |
| 62 | 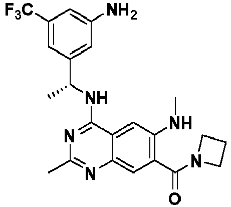 | (*R*)-(4-((1-(3-amino-5-(trifluoromethyl)phenyl)ethyl)amin o)-2-methyl-6-(methylamino)quinazoline-7-yl)(azetidine-1-yl)methanone | 459.2 [M+H]⁺ |
| | ¹H-NMR (300 MHz, DMSO-*d₆*): *δ* 8.16 (m, 1H), 7.42 (s, 1H), 7.20 (s, 1H), 6.88 (m, 2H), 6.69 (s, 1H), 6.35 (m, 1H), 5.56 (m, 3H), 4.12 (m, 2H), 4.04 (m, 2H) 2.87 (m, 3H), 2.34 (s, 3H), 2.22 (m, 2H), 1.56 (d, 3H). | | |
| 63 | 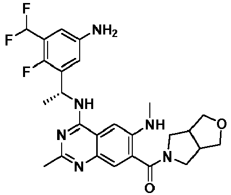 | (4-(((R)-1-(5-amino-3-(difluoromethyl)-2-fluorophenyl)ethyl)amino)-2-methyl-6-(methylamino)quinazoline-7-yl)(tetrahydro-1H furo[3,4-c]pyrrol-5(3H)-yl)methanone | 515.2 [M+H]⁺ |
| | ¹H-NMR (300 MHz, DMSO-*d₆*): *δ* 9.56 (m, 1H), 7.44 (d, 2H), 7.27-6.91 (m, 1H), 6.80 (d, 1H), 6.67 (m, 1H), 6.06-5.32 (m, 4H), 3.80-3.43 (m, 8H), 2.91 (m, 5H), 2.50 (s, 3H), 1.65 (d, 3H). | | |
| 64 | 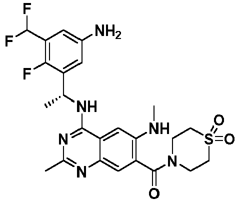 | (*R*)-(4-((1-(5-amino-3-(difluoromethyl)-2-fluorophenyl)ethyl)amino)-2-methyl-6-(methylamino)quinazoline-7-yl)(1,1-dioxothiomorpholino)methanone | 537.2 [M+H]⁺ |
| | ¹H-NMR (300 MHz, DMSO-*d₆*): *δ* 8.09 (m, 1H), 7.41 (s, 1H), 7.26 (t, 1H), 6.75 (m, 2H), 6.59 (m, 1H), 5.72 (m, 1H), 5.19 (m, 2H), 4.07 (m, 2H), 3.58 (m, 2H), 3.31 (m, 2H), 3.19 (m, 2H), 2.87 (m, 3H), 2.29 (s, 3H), 1.55 (d, 3H). | | |
| 65 | 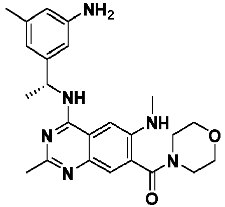 | (*R*)-(4-((1-(3-amino-5-methylphenyl)ethyl)amino)-2-methyl-6-(methylamino)quinazolin-7-yl)(morpholino)methanone | 435.2 [M+H]⁺ |
| | ¹H-NMR (300 MHz, CD₃OD): δ 7.37 (s, 1H), 7.28 (s, 1H), 6.66 (m, 2H), 6.47 (s, 1H), 5.65 (m, 1H), 3.98 (m, 8H), 2.96 (s, 3H), 2.48 (s, 3H), 2.24 (s, 3H), 1.66 (s, 3H). | | |
| 66 | 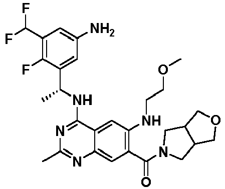 | (4-(((*R*)-1-(5-amino-3-(difluoromethyl)-2-fluorophenyl)ethyl)amino)-6-((2-methoxyethyl)amino)-2-methylquinazoline-7-yl)(tetrahydro-1*H*-furo[3,4-c]pyrrol-5(3*H*)-yl)methanone | 559.2 [M+H]⁺ |
| | ¹H-NMR (300 MHz, DMSO-*d₆*): *δ* 8.63 (m, 1H), 7.46 (d, 2H), 7.09-6.91 (m, 1H), 6.76 (d, 1H), 6.63 (m, 1H), 5.78-5.24 (m, 4H), 4.11-3.32 (m, 15H), 2.91 (m, 2H), 2.37 (s, 3H), 1.60 (d, 3H). | | |
| 67 | 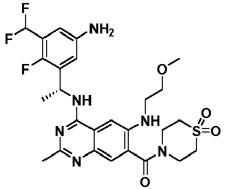 | (*R*)-(4-((1-(5-amino-3-(difluoromethyl)-2-fluorophenyl)ethyl)amino)-6-((2-methoxyethyl)amino)-2-methylquinazoline-7-yl)(1,1-dioxothiomorpholino)methanone | 581.2 [M+H]⁺ |
| | ¹H-NMR (300 MHz, CD₃OD): δ 7.40 (s, 1H), 7.38 (s, 1H), 6.88 (t, 1H), 6.85 (m, 1H), 6.77 (m, 1H), 5.77 (m, 1H), 4.59 (m, 4H), 3.69 (m, 6H), 3.48 (m, 2H), 3.42 (s, 3H), 2.39 (s, 3H), 1.65 (d, 3H). | | |
| 68 | 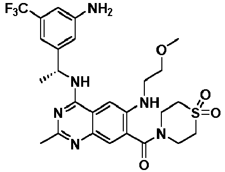 | (R)-(4-((1-(3-amino-5-(trifluoromethyl)phenyl)ethyl)amin o)-6-((2-methoxyethyl)amino)-2-methylquinazoline-7-yl)(1, 1-dioxothiomorpholino)methanone | 581.2 [M+H]⁺ |
| | ¹H-NMR (300 MHz, DMSO-*d₆*): *δ* 8.07 (m, 1H), 7.42 (s, 1H), 7.32 (s, 1H), 6.87 (m, 2H), 6.69 (s, 1H), 5.56 (m, 3H), 5.31 (m, 1H), 3.59 (m, 2H), 3.41 (m, 4H), 3.29 (m, 9H), 2.33 (s, 3H), 1.55 (d, 3H). | | |
| 69 | 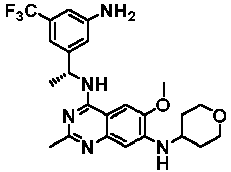 | (*R*)-*N*⁴-(1-(3-amino-5-(trifluoromethyl)phenyl)ethyl)-6-methoxy-2-methyl-N⁷-(tetrahydro-2H pyran-4-yl)quinazoline-4,7-diamine | 476.2 |
| | ¹H-NMR (300 MHz, CD₃OD): 6 7.52 (s, 1H), 6.96 (m, 2H), 6.80 (m, 1H), 6.61 (s, 1H), 5.63 (m, 1H), 3.98 (m, 5H), 3.68 (m, 1H), 3.58 (m, 2H), 2.44 (s, 3H), 2.04 (m, 2H), 1.67 (m, 5H). | | |

### Experimental Example 1: Testing the ability of SOS1 to inhibit GTP substitution

In order to determine whether the substitution function of SOS1 binding to KRAS G12C is inhibited by the above-described example compounds, a homogeneous time-resolved fluorescence (HTRF) test was performed using G12C(#MSC-11-538) and SOS1-Strep(#MSC-11-502) purchased from Reaction Biology Corporation (MA, USA). The activity of SOS1 enzyme was determined by measuring the amount of GTP bound to KRAS G12C proteins, using the principle of time-resolved fluorescence energy transfer (TR-FRET). Based on the principle that terbium bound to the GST-binding antibody of the GST-KRAS G12C protein acts as a donor of fluorescence resonance energy transfer (FRET), and the GTP bound to the KRAS G12C protein is labeled with the fluorescent agent DY-647P1 and acts as a receiver, when the more GTP is bound to the KRAS G12C protein by the substitution reaction of SOS1 according to this test, the higher HTRF or FRET signal is measured.

To summarize, glutathione S-transferase (GST)-tagged KRAS G12C proteins (consisting of amino acid residues 2 to 169) required for the reaction, streptavidin-tagged SOS1 proteins (consisting of amino acid residues 564 to 1049 which are the catalytic domain), anti-GST antibody (#61GSTKLA) purchased from CISBIO corporation, nucleic acid (#NU-820-647P1) purchased from Jena Bioscience GmbH were mixed in a buffer solution formed from 10 mM HEPES pH7.4, 150 mM NaCl, 5 mM MgCl₂, and 1 mM dithiothreitol (DTT), and the mixture was added to a 384-well plate and reacted at room temperature. Next, FRET signals were measured with a Perkin Elmer Envision microplate reader. In this case, an excitation signal (excitation) was measured at 320 nm, and an emission signal (emission) was measured at 615/665 nm. The fluorescence measurement value when the SOS1 protein was not included was calculated as a background signal value, and the background signal value was subtracted from all of the measured HTRF values. The fluorescence measurement value when the synthesized compound was not included was measured as a control value, and this value was selected as a reference point of 100%. For each of the example compounds, fluorescence was measured at concentrations of 1.6, 8, 40, 200, and 1,000 nM (5 points, 5-fold), and the 50% activity inhibition value (IC₅₀) of each of the compounds was calculated using GraphPad Prism. As a result, the KRAS G12C-SOS1 binding inhibitory ability of each of the example compounds is shown in Table 2 below.

When the IC₅₀ value was 50 nM or less, the inhibitory ability was denoted as +++. When the IC₅₀ value was more than 50 nM and 100 nM or less, the inhibitory ability was denoted as ++. When the IC₅₀ value was more than 100 nM, the inhibitory ability was denoted as +.

**[Table 2]**

| **Synthesis compound** | **IC₅₀ (nM)** | **Synthesis compound** | **IC₅₀ (nM)** | **Synthesis compound** | **IC₅₀ (nM)** |
|---|---|---|---|---|---|
| Example 1 | +++ | Example 24 | ++ | Example 47 | ++ |
| Example 2 | ++ | Example 25 | +++ | Example 48 | + |
| Example 3 | + | Example 26 | +++ | Example 49 | ++ |
| Example 4 | ++ | Example 27 | ++ | Example 50 | +++ |
| Example 5 | + | Example 28 | +++ | Example 51 | +++ |
| Example 6 | ++ | Example 29 | +++ | Example 52 | ++ |
| Example 7 | + | Example 30 | ++ | Example 53 | +++ |
| Example 8 | + | Example 31 | ++ | Example 54 | ++ |
| Example 9 | +++ | Example 32 | + | Example 55 | +++ |
| Example 10 | + | Example 33 | + | Example 56 | +++ |
| Example 11 | + | Example 34 | + | Example 57 | ++ |
| Example 12 | ++ | Example 35 | +++ | Example 58 | +++ |
| Example 13 | ++ | Example 36 | ++ | Example 59 | +++ |
| Example 14 | + | Example 37 | + | Example 60 | +++ |
| Example 15 | + | Example 38 | + | Example 61 | +++ |
| Example 16 | ++ | Example 39 | + | Example 62 | ++ |
| Example 17 | +++ | Example 40 | +++ | Example 63 | +++ |
| Example 18 | +++ | Example 41 | ++ | Example 64 | +++ |
| Example 19 | + | Example 42 | + | Example 65 | ++ |
| Example 20 | +++ | Example 43 | +++ | Example 66 | |
| Example 21 | ++ | Example 44 | ++ | Example 67 | |
| Example 22 | + | Example 45 | ++ | Example 68 | +++ |
| Example 23 | ++ | Example 46 | ++ | Example 69 | + |

## Claims

1. A compound selected from compounds of Chemical Formula 1 below and pharmaceutically acceptable salts, optical isomers, diastereomers, hydrates, and solvates of the compounds of Chemical Formula 1: 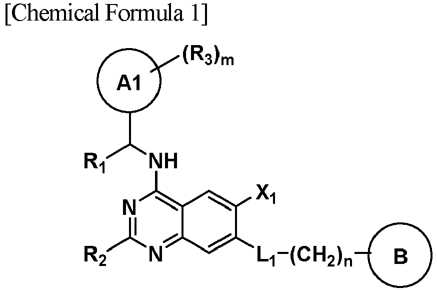 In Chemical Formula 1,
Ri is hydrogen or C₁₋₄ alkyl;
R₂ is hydrogen, C₁₋₄ alkyl, halo C₁₋₄ alkyl, C₂₋₆ alkenyl or C₂₋₆ alkynyl;
R₃ is R₃ₐ or -L₂- 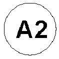 ;
R₃ₐ are each independently halogen, hydroxy, cyano, amino, amine, nitro, oxo (=O), C₁₋₆ alkyl, C₂₋₆ alkenyl, C₂₋₆ alkynyl, halo C₁₋₆ alkyl, amino C₁₋₆ alkyl, C₁₋₆ alkoxy, hydroxy-C₁₋₄ alkyl, - CF₂H, -(CH₂)ᵣ-NH(CO)-Rₐ, or -(CH₂)r-NRₐR_{b}, and Rₐ and R_{b} are each independently any one selected from the group consisting of hydrogen, C₁₋₆ alkyl, halo C₁₋₆ alkyl, hydroxy-C₁₋₆ alkyl, C₂₋₆ alkenyl, C₂₋₆ alkynyl, C₁₋₆ alkoxy, -CF₂H, and C₃₋₈ carbocyclyl;
r is an integer in the range of 0 to 1;
m is an integer in the range of 0 to 5;
L₂ is a direct bond, -O-(CH₂)ₚ, or -CH=CH-(CH₂)_{q};
p is an integer in the range of 0 to 3;
q is an integer in the range of 0 to 2;
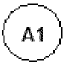 and 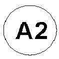 are each independently C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl, wherein C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl of 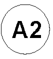 is unsubstituted or substituted with one or more R₃ₐ,
X₁ is -O(R₄) or -N(R₅)(R₆);
R₄ is hydrogen, C₁₋₆ alkyl, hydroxy C₁₋₆ alkyl, C₂₋₆ alkenyl, C₂₋₆ alkynyl, C₁₋₆ alkoxy, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridge carbocyclyl, or C₆₋₁₄ bridged heterocyclyl each of which is unsubstituted or substituted with halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, -C(O)-NR_{c}R_{d}, -C(O)OR_{c}, -OR_{c}, or -NR_{c}R_{d}, wherein R_{c} and R_{d} are each independently hydrogen or C₁₋₆ alkyl;
R₅ and R₆ are each hydrogen, C₁₋₆ alkyl, hydroxy C₁₋₆ alkyl, C₂₋₆ alkenyl, C₂₋₆ alkynyl, C₁₋₆ alkoxy, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridged carbocyclyl, or C₆₋₁₄ bridged heterocyclyl, wherein C₁₋₆ alkyl, hydroxy C₁₋₆ alkyl, C₂₋₆ alkenyl, C₂₋₆ alkynyl, C₁₋₆ alkoxy, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridged carbocyclyl, or C₆₋₁₄ bridged heterocyclyl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, - C(O)-NRₑR_{f}, -C(O)ORₑ, -ORₑ, and -NRₑR_{f}, wherein Rₑ and Rf are each independently hydrogen or C₁₋₆ alkyl, or
alternatively, the -N(R₅)(R₆) is C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl in each of which R₅ and R₆ are linked to each other and form a ring in conjunction with a nitrogen atom contained in the -N(R₅)(R₆), in which C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆alkynyl, C₁₋₆ alkoxy, halo C₁₋₆ alkoxy, -S(O)-C₁₋₄ alkyl, -S(O)₂-C₁₋₄ alkyl, -C(O)-NR_{g}Rₕ, -C(O)OR_{g}, -OR_{g}, and -NR_{g}Rₕ, wherein R_{g} and Rₕ are each independently hydrogen, C₁₋₆ alkyl, hydroxy-C₁₋₆ alkyl, C₂₋₆ alkenyl, C₂₋₆ alkynyl, C₁₋₆ alkoxy, halo C₁₋₆ alkoxy, or C₃₋₈ carbocyclyl;
L₁ is a direct bond, -C(O)-, -O-, or -NH-;
n is an integer in the range of 0 to 2; and
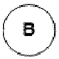 is C₃₋₁₀ carbocyclyl, C₂₋₉ heterocyclyl, C₆₋₁₀ aryl, C₄₋₁₀ heteroaryl, C₆₋₁₄ heterospirocarbocyclyl, C₆₋₁₄ fused heterocyclyl, or C₆₋₁₄ bridged heterocyclyl, wherein C₃₋₁₀ carbocyclyl, C₂₋₉ heterocyclyl, C₆₋₁₀ aryl, C₄₋₁₀ heteroaryl, C₆₋₁₄ heterospirocarbocyclyl, C₆₋₁₄ fused heterocyclyl, or C₆₋₁₄ bridged heterocyclyl is unsubstituted or substituted with one or more functional groups selected from the group consisting of halogen, hydroxy, nitro, oxo (=O), halo C₁₋₆ alkyl, C₁₋₆ alkyl, C₂₋₆ alkenyl, C₂₋₆ alkynyl, C₁₋₆ alkoxy, -S(O)-C₁₋₄ alkyl, -S(O)₂-C₁₋₄ alkyl, -C(O)-NRᵢRⱼ, - C(O)ORᵢ, -ORᵢ, and -NRᵢRⱼ, wherein Rᵢ and Rⱼ are each independently hydrogen or C₁₋₆ alkyl.

2. The compound according to claim 1, wherein 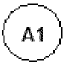 and 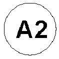 are each independently C₆₋₁₀ aryl or C₄₋₁₀ heteroaryl.

3. The compound according to claim 1, wherein R₃ₐ is each independently halogen, hydroxy, cyano, amino, amine, nitro, C₁₋₆ alkyl, halo C₁₋₆ alkyl, amino C₁₋₆ alkyl, C₁₋₆ alkoxy, -CF₂H, C₆₋₁₀ aryl, C₃₋₆ cyclyl, -(CH₂)ᵣ-C₂₋₆ heterocyclyl, -(CH₂)ᵣ-NH(CO)-Rₐ or -(CH₂)ᵣ-NRₐR_{b}, wherein Rₐ and R_{b} are each independently hydrogen, C₁₋₆ alkyl, -CF₃, or -CF₂H.

4. The compound according to claim 1, wherein R₄ is hydrogen, C₁₋₆ alkyl, C₂₋₆ alkenyl, C₂₋₆ alkynyl, C_{1 -6} alkoxy, C₃₋₁₀ carbocyclyl, or C₂₋₉ heterocyclyl, and
R₅ and R₆ are each independently hydrogen, C₁₋₆ alkyl, C₂₋₆ alkenyl, C₂₋₆ alkynyl, C₁₋₆ alkoxy, C₃₋₁₀ carbocyclyl, or C₂₋₉ heterocyclyl, or the -N(R₅)(R₆) is C₂₋₉ heterocyclyl.

5. The compound according to claim 1, wherein the compound represented by Chemical Formula 1 is a compound represented by Chemical Formula 2 below: 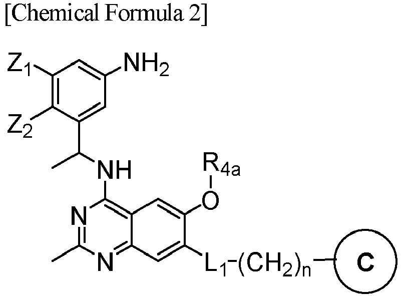 In Chemical Formula 2,
L₁ is a direct bond, -C(O)-, -O-, or -NH-;
n is an integer in the range of 0 to 2; and
Z₁ and Z₂ are each independently hydrogen, -F, -CF₂H, -CF₃, -CH₃, or -NH₂, but for the case in which Z₁ and Z₂ are both hydrogen;
R₄ₐ is hydrogen, C₁₋₆ alkyl, C₃₋₁₀ carbocyclyl, or C₂₋₉ heterocyclyl;
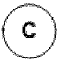 is morpholinyl, thiomorpholinyl, dioxothiomorpholinyl, piperazinyl, thiazolidinyl, tetrahydropyranyl, hexahydro-1*H*-furo[3,4-c]pyrrolyl, oxetanyl or azetidinyl, wherein morpholinyl, thiomorpholinyl, dioxothiomorpholinyl, piperazinyl, thiazolidinyl, tetrahydropyranyl, hexahydro-1*H*-furo[3,4-c]pyrrolyl, oxetanyl or azetidinyl is unsubstituted or substituted with one or more functional groups selected from the group consisting of halogen or -CH₃.

6. The compound according to claim 1, wherein the compound represented by Chemical Formula 1 is a compound represented by Chemical Formula 3 below: 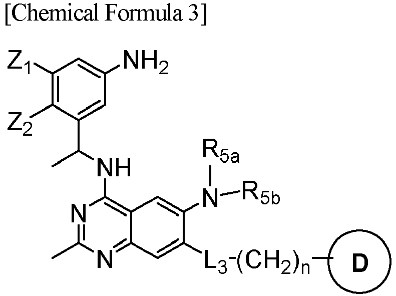 In Chemical Formula 3,
L₃ is a direct bond or -C(O)-;
n is an integer in the range of 0 to 2; and
Z₁ and Z₂ are each independently hydrogen, -F, -CF₂H, -CF₃, -CH₃, or -NH₂, but for the case in which Z₁ and Z₂ are both hydrogen;
R₅ₐ and R_{5b} are each independently hydrogen, C₁₋₆ alkyl, Ci ₋₆ alkoxy, C₃₋₁₀ carbocyclyl, or C₂₋₉ heterocyclyl, or
alternatively the -N(R₅ₐ)(R_{5b}) is a C₂₋₉ heterocyclyl in which R₅ₐ and R_{5b} are linked to each other and form a ring in conjunction with a nitrogen atom contained in the -N(R₅ₐ)(R_{5b});
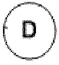 is morpholinyl, dioxothiomorpholinyl, thiomorpholinyl, piperazinyl, thiazolidinyl, tetrahydropyranyl, hexahydro- l//-furo[3,4-c]pyrrolyl, oxetanyl or azetidinyl, wherein morpholinyl, dioxothiomorpholinyl, thiomorpholinyl, piperazinyl, thiazolidinyl, tetrahydropyranyl, hexahydro-1*H*-furo[3,4-c]pyrrolyl, oxetanyl or azetidinyl is unsubstituted or substituted with one or more functional groups selected from the group consisting of halogen or -CH₃.

7. The compound according to claim 5, wherein the compound represented by Chemical Formula 2 is a compound represented by Chemical Formula 4 below: 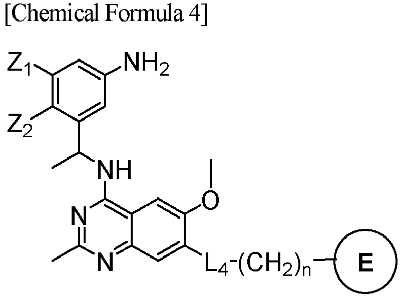 In Chemical Formula 4,
L₄ is a direct bond, -C(O)- or -O-;
n is an integer in the range of 0 to 2; and
Z₁ and Z₂ are each independently hydrogen, -F, -CF₂H, -CF₃, -CH₃, or -NH₂, but for the case in which Z₁ and Z₂ are both hydrogen;
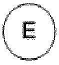 is morpholinyl, thiomorpholinyl, piperazinyl, thiazolidinyl, or tetrahydropyranyl, wherein the morpholinyl, thiomorpholinyl, piperazinyl, thiazolidinyl, or tetrahydropyranyl is unsubstituted or substituted with one or more functional groups selected from the group consisting of halogen or -CH₃.

8. The compound according to claim 6, wherein the compound represented by Chemical Formula 3 is a compound represented by Chemical Formula 5 below: 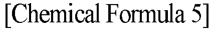 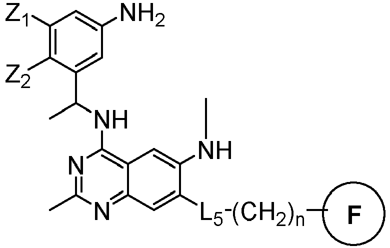 In Chemical Formula 5,
L₅ is a direct bond or -C(O)-;
n is an integer in the range of 0 to 2; and
Z₁ and Z₂ are each independently hydrogen, -F, -CF₂H, -CF₃, -CH₃, or -NH₂, but for the case in which Z₁ and Z₂ are both hydrogen;
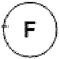 is morpholinyl, dioxothiomorpholinyl, thiomorpholinyl, piperazinyl, thiazolidinyl, or hexahydro-1*H*-furo[3,4-c]pyrrolyl, wherein the morpholinyl, dioxothiomorpholinyl, thiomorpholinyl, piperazinyl, thiazolidinyl, or hexahydro-1*H*furo[3,4-c]pyrrolyl is unsubstituted or substituted with one or more functional groups selected from the group consisting of halogen or - CH₃.

9. The compound according to claim 1, wherein the compound of Chemical Formula 1 is a compound selected from the group consisting of
(*R*)-(4-((1-(3-amino-5-(trifluoromethyl)phenyl)ethyl)amino)-6-methoxy-2-methylquinazoline-7-yl)(morpholino)methanone;
(6-methoxy-2-methyl-4-((1-(4-(2-((methylamino)methyl)phenyl)thiophen-2-yl)ethyl)amino)quinazoline-7-yl)(morpholino)methanone;
(4-(((*R*)-1-(3-amino-5-(trifluoromethyl)phenyl)ethyl)amino)-6-methoxy-2-methylquinazoline-7-yl)((3R, 5*S*)-3,5-dimethylpiperazin-1-yl)methanone;
(*R*)-(4-((1-(3-amino-5-(trifluoromethyl)phenyl)ethyl)amino)-6-methoxy-2-methylquinazoline-7-yl)(thiomorpholino)methanone;
(4-(((*R*)-1-(3-amino-5-(trifluoromethyl)phenyl)ethyl)amino)-6-methoxy-2-methylquinazoline-7-yl)(tetrahydro-1*H*-furo[3,4-c]pyrrol-5(3*H*)-yl)methanone;
(*R*)-(4-((1-(3-(difluoromethyl)-2-fluorophenyl)ethyl)amino)-6-methoxy-2-methylquinazoline-7-yl)(morpholino)methanone;
(*R*)-(4-((1-(3-amino-5-(trifluoromethyl)phenyl)ethyl)amino)-6-methoxy-2-methylquinazoline-7-yl)(azetidine-1-yl)methanone;
(6-methoxy-2-methyl-4-((1-(4-(1,2,3,4-tetrahydroisoquinolin-8-yl)thiophen-2-yl)ethyl)amino)quinazoline-7-yl)(morpholino)methanone;
(*R*)-(4-((1-(3-amino-5-(trifluoromethyl)phenyl)ethyl)amino)-6-methoxy-2-methylquinazoline-7-yl)(piperazine-1-yl)methanone;
(*R*)-2,2,2-trifluoro-*N*-(3-(1-((6-methoxy-2-methyl-7-(morpholine-4-carbonyl)quinazoline-4-yl)amino)ethyl)-5-(triffuoromethyl)phenylacetamide;
(*R*)-(4-((1-(3-amino-5-(trifluoromethyl)phenyl)ethyl)amino)-6-methoxy-2-methylquinazoline-7-yl)(3-ffuorazetidine-1-yl)methanone;
(4-((1-(4-(2-((dimethylamino)methyl)phenyl)thiophen-2-yl)ethyl)amino)-6-methoxy-2-methoxyquinazolin-7-yl)(morpholino)methanone;
(4-((1-(4-(2-((aminomethyl)phenyl)thiophen-2-yl)ethyl)amino)-6-methoxy-2-methylquinazoline-7-yl)(morpholino)methanone;
(4-((1-(4-(2-((hydroxymethyl)phenyl)thiophen-2-yl)ethyl)amino)-6-methoxy-2-methylquinazolin-7-yl)(morpholino)methanone;
(*R*)-(6-methoxy-2-methyl-4-((1-(3-(trifluoromethyl)phenyl)ethyl)amino)quinazolin-7-yl)(morpholino)methanone;
(*R*)-(4-((1-(5-amino-2-methyl-3-(trifluoromethyl)phenyl)ethyl)amino)-6-methoxy-2-methylquinazolin-7-yl)(morpholino)methanone;
(*R*)-(4-((1-(3-amino-5-(fluorophenyl)ethyl)amino)-6-methoxy-2-methylquinazolin-7-yl)(morpholino)methanone;
(*R*)-(4-((1-(3-amino-5-(trifluoromethyl)phenyl)ethyl)amino)-6-methoxy-2-methylquinazolin-7-yl)(1,1-dioxothiomorpholino)methanone;
(*R*)-(4-((1-(3-amino-2-methoxyphenyl)ethyl)amino)-6-methoxy-2-methylquinazolin-7-yl)(morpholino)methanone;
(*R*)-(4-((1-(3-amino-5-(trifluoromethyl)phenyl)ethyl)amino)-6-methoxy-2-methylquinazolin-7-yl)(thiazolidine-3-yl)methanone;
(*R*)-(4-((1-(3-amino-5-methylphenyl)ethyl)amino)-6-methoxy-2-methylquinazolin-7-yl)(morpholino)methanone;
(*R*)-3-amino-5-(1-((6-methoxy-2-methyl-7-(morpholine-4-carbonyl)quinazolin-4-yl)amino)ethyl)benzonitrile;
(R)-(4-((1-(2,3-dihydro-1*H*-inden-4-yl)ethyl)amino)-6-methoxy-2-methylquinazolin-7-yl)(morpholino)methanone;
(*R*)-(4-((1-(3-amino-5-cyclopropylphenyl)ethyl)amino)-6-methoxy-2-methylquinazolin-7-yl)(morpholino)methanone;
(*R*)-(4-((1-(5-amino-2-fluoro-3-(trifluoromethyl)phenyl)ethyl)amino)-6-methoxy-2-methylquinazolin-7-yl)(morpholino)methanone;
(*R*)-(4-((1-(5-amino-3-(difluoromethyl)-2-fluorophenyl)ethyl)amino)-6-methoxy 2-methylquinazolin-7-yl)(morpholino)methanone;
(4-(((*R*)-1-(3-amino-5-(trifluoromethyl)phenyl)ethyl)amino)-2-methyl-6-(((*S*)-tetrahydrofuran-3-yl)oxy)quinazolin-7-yl)(morpholino)methanone;
(*R*)-(4-((1-(3-amino-5-(furan-3-yl)phenyl)ethyl)amino)-6-methoxy-2-methylquinazolin-7-yl)(morpholino)methanone;
(*R*)-(4-((1-(3-amino-5-(difluoromethyl)phenyl)ethyl)amino)-6-methoxy-2-methylquinazolin-7-yl)(morpholino)methanone;
(*R*)-(4-((1-(3-amino-5-(thiazole-5-yl)phenyl)ethyl)amino)-6-methoxy-2-methylquinazolin-7-yl)(morpholino)methanone;
(*R*)-(4-((1-(3-(ethylamino)-5-(trifluoromethyl)phenyl)ethyl)amino)-6-methoxy-2-methylquinazolin-7-yl)(morpholino)methanone;
methyl (*R*)-(4-((1-(3-amino-5-(trifluoromethyl)phenyl)ethyl)amino)-6-(2-methoxyethoxy)-2-methylquinazolin-7-yl)(morpholino)methanone;
(*R*)-(4-((1-(3-amino-5-(trifluoromethyl)phenyl)ethyl)amino)-2-(fluoromethyl)-6-methoxyquinazolin-7-yl)(morpholino)methanone;
(R)-N-(1-(3-amino- 5-(trifluoromethyl)phenyl)ethyl)-6-methoxy-2- methyl-7-(morpholinomethyl)quinazoline-4-amine;
(*R*)-*N*-(1-(5-amino-3-(difluoromethyl)-2-fluorophenyl)ethyl)-6-methoxy-2-methyl-7-(morpholinomethyl)quinazoline-4-amine;
(*R*)-*N*-(1-(3-amino-5-(trifluoromethylphenyl)ethyl)-6-methoxy-2-methyl-7-((tetrahydro-2*H*-pyran-4-yl)oxy)quinazolin-4-amine;
(*R*)-*N*-(1-(5-amino-3-(difluoromethyl)-2-fluorophenyl)ethyl)-6-methoxy-2-methyl-7-((tetrahydro-2*H*-pyran-4-yl)oxy)quinazolin-4-amine;
(*R*)-*N*-(1-(3-amino-5-(trifluoromethyl)phenyl)ethyl)-6-methoxy-2-methyl-7-((tetrahydro-2*H*-pyran-4-yl)methoxy)quinazolin-4-amine;
(*R*)-*N*-(1-(3-amino-5-(trifluoromethyl)phenyl)ethyl)-6-methoxy-2-methyl-7-(oxetan-3-ylmethoxy)quinazoline-4-amine;
(*R*)-(4-((1-(3-amino-5-(trifluoromethyl)phenyl)ethyl)amino)-2-methyl-6-(methylamino)quinazolin-7-yl)(morpholino)methanone;
(*R*)-(4-((1-(3-amino-5-(trifluoromethyl)phenyl)ethyl)amino)-6-(dimethylamino)-2-methylquinazolin-7-yl)(morpholino)methanone;
(*R*)-(4-((1-(3-amino-5-(trifluoromethyl)phenyl)ethyl)amino)-2-methyl-6-(pyrrolidin-1-yl)quinazoline-7-yl)(morpholino)methanone;
(R)-(4-((1-(5-amino-3-(diffuoromethyl)-2-fluorophenyl)ethyl)amino)-2-methyl-6-(methylamino)quinazoline-7-yl)(morpholino)methanone;
(*R*)-(4-((1-(3-amino-5-(trifluoromethyl)phenyl)ethyl)amino)-6-((2-methoxyethyl)amino)-2-methylquinazoline-7-yl)(morpholino)methanone;
(*R*)-(4-((1-(3-amino-5-(trifluoromethyl)phenyl)ethyl)amino)-6-(cyclopentylamino)-2-methylquinazolin-7-yl)(morpholino)methanone;
(*R*)-(4-((1-(3-amino-5-(trifluoromethyl)phenyl)ethyl)amino)-6-(ethylamino)-2-methylquinazolin-7-yl)(R)-(4-((1-(3-amino-5-(trifluoromethyl)phenyl)ethyl)amino)-6-(ethylamino)-2-methylquinazolin-7-yl)(morpholino)methanone;
(R)-(4-((1-(3-amino-5-(triffuoromethyl)phenyl)ethyl)amino)-6-(isopropylamino)-2-methylquinazolin-7-yl)(morpholino)methanone;
(*R*)-(4-((1-(3-amino-5-(trifluoromethyl)phenyl)ethyl)amino)-2-methyl-6-((tetrahydro-2*H-*pyran-4-yl) amino)quinazolin-7-yl)(morpholino)methanone;
(*R*)-*N*⁴-(1-(3-amino-5-(trifluoromethyl)phenyl)ethyl)-N⁶,2-dimethyl-7-(morpholinomethyl)quinazoline-4,6-diamine;
(*R*)-(4-((1-(5-amino-2-fluoro-3-(trifluoromethyl)phenyl)ethyl)amino)-2-methyl-6-(methylamino)quinazoline-7-yl)(morpholino)methanone;
(R)-(4-((1-(3-amino-5-(difluoromethyl)phenyl)ethyl)amino)-2-methyl-6-(methylamino)quinazolin-7-yl)(morpholino)methanone;
(*R*)-(4-((1-(5-amino-3-(difluoromethyl)-2-fluorophenyl)ethyl)amino)-6-(ethylamino)-2-methylquinazoline-7-yl)(morpholino)methanone;
(*R*)-(4-((1-(3-amino-5-(trifluoromethyl)phenyl)ethyl)amino)-2-methyl-6-(methylamino)quinazolin-7-yl)(thiazolidine-3-yl)methanone;
(*R*)-(4-((1-(3-amino-5-(furan-3-yl)phenyl)ethyl)amino)-2-methyl-6-(methylamino)quinazolin-7-yl)(morpholino)methanone;
(*R*)-4-((1-(5-amino-3-(difluoromethyl)-2-fluorophenyl)ethyl)amino)-6-(isopropylamino)-2-methylquinazoline-7-(yl)(morpholino)methanone;
(*R*)-(4-((1-(5-amino-3-(diffuoromethyl)-2-fluorophenyl)ethyl)amino)-6-((2-methoxyethyl)amino)-2-methylquinazolin-7-yl)(morpholino)methanone;
(*R*)-(4-((1-(3-amino-5-(trifluoromethyl)phenyl)ethyl)amino)-2-methyl-6-(methylamino)quinazoline-7-yl)(4-methylpiperazin-1-yl)methanone;
(4-(((*R*)-1-(3-amino-5-(trifluoromethyl)phenyl)ethyl)amino)-2-methyl-6-(methylamino)quinazolin-7-yl)(tetrahydro-1*H*-furo[3,4-c]pyrrol-5(3*H*)-yl)methanone;
(*R*)-(4-((1-(3-amino-5-(trifluoromethyl)phenyl)ethyl)amino)-2-methyl-6-(methylamino)quinazolin-7-yl)(1,1-dioxothiomorpholino)methanone;
(*R*)-(4-((1-(3-amino-5-(trifluoromethyl)phenyl)ethyl)amino)-2-methyl-6-(methylamino)quinazolin-7-yl)(thiomorpholino)methanone;
(*R*)-(4-((1-(3-amino-5-(trifluoromethyl)phenyl)ethyl)amino)-2-methyl-6-(methylamino)quinazolin-7-yl)(piperazine-1-yl)methanone;
(*R*)-(4-((1-(3-amino-5-(trifluoromethyl)phenyl)ethyl)amino)-2-methyl-6-(methylamino)quinazolin-7-yl)(azetidine-1-yl)methanone;
(4-(((*R*)-1-(5-amino-3-(diffuoromethyl)-2-fluorophenyl)ethyl)amino)-2-methyl-6-(methylamino)quinazoline-7-yl)(tetrahydro-1*H*-furo[3,4-c]pyrrol-5(3*H*)-yl)methanone;
(*R*)-(4-((1-(5-amino-3-(diffuoromethyl)-2-fluorophenyl)ethyl)amino)-2-methyl-6-(methylamino)quinazoline-7-yl)(1,1-dioxothiomorpholino)methanone;
(*R*)-(4-((1-(3-amino-5-methylphenyl)ethyl)amino)-2-methyl-6-(methylamino)quinazolin-7-yl)(morpholino)methanone;
(4-(((*R*)-1-(5-amino-3-(difluoromethyl)-2-fluorophenyl)ethyl)amino)-6-((2-methoxyethyl)amino)-2-methylquinazolin-7-yl)(tetrahydro-1*H*-furo[3,4-c]pyrrol-5(3*H*)-yl)methanone;
(*R*)-(4-((1-(5-amino-3-(diffuoromethyl)-2-fluorophenyl)ethyl)amino)-6-((2-methoxyethyl)amino)-2-methylquinazolin-7-yl)(1,1-dioxothiomorpholino)methanone;
(*R*)-(4-((1-(3-amino-5-(trifluoromethyl)phenyl)ethyl)amino)-6-((2-methoxyethyl)amino)-2-methylquinazoline-7-yl)(1,1-dioxothiomorpholino)methanone; and
(*R*)-*N*⁴-(1-(3-amino-5-(trifluoromethyl)phenyl)ethyl)-6-methoxy-2-methyl-N⁷-(tetrahydro-277-pyran-4-yl)quinazoline-4,7-diamine.

10. A preventive or therapeutic composition comprising the compound according to any one of claims 1 to 9 or a pharmaceutically acceptable salt thereof as an active ingredient.

11. The pharmaceutical composition according to claim 10, wherein the pharmaceutical composition is used for treating cancer or a tumor that can be treated by inhibiting binding of SOS 1 to RAS family proteins and/or RAC1.

12. A pharmaceutical formulation comprising the pharmaceutical composition of claim 10.

13. The pharmaceutical formulation according to claim 12, wherein the pharmaceutical preparation is in the form of tablets, pills, powders, capsules, syrups or emulsions.

14. The pharmaceutical formulation according to claim 12, further comprising at least one selected from the group consisting of pharmaceutically acceptable carriers, stiffening agents, and excipients.

15. A method of inhibiting binding of SOS1 to a RAS family protein and/or RAC1 in a subject or cell, the method comprising administering to the subject a pharmaceutically effective amount of the compound according to any one of claims 1 to 9.

16. A method of inhibiting tyrosine kinase in a subject or cell, the method comprising administering to the subject a pharmaceutically effective amount of the compound according to any one of claims 1 to 9.

17. A method for preventing or treating cancer in a subject, the method comprising administering to the subject a pharmaceutically effective amount of the compound according to any one of claims 1 to 9.

18. A use of the compound according to any one of claims 1 to 9 or a pharmaceutically acceptable salt thereof for preventing or treating cancer or tumor.
